# EUROPEAN PATENT APPLICATION

(11) **EP 4 560 315 A2**
(43) Date of publication of application: **28.05.2025**
(21) Application number: 25165683.1
(22) Date of filing: 05.06.2020
(51) Int. Cl.: G01N 33/543

(54) **HYBRIDIZING ALL-LNA OLIGONUCLEOTIDES**

(30) Priority: 07.06.2019 EP 19179046
(62) Divisional of application: 20730280.3
(71) Applicant: F. Hoffmann-La Roche AG, 4070 Basel (CH); Roche Diagnostics GmbH, 68305 Mannheim (DE)
(72) Inventor: Bergmann, Frank, 82377 Penzberg (DE); Heindl, Dieter, 82377 Penzberg (DE); Schraeml, Michael, 82377 Penzberg (DE); Stoeckel, Johannes, 82377 Penzberg (DE)
(74) Representative: Altmann Stößel Dick Patentanwälte PartG mbB

(57) **Abstract**

The present report relates to hybridizing single-stranded (ss-) oligonucleotides which entirely consist of locked nucleic acid (LNA) monomers. The present document shows hybridization experiments with pairs of entirely complementary ss-oligonucleotides which fail to form a duplex within a given time interval. The present report provides methods to identify such incompatible oligonucleotide pairs. **In** another aspect, the present report provides pairs of complementary ss-oligonucleotides which are capable of rapid duplex formation. The present report also provides methods to identify and select compatible oligonucleotide pairs. **In** yet another aspect the present report provides use of compatible oligonucleotide pairs as binding partners in binding assays, e.g. receptor-based assays.

## Description

The present report relates to hybridizing single-stranded (ss-) oligonucleotides which entirely consist of locked nucleic acid (LNA) monomers. The present document shows hybridization experiments with pairs of entirely complementary ss-oligonucleotides which fail to form a duplex within a given time interval. The present report provides methods to identify such incompatible oligonucleotide pairs. In another aspect, the present report provides pairs of complementary ss-oligonucleotides which are capable of rapid duplex formation. The present report also provides methods to identify and select compatible oligonucleotide pairs. In yet another aspect the present report provides use of compatible oligonucleotide pairs as binding partners in binding assays, e.g. immunoassays. Specific embodiments are discussed in which compatible LNA oligonucleotide pairs are employed for immobilizing an analyte-specific capture molecule, in an assay to detect or determine the analyte in a sample.

### Background of the Invention

Particular focus is directed to general biochemical applications in which the specific interaction of the two partners of a binding pair and their eventual connection with each other, by way of molecular recognition, has a functional role. Very frequently, e.g. in immunoassays the biotin:(strept)avidin binding pair is used to immobilize an analyte-specific capture receptor to a solid phase. The present report conceptualizes, explains and details applications such as immunoassays with alternative binding pairs. Specifically, an alternative binding pair made of two single-stranded LNA oligonucleotides capable of forming a duplex by way of hybridization provides a technical alternative to the biotin:(strept)avidin binding pair.

A focus of the present disclosure is the means with which in the course of an immunoassay the capture receptor is anchored on the solid phase. In particular, the present disclosure focuses on a binding pair which facilitates immobilization of an analyte-specific capture receptor in the presence of a sample containing the analyte, and/or which is capable of anchoring a detection complex after the complex has formed. A binding pair in an immunoassay is required to have specific technical features. Firstly, the interaction of the two binding partners has to be specific. Furthermore, the kinetics of forming the connection of the binding partners has to ensure high speed with which the two separate partners of the binding pair interact and eventually associate, i.e. bind to each other. In addition, the connection of the two binding partners is desired to be stable once formed. Moreover, the binding partners must be amenable to chemical conjugation with other molecules such as analyte-specific receptors and solid phase surfaces, for their application in immunoassays.

It is important to appreciate that in immunoassays receptors and typically also the analytes to be detected retain their conformation and function only under certain conditions. Such conditions may differ depending on the particular receptor or analyte under consideration; thus, a receptor molecule or an analyte may tolerate only limited deviation from these conditions. Such conditions may comprise (but are not limited to) a buffered aqueous solution with a pH in the range of about pH 6 to about pH 8, one or more dissolved salts, one or more helper substances (e.g. selected from stabilizers, oxygen scavengers, preservatives, detergents), a total amount of solutes from about 200 to about 500 mosm/kg, absence of denaturing compounds such as certain non-aqueous solvents, helix destabilizers such as formamide and chaotropes, and a preferred storage and/or assay temperature in the range of 0 °C to 40 °C, to name but a few. Essential, however, are any ingredients and/or conditions which can cause denaturation of the analyte to be assayed, or the analyte-specific receptor to be used in a particular assay.

The separate partners of a binding pair are required to be amenable to conjugation, specifically conjugation with capture molecules i.e. receptors, and conjugation with solid phase surfaces, without losing their ability to specifically associate with, and bind, each other. With regards to conjugates in immunoassays each separate binding partner of the alternative binding pair must be functional under the assay conditions. The same reasoning applies to all other desired materials for conjugation with a binding partner, such as, but not limited to, an analyte, a carrier material, a solid phase, and other substances or compounds that may be present during the course of an assay.

Single-stranded oligonucleotides with complementary sequences, i.e. oligonucleotides capable of forming a duplex by way of hybridization have been proposed earlier as binding pair means to connect macromolecules, or to attach molecules to a solid phase. EP 0488152 discloses a heterogeneous immunoassay with a solid phase on which an analyte-specific capture antibody is immobilized by a nucleic acid duplex which connects the antibody and the solid phase. An embodiment is shown where one hybridized oligonucleotide is attached to the antibody and the complementary oligonucleotide is attached to the solid phase, thereby forming a connecting duplex. Similar disclosures are provided in the documents EP 0698792, WO 1995/024649, WO 1998/029736, and EP 0905517. WO 2013/188756 discloses methods of flow cytometry and a composition comprising an antibody conjugated to a first oligonucleotide, an oligosphere conjugated to a second oligonucleotide having a sequence identical to that of the first oligonucleotide, and an oligonucleotide probe with a label and a third sequence that is complementary to the first and the second oligonucleotides. In a specific embodiment the oligosphere is magnetic. The document reports specific uses of oligospheres as references in standardization procedures.

Modifed oligonucleotides such as peptide nucleic acid (PNA) and locked nucleic acid (LNA) have been explored for a range of primarily biochemical applications. LNA possesses a methylene linker between the 2'-oxygen and 4'-carbon atom of the ribose moiety that consequently locks the sugar into a C3*-endo* conformation, hence the name "locked nucleic acid". This chemical modification confers nuclease resistance as well as higher affinity and greater specificity for oligonucleotide targets in applications which involve duplex formation by hybridization of LNA monomer-containing oligonucleotides with complementary target sequences. LNA monomers are provided as 2'-*O*,4'-*C*-methylene-(D-ribofuranosyl) nucleoside monomers (Singh S.K. et al. Chem. Commun. 4 (1998) 455-456; Koskin A.A. et al. Tetrahedron 54 (1998) 3607-3630; Wengel J. Acc. Chem. Res. 32 (1999) 301-310). Further, WO 1998/39352 discloses locked nucleic acid (LNA) structures. By way of chemical synthesis, single strands consisting of LNA nucleoside analog monomers only ("all-LNA") can be synthesized.

Mixed DNA-LNA oligonucleotides that contain DNA and LNA monomers have enhanced thermal stability when hybridized to complementary DNA and RNA. In fact, in comparison with other high-affinity nucleic acid mimics that have been synthesized, e.g. peptide nucleic acids (PNAs), hexitol nucleic acids (HNAs) and 2'-fluoro N3'-phosphoramidates, LNA displays exceptional binding affinities. Hybridization kinetics of LNA-DNA mixed oligonucleotides, also known as "mixmers" were reported by Christensen U. et al. (Biochem J 354 (2001) 481-484). A crystal structure of an 'All Locked' nucleic acid duplex from two complementary ss-oligonucleotides, each consisting of 7 LNA monomers was reported by Eichert A. et al. (Nucleic Acids Research 38 (2010) 6729-6736).

For the most part, single-stranded mixed LNA/DNA oligonucleotides (LNA/DNA and LNA/RNA, i.e. mixmer single strands) were analyzed. Fewer reports of the characterization of hybridizing single-stranded oligonucleotides made exclusively from LNA monomers (i.e. "all-LNA" single-stranded oligonucleotides) were published, so far, particularly by Koshkin A.A. et al. (J Am Chem Soc 120 (1998) 13252-13253) and Möhrle B.P. et al. (Analyst 130 (2005) 1634-1638). Eze N.A. et al. (Biomacromolecules 18 (2017) 1086-1096) report association rates from DNA/LNA mixmers and DNA probes to be below 10⁵ M⁻¹ s⁻¹. According to these authors, the hybridization kinetics in solution does not seem to be affected by substituting one or more DNA monomers with LNA monomers, considering one third of monomers available for substitution. Childs J.L. (PNAS 99 (2002) 11091-11096) report an all-LNA octamer (TACCTTTC) capable of concentration-dependent inhibition of the self-splicing of a C. albicans group I intron in vitro. For the purpose of annealing the octamer to a target RNA, the octamer was heated to a temperature of 68 °C followed by cooling to 37 °C. The annealed LNA oligomer was found to perturb the tertiary structure of the intron, thereby affecting its biological function.

WO2000/066604 and WO2000/056746 disclose certain stereoisomers of LNA nucleoside monomers.

WO 1999/14226 suggests the use of oligonucleotides with LNA monomers in the construction of affinity pairs for attachment to molecules of interest and solid supports. However it is also known to the art that hybridization of complementary all-LNA single strands poses technical problems. An LNA-related user manual authored by Jesper Wengel and published by Exiqon mentions a tendency of single-stranded LNA-containing oligonucleotides to form intramolecular LNA:LNA duplexes, also referred to as self-hybridization. Thus, the document deems secondary structures as application-limiting, i.e. as a technical obstacle ("LNA Hybridization" in: "Locked Nucleic Acid Technology™: A brief overview", obtained on May 08, 2019 by way of internet download as electronic file https://www.exiqon.com/ls/Documents/Scientific/Locked%20Nucleic%20Acid%2 0Technology%20a%20brief%20overview.pdf). Thus, thermodynamic analysis of hybridization of oligonucleotide analogues consisting only of LNA is largely empirical, and sequence prediction of hybridizing pairs of complementary all-LNA oligomers in the absence of a prior denaturation step (e.g. heating prior to hybridization to remove intramolecular secondary structures) does not appear to be possible, so far.

Predictions concerning thermodynamic behavior of LNA-containing oligonucleotides are aided by dedicated computer programs referred to by Tolstrup N. et al. (Nucleic Acids Research 31 (2003) 3758-3762). However, this report explicitly mentions a higher prediction error for LNA oligonucleotides due to the more complex properties of these oligonucleotides, rather than lack of experimental data. In addition, the disclosed algorithms do not appear to provide guidance in the design of complementary pairs of all-LNA oligonucleotides. The same conclusion can be drawn from a more recent publication (Fakhfakh K. et al. American Institute of Chemical Engineers Journal 61 (2015) 2711-2731) reporting on the molecular thermodynamics of LNA:LNA base pairs in DNA/LNA mixmer oligonucleotides.

Specifically, the present report demonstrates that complementary single-stranded oligonucleotides solely consisting of LNA monomers can indeed be unpredictable with regards to their ability to form duplex molecules with Watson-Crick base pairing. Thus, in order to provide a technically suited replacement of the biotin:(strept)avdin binding pair in applications that make specific use of such molecular recognition, there is a need for technical means to select and provide alternative binding pairs; for the purpose of the present report, such binding pairs are desired to consist of complementary single-stranded oligonucleotides which solely contain LNA monomers, wherein
- the oligonucleotide pairs must comprise complementary sequences, and the complementary sequences must be capable of duplex formation and Watson-Crick base pairing;
- under conditions of storage and routine biochemical application in molecular recognition, the oligonucleotide pairs must not require any denaturation treatment prior to actual use of the binding pair in an application; this translates into the technical requirement that each oligonucleotide of the binding pair needs to be free of any inter- or intramolecularly formed secondary structure that would substantially reduce the respective oligonucleotide's capability of aligning and forming a duplex with its binding partner, i.e. a complementary oligonucleotide or a complementary sequence therein;
- under conditions of routine biochemical applications a single-stranded pair must be able of forming sufficiently rapid the duplex of Watson-Crick-paired oligonucleotides while at the same time ensuring sufficient specificity in molecular recognition;
- the duplex formed by the pair of complementary oligonucleotides is desired to be sufficiently stable and preferably irreversibly formed during the course of a given biochemical application that is using molecular recognition of the binding pair.

A general objective of the present report is therefore the identification and provision of binding pairs of single-stranded all-LNA oligonucleotides which in the absence of a prior denaturation step are capable of hybridizing, thereby being capable of forming duplex molecules with Watson-Crick base pairing as a binding pair in analyte detection assays under suitable assay conditions. In other words, binding pairs are sought which are capable of duplex formation under non-denaturing conditions, more specifically under conditions which are compatible with the function of analyte-specific receptors in an analyte detection assay (such as, but not limited to, an immunoassay). Importantly, single-stranded all-LNA oligonucleotides are sought which can be stored under ambient conditions or even refrigerated without forming inter or intramolecular secondary structures that could inhibit hybridization and duplex formation of complementary oligonucleotides. Also, single-stranded all-LNA oligonucleotides are sought which without prior denaturation can be hybridized with each other under assay conditions, such like in aqueous solution at ambient temperatures (e.g. room temperature). Absence of denaturation specifically means an intermittent step to remove any inter or intramolecular secondary structures which could inhibit hybridization and duplex formation of the complementary oligonucleotides used as binding pair in the analyte detection assay.

### Summary of the Invention

The present disclosure, in a first aspect being related to all other aspects and embodiments as disclosed herein, unexpectedly provides a pair of separate ss-oligonucleotides, each oligonucleotide consisting of 5 to 15 LNA monomers, the separate ss-oligonucleotides in aqueous solution being capable of forming with each other an antiparallel duplex in the absence of denaturing conditions prior to duplex formation or during duplex formation. The present report further discloses another embodiment of the first aspect, the embodiment being a pair of separate ss-oligonucleotides, each oligonucleotide consisting of 5 to 15 LNA monomers, the separate ss-oligonucleotides in aqueous solution being capable of forming with each other an antiparallel duplex comprising 5 to 15 consecutive base pairs in the absence of denaturing conditions prior to duplex formation or during duplex formation. The present report further discloses another embodiment of the first aspect, the embodiment being a pair of separate ss-oligonucleotides, each oligonucleotide consisting of 5 to 15 LNA monomers, the separate ss-oligonucleotides in aqueous solution being capable of forming with each other an antiparallel duplex comprising 5 to 7 consecutive base pairs in the absence of denaturing conditions prior to duplex formation or during duplex formation. The present report further discloses yet another embodiment of the first aspect, the embodiment being a pair of separate ss-oligonucleotides, each oligonucleotide consisting of 5 to 7 LNA monomers, the separate ss-oligonucleotides in aqueous solution being capable of forming with each other an antiparallel duplex comprising 5 to 7 consecutive base pairs in the absence of denaturing conditions prior to duplex formation or during duplex formation.

The present disclosure, in a second aspect being related to all other aspects and embodiments as disclosed herein, provides a method for selecting and providing a binding pair of single-stranded all-LNA oligonucleotides capable of forming in aqueous solution at a temperature from 0 °C to 40 °C an antiparallel duplex with 5 to 15 consecutive base pairs, the method comprising the steps of
(a) providing a first single-stranded (= ss-) oligonucleotide consisting of 5 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 5 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 5 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

The present disclosure, in a second aspect being related to all other aspects and embodiments as disclosed herein, provides a liquid composition comprising an aqueous solvent and a binding pair consisting of a first single-stranded oligonucleotide and a second single-stranded oligonucleotide,
wherein each oligonucleotide consists of 5 to 15 locked nucleic acid (= LNA) monomers, each monomer comprising a nucleobase, the nucleobases of the monomers forming a first nucleobase sequence of the first oligonucleotide and a second nucleobase sequence of the second oligonucleotide,
wherein the first nucleobase sequence and the second nucleobase sequence are selected that the first oligonucleotide and the second oligonucleotide are capable of forming an antiparallel duplex of 5 to 15 consecutive Watson-Crick base pairs at a temperature from 0 °C to 40 °C,
and wherein the binding pair is obtainable by a method according to the first aspect as disclosed herein.

### Detailed Description of the Invention

The terminology used herein is for the purpose of describing particular embodiments only and is not intended to be limiting of the invention.

The articles "a" and "an" are used herein to refer to one or to more than one (i.e., to at least one) of the grammatical object of the article. By way of example, "an item" means one item (one single item) or more than one item (a plurality of the item). In case "a" relates to a member that is part of a pair of two members, "a" denotes either one member of the pair or the plurality of both members, i.e. one single member of the pair or the two members altogether.

It is further understood that the root terms "include" and/or "have", when used in this specification, specify the presence of stated features, items, integers, steps, operations, elements, and/or components, but do not preclude the presence or addition of at least one other feature, integer, step, operation, element, component, and/or groups thereof. In an analogous way, "with" also specify the presence of stated features, etc.

As used herein, the terms "comprises," "comprising,", "contains", "containing", "includes," "including," "has," "having" or any other variation thereof, are intended to cover a non-exclusive inclusion, i.e. indicate an open list of features. For example, a process, method, article, or apparatus that comprises a list of features is not necessarily limited only to those features but may include other features not expressly listed or inherent to such process, method, article, or apparatus. In contrast, "consists of", "consisting of" or any other variation thereof specify a closed list of features. Notably, the closed list of given features is understood as representing a specific embodiment of an open list of these features.

As used herein, and unless expressly stated to the contrary, "or" refers to an inclusive-or and not to an exclusive-or. For example, a condition A or B is satisfied by any one of the following: A is true (or present) and B is false (or not present), A is false (or not present) and B is true (or present), and both A and B are true (or present).

As used herein "substantially", "relatively", "generally", "typically", "about", and "approximately" are relative modifiers intended to indicate permissible variation from the characteristic so modified. They are not intended to be limited to the absolute value or characteristic which it modifies but rather approaching or approximating such a physical or functional characteristic. If not stated otherwise, it is understood that the term "about" in combination with a numerical value n ("about n") indicates a value x in the interval given by the numerical value ±5 % of the value, i.e. n-0.05*n≦x≦n+0.05*n. In case the term "about" in combination with a numerical value n describes an embodiment of the invention, the value of n is most preferred, if not indicated otherwise.

In this detailed description, references to "one embodiment", "an embodiment", or "in embodiments" mean that the feature being referred to is included in at least one embodiment of the technology with regards to all its aspects according to present disclosure. Moreover, separate references to "one embodiment", "an embodiment", or "embodiments" do not necessarily refer to the same embodiment; however, neither are such embodiments mutually exclusive, unless so stated, and except as will be readily apparent to those skilled in the art. Thus, the technology in all its aspects according to present disclosure can include any variety of combinations and/or integrations of the embodiments described herein.

The term "solid phase" as used herein refers to a wide variety of materials including solids, semi-solids, gels, films, membranes, meshes, felts, composites, particles, papers and the like typically used by those of skill in the art to sequester molecules. The solid phase can be non-porous or porous. Suitable solid phases include those developed and/or used as solid phases in solid phase binding assays. See, e.g., chapter 9 of Immunoassay, E. P. Dianiandis and T. K. Christopoulos eds., Academic Press: New York, 1996, hereby incorporated by reference. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. See, e.g., Leon et al., Bioorg. Med. Chem. Lett. 8: 2997, 1998; Kessler et al., Agnew. Chem. Int. Ed. 40: 165, 2001; Smith et al., J. Comb. Med. 1: 326, 1999; Orain et al., Tetrahedron Lett. 42: 515, 2001; Papanikos et al., J. Am. Chem. Soc. 123: 2176, 2001; Gottschling et al., Bioorg. Med. Chem. Lett. 11: 2997, 2001.

Surfaces of solid phases as those described above may be modified to provide linkage sites, for example by bromoacetylation, silation, addition of amino groups using nitric acid, and attachment of intermediary proteins, dendrimers and/or star polymers. This list is not meant to be limiting, and any method known to those of skill in the art may be employed.

Particle-based analyte-specific binding assays are widely used in e.g. certain nephelometric assays, certain latex agglutination assays and many sensitive sandwich type assays employing a broad variety of labeling or detection techniques.

A particle is an embodiment of a solid phase. A "particle" as used herein means a small, localized object to which can be ascribed a physical property such as volume, mass or average size. Microparticles may accordingly be of a symmetrical, globular, essentially globular or spherical shape, or be of an irregular, asymmetric shape or form. The size of a particle envisaged by the present invention may vary. In one embodiment used are of globular shape, e.g. microparticles with a diameter in the nanometer and micrometer range. In one embodiment the microparticles used in a method according to the present disclosure have a diameter of 50 nanometers to 20 micrometers. In a further embodiment the microparticles have a diameter of between 100 nm and 10 µm. In one embodiment the microparticles used in a method according to the present disclosure have a diameter of 200 nm to 5 µm or from 750 nm to 5 µm.

Microparticles as defined herein above may comprise or consist of any suitable material known to the person skilled in the art, e.g. they may comprise or consist of or essentially consist of inorganic or organic material. Typically, they may comprise or consist of or essentially consist of metal or an alloy of metals, or an organic material, or comprise or consist of or essentially consist of carbohydrate elements. Examples of envisaged material for microparticles include agarose, polystyrene, latex, polyvinyl alcohol, silica and ferromagnetic metals, alloys or composition materials. In one embodiment the microparticles are magnetic or ferromagnetic metals, alloys or compositions. In further embodiments, the material may have specific properties and e.g. be hydrophobic, or hydrophilic. Such microparticles typically are dispersed in aqueous solutions and retain a small negative surface charge keeping the microparticles separated and avoiding nonspecific clustering.

In one embodiment of the present invention, the microparticles are paramagnetic microparticles and the separation of such particles in the measurement method according to the present disclosure is facilitated by magnetic forces. Magnetic forces are applied to pull the paramagnetic or magnetic particles out of the solution/suspension and to retain them as desired while liquid of the solution/suspension can be removed and the particles can e.g. be washed. The microparticles used in a method according to the present invention are coated with the first member of a specific binding pair.

Generally, the term "receptor" denotes any compound or composition capable of recognizing a particular spatial and polar organization of a target molecule i.e. an epitopic site of an analyte. Thus, the term "analyte-specific receptor" as referred to herein includes analyte-specific reactants capable of binding to or complexing an analyte. This includes but is not limited to antibodies, specifically monoclonal antibodies or antibody fragments. Such a receptor can act as a catcher of the analyte, e.g. to immobilize the analyte. An epitope recognized by the antibody is bound, followed by labeled antibodies specific to another epitope of the analyte. Other receptors are known to those of skill in the art. The particular use of various receptors in a receptor-based assay will be understood by those of skill in the art with reference to this disclosure.

An "analyte" can be any molecule which can be bound by an analyte-specific receptor. In one embodiment, an analyte within the context of the present disclosure is a nucleic acid (DNA or RNA) molecule, a peptide, a protein, a drug molecule, a hormone or a vitamin. In one embodiment, an analyte within the context of the present disclosure is a peptide, a protein, a drug molecule, a hormone or a vitamin. In another embodiment, an analyte comprises several variants, in an embodiment different genotypes, isoenzymes, isoforms, serotypes or mutants of an analyte. In an embodiment, an analyte is an antigen of an infectious agent. Examples of infectious agents are viruses, bacteria and protozoic pathogens that infect humans. In an embodiment, an analyte is a viral antigen, in an embodiment a hepatitis virus antigen or a human retroviral antigen. In an embodiment, an analyteis a hepatitis C virus or hepatitis B virus or HIV antigen.

In context of the present disclosure, the term "antibody" relates to full immunoglobulin molecules, specifically IgMs, IgDs, IgEs, IgAs or IgGs, as well as to parts of such immunoglobulin molecules, like Fab-fragments or V_{L}-, V_{H}- or CDR-regions. Furthermore, the term relates to modified and/or altered antibody, like chimeric and humanized antibodies. The term also relates to modified or altered monoclonal or polyclonal antibodies as well as to recombinantly or synthetically generated/synthesized antibodies. The term also relates to intact antibodies as well as to antibody fragments/parts thereof, like, separated light and heavy chains, Fab, Fab/c, Fv, Fab', F(ab')₂. The term "antibody" also comprises antibody derivatives, bifunctional antibodies and antibody constructs, like single chain Fvs (scFv), bispecific scFvs or antibody-fusion proteins.

A "detectable label" includes a moiety that is detectable or that can be rendered detectable. The skilled person knows a label as a compound or composition capable of providing a detectable signal in conjunction with physical activation (or excitation) or chemical reagents and capable of being modified, so that the particular signal is diminished or increased.

Specific embodiments of a detectable label include labels which are detectable by a number of commercially available instruments that utilize electrochemiluminescence (ECL) for analytical measurements. Species that can be induced to emit ECL (ECL-active species) have been used as ECL labels. Examples of ECL labels include: i) organometallic compounds where the metal is from, for example, the noble metals of group VIII, including Ru-containing and Os containing organometallic compounds such as the tris-bipyridyl-ruthenium (RuBpy) moiety and ii) luminol and related compounds. Species that participate with the ECL label in the ECL process are referred to herein as ECL coreactants. Commonly used coreactants include tertiary amines (e.g., see US5,846,485), oxalate, and persulfate for ECL from RuBpy and hydrogen peroxide for ECL from luminol (see, e.g., US5,240,863. The light generated by ECL labels can be used as a reporter signal in diagnostic procedures (Bard et al., US5,238,808 ). For instance, an ECL label can be covalently coupled to a binding agent such as an antibody, nucleic acid probe, receptor or ligand; the participation of the binding reagent in a binding interaction can be monitored by measuring ECL emitted from the ECL label. Alternatively, the ECL signal from an ECL-active compound may be indicative of the chemical environment (see, e.g., US5,641,623 which describes ECL assays that monitor the formation or destruction of ECL coreactants). For more background on ECL, ECL labels, ECL assays and instrumentation for conducting ECL assays see US5,093,268; US5,147,806; US5,324,457; US5,591,581; US5,597,910; US5,641,623; US5,643,713; US5,679,519; US5,705,402; US5,846,485; US5,866,434; US5,786,141; US5,731,147; US6,066,448; US6,136,268; US5,776,672; US5,308,754; US5,240,863; US6,207,369 and US5,589,136; and WO99/63347, WO00/03233, WO99/58962, WO99/32662, WO99/14599, WO98/12539, WO97/36931 and WO98/57154.

In line with general knowledge in the field of biochemistry, a "binding pair" is understood as being a set of two different partners, i.e. a first and a second partner or species of the partners or partner species, or a first and a second member of the pair, or a first and a second species. Under non-denaturing conditions a partner is capable of specifically recognizing the partner of the other species on the molecular level. Upon recognition, the partners of the binding pair form a stable non-covalent intermolecular bond connecting the first partner with the second partner. When selecting partner species to create a binding pair it is important that each partner does not form a bond with another partner of the same species. That is to say no stable intramolecular bond between two first partners or two second partners should be formed.

Throughout this document between a first and a second member of a binding pair the punctuation mark (":") can be used to denote the specific connection, or the capability to form such a specific connection, of a first member and a second member of a binding pair, thus being represented by "member1:member2". Typically, the first and the second member belong to different species, i.e. first member and the second member are not identical compounds. Accordingly, depending on context, "member1:member2" can mean that member1 and member2 can form a binding pair, and that member1 is capable of specifically recognizing and bind to member2; or, depending on context, "member1:member2" can mean that member1 and member2 are a connected pair. It is also understood that unless specifically described differently, a member includes not only the member as an isolated compound but also the member being attached to another entity, e.g. forming a moiety of the other entity. By way of example, the "(strept)avidin:biotin" (= "biotin:(strept)avidin") binding pair is perfectly known to the person skilled in the art. A biotin or a biotin moiety on the one hand and (strept)avidin or (strept)avidin coupled to another structure on the other hand represent the two members of this exemplary binding pair.

A single-stranded "nucleic acid" is a polymer composed of monomeric units of nucleotides. Each nucleotide that makes up a nucleic acid is comprised of phosphoric acid, sugar, and nucleobase. The chains of nucleotides in a nucleic acid are linked by 3', 5' phosphodiester linkages. This means that the 5'-phosphoric group of one nucleotide is esterified with the 3'-hydroxyl of the adjoining nucleotide.

A single-stranded "oligonucleotide" is a short nucleic-acid usually consisting of up to approximately 15 nucleotide monomers which are connected by phosphodiester bonds between the 3' carbon atom of one sugar molecule and the 5' carbon atom of another. The monomers comprised in an oligonucleotide (in a generic sense) can not only be naturally occurring monomers but also non-naturally occurring monomers, also referred to as nucleotide analogs. In a non-limiting way, exemplary analogs comprise sugar moieties other than ribose or deoxyribose, particularly a ribose in which the ring of the sugar is "locked" by a methylene bridge connecting the 2'-O atom and the 4'-C atom. For the purpose of the present disclosure, the term nucleotide encompasses naturally and non-naturally occurring nucleotides as monomers in an oligonucleotide. Thus, an oligonucleotide according to this definition can be composed of naturally or non-naturally occurring monomers exclusively, or it can be composed of a mixture thereof. In addition, it is understood that different classes of non-naturally occurring monomers (e.g. PNA, D-LNA, L-LNA, homo-DNA (with hexose sugar), HNA (with hexitol sugar, hexitol nucleic acid), L-DNA, etc.) can be comprised in an oligonucleotide, if not stated otherwise.

A non-naturally occurring monomer can include a nucleobase which itself can be a naturally-occurring nucleobase or a non-naturally occurring analog thereof. A "nucleobase" is a nitrogen-containing unsaturated hydrocarbon compound and includes a planar heterocyclic moiety. The naturally occurring nucleobases can be grouped into two major forms: purines and pyrimidines. While both purines and pyrimidines are heterocyclic aromatic compounds, they can be distinguished from each other based on the chemical structure. The purines occur as two carbon rings whereas the pyrimidines occur as one carbon ring. The purine has a pyrimidine ring fused to an imidazole ring. The pyrimidine has only a pyrimidine ring, and the purine has four nitrogen atoms whereas the pyrimidine has two. A nucleobase forms a nucleoside when it is attached to a sugar moiety which typically is a five-carbon ribose or deoxyribose, or a derivative thereof (e.g. a locked ribose). Thus, nucleosides are glycosylamines and include e.g. cytidine, uridine, adenosine, guanosine, thymidine and inosine. In these examples the anomeric carbon of the five-carbon sugar is linked through a glycosidic bond to the N9 of a purine or the N1 of a pyrimidine.

A nucleoside is a component of a nucleotide which further includes a phosphate moiety or a derivative or functional analog thereof. A nucleotide is the monomeric unit of a single-stranded nucleic acid. In double-stranded nucleic acids like DNA, the nucleobases are paired. The two nucleobases that are complementary are connected by a hydrogen bond.

The term "nucleobase" comprises canonical and non-canonical naturally occurring nucleobases and analogs thereof. A large number of non-naturally occurring nucleobases are known. For the purpose of the present disclosure those nucleobase analogs are specifically considered as embodiments, wherein a nucleobase analog being part of a first oligonucleotide strand can form one or more hydrogen bonds with another adjacent nucleobase in a second oligonucleotide strand, wherein the two oligonucleotide strands are paired and form a duplex, specifically an antiparallel duplex. Typically, the nucleobases of a base pair in a duplex are in planar orientation. For the purpose of the present disclosure, a non-limiting compilation of nucleobases includes a compound selected from the group consisting of N⁴-acetylcytosine, 5-acetyluracil, 4-amino-6-chloropyrimidine, 4-amino-5-fluoro-2-methoxypyrimidine, 6-amino-1-methyluracil, 5-aminoorotic acid, 5-aminouracil, 6-aminouracil, 6-azauracil, N⁴-benzoylcytosine, 5-bromouracil, 5-chlorouracil, 6-chlorouracil, 6-chloromethyluracil, 6-chloro-3-methyluracil, cytosine, 5,6-dimethyluracil, 5-ethyluracil, 5-ethynyluracil, 5-fluorocytosine, 5-fluoroorotic acid, 5-fluorouracil, 5-iodo-2,4-dimethoxypyrimidine, 5-iodouracil, isocytosine, 5-methylcytosine, 6-methyl-5-nitrouracil, 2-methylthio-4-pyrimidinol, 5-methyl-2-thiouracil, 6-methyl-2-thiouracil, 6-methyluracil, 5-nitrouracil, orotic acid, 6-phenyl-2-thiouracil, 6-propyl-2-thiouracil, 2-thiouracil, 4-thiouracil, thymine, 5-(trifluoromethyl)uracil, uracil, adenine, 8-azahypoxanthine, 8-azaguanine, allopurinol, 4-aminopyrazolo [3,4-*d*]pyrimidine, 2-aminopurine, 2-acetamido-6-hydroxypurine, 2-amino-6-chloropurine, 2-amino-6-iodopurine, azathioprine, 4-amino-6-hydroxypyrazolo [3,4-*d*]pyrimidine, aminophylline, N⁶-benzyladenine, N⁶-benzoyladenine, 6-benzyloxypurine, 8-bromotheophylline, 8-bromo-3-methylxanthine, 8-bromo-7-(2-butyn-1-yl)-3-methylxanthine, 6-chloropurine, 8-chlorotheophylline, 6-chloro-2-fluoropurine, 6-chloro-7-deazapurine, 2-chloroadenine, 6-chloro-7-iodo-7-deazapurine, 2,6-diaminopurine, 2,6-dichloropurine, 6-(dimethylamino)purine, 2,6-dichloro-7-deazapurine, 5,6-dichlorobenzimidazole hydrochloride, 7-deazahypoxanthine, 2-fluoroadenine, guanine, hypoxanthine, , isoguanine, 3-iodo-1*H*-pyrazolo-[3,4-d]pyrimidin-4-amine, kinetin, 6-mercaptopurine, 6-methoxypurine, 3-methylxanthine, 1-methylxanthine, 3-methyladenine, O⁶-(cyclohexylmethyl)guanine, 6-thioguanine, 2-thioxanthine, xanthine, 5-propynyl-uracil, 5-propynyl-cytidine, 7-deazaadenine, 7-deazaguanine, 5-propynyl-uracil, 5-propynyl-cytidine, 7-deazaadenine, 7-deazaguanine, 7-propynyl-7-deazaadenine, 7-propynyl-7-deazaguanine, and a derivative thereof.

Complementary single-stranded oligo- or polynucleotides can form a double-stranded ("duplex") nucleic acid. Duplex formation is also known by the term "hybridization" denoting the formation of a partially or completely double-stranded (duplex) nucleic acid (e.g. DNA:DNA, DNA:RNA, RNA:RNA, LNA:DNA, LNA:LNA, etc.) by sequence-specific interaction of two at least partially complementary single-stranded nucleic acids as embodiments of a binding pair. Association of complementary single-stranded nucleic acids or renaturation of separated (denatured) double strands are often used to describe hybridization between completely complementary strands.

Hybridization in aqueous solution, also known as "annealing", is an integral part of the present disclosure. With respect to duplex molecules of hybridized oligo- or polynucleotides (including the analogs thereof), the skilled person appreciates that melting temperatures, hybridization rates, and dissociation rates and temperatures are interrelated.

In line with common knowledge, a "Watson-Crick base pair" is a single non-covalent cross-link in a double-stranded nucleic acid helix (= duplex), wherein each single strand of the duplex is an oligonucleotide. Thus, in an exemplary embodiment of a duplex the two oligonucleotide strands are cross-linked by pairs of purine and pyrimidine bases projecting inward from the oligonucleotide backbone sugars, and joined by hydrogen bonds with e.g. adenine paired with thymine and with cytosine paired with guanine. In line with the above, a nucleobase can be a naturally occurring nucleobase or an analog thereof, as long as long as a pair of nucleobases can complementarily interact, thereby forming a single non-covalent cross-link in a double-stranded nucleic acid helix (duplex).

It is common knowledge that secondary structure motifs in single-stranded nucleic acids generally impair intended hybridization reactions (e.g. discussed by Koehler R.T. & Peyret N. Comput Biol Chem. 29 (2005) 393-397). This finding also applies to ss-oligonucleotides, including single-stranded LNA oligonucleotides. A secondary structure can arise by internal folding of the single-stranded molecule, driven by intramolecular interactions such as hydrogen bonds or hydrophobic interactions. In the particular case of a first single-stranded oligonucleotide a certain folded structure can be thermodynamically favoured, wherein the folded structure then interferes with unencumbered presentation of the nucleobase sequence to that of a complementary second oligonucleotide. Thus, efforts are necessary to predict and avoid such structures. Vice versa, the secondary structure at a targeted binding site may also impair hybridization. Thus, evaluation of the secondary structures of both partners of a binding pair consisting of oligonucleotides is necessary. Several challenges confound this goal, including imperfect empirical rules and parameters underlying predictions, and the fact that folding algorithms scale poorly with respect to sequence length.

In addition, among members of the same oligonucleotide species, i.e. oligonucleotides sharing identical nucleobase sequence, there may be one or more portions which could be partially complementary, thereby potentially giving rise to intermolecular interaction by Watson-Crick base pairing of one or more nucleobases. Otherwise, there could also be one or more portions within the sequence which could give rise to intermolecular interaction by non-Watson-Crick (e.g. Hoogsteen) base pairing, or by other forms of intermolecular interaction. As in the case of intramolecular folding (see above), intramolecular interactions among members of a first single-stranded oligonucleotide can result in thermodynamically favoured structures, wherein such structures then interfere with unencumbered presentation of the nucleobase sequence to that of a complementary second oligonucleotide.

If unwanted inter- or intramolecular structures occur they can be eliminated by denaturation. From the use of ss-oligonucleotides in technical field of polymerase chain reaction (PCR) it is known that an annealing step is typically preceded by a step of heating, whereby in the heating step the oligonucleotides are denatured, i.e. intramolecular secondary structures are broken up. Upon the heating step typically follows a gradual and controlled temperature reduction intended to provide suitable conditions of annealing between oligonucleotide and target sequence. However, PCR is a process which involves sufficiently thermostable reaction partners, e.g. oligonucleotide primers, nucleoside triphosphates, salts, buffers, and thermostable polymerase enzymes. But other processes in which annealing of oligonucleotides could play a role are prohibitive to applying heat or other kinds of denaturing treatment because such processes may include denaturation-sensitive components which might irreversibly degrade. This is particularly (but not exclusively) the case for analyte detection assays in which proteinaceous analyte-specific receptors such as antibodies play a key functional role. The present disclosure and report of surprising findings therefore specifically deal with technical settings in which application of heat, specifically incubation at a temperature above 68 °C (as in Childs J.L. PNAS 99 (2002) 11091-11096), is not possible. For practical reasons, in most assays such as but not limited to immunoassays a temperature above 40 °C is not desired. That is to say, for the practical use of a binding pair consisting of complementary oligonucleotides, particularly desired conditions are from 0 °C to 40 °C. And under these conditions any technical application must be unaffected by intra- or intermolecular structures that could possibly be the case with regard to an isolated binding partner of an oligonucleotide binding pair.

Other options for denaturation of nucleic acids including oligonucleotides are known to the skilled person from reports on DNA. Several methods of DNA denaturation are known to the art, including heating, incubation under alkaline conditions equivalent to more than 0.01 mol/L NaOH in water (pH 12 or higher), incubation in the presence of dimethyl sulfoxide (DMSO), incubation in the presence of formamide, incubation in the presence of a chaotropic compound, incubation in the presence of sonication. While it remains to be shown that such treatments not only provide conditions for making and/or stabilizing ss-DNA but also ss-LNA, it is nevertheless clear that they are not desired in assays to detect an analyte in which proteinaceous analyte-specific receptors such as antibodies play a key functional role.

Thus, a "denaturing condition" which on the one hand might be capable of counteracting an undesired intra- and intermolecular structure in a species of an all-LNA ss-oligonucleotide in aqueous solution, but which on the other hand is to be avoided in any aspect and embodiment of the technical approach presented in the present report, is selected from the group consisting of application of a temperature higher than 40 °C, application of a temperature higher than 68 °C (heat), application of sonication, incubation under alkaline conditions equivalent to more than 0.01 mol/L NaOH in water, incubation in the presence of dimethyl sulfoxide (DMSO) at a concentration capable of breaking the intra- and intermolecular structure, incubation in the presence of formamide at a concentration capable of breaking the intra- and intermolecular structure, incubation in the presence of a chaotropic compound at a concentration capable of breaking the intra- and intermolecular structure, and a mixture thereof. For the purpose of the present report, an embodiment of the absence of a denaturing condition (= an embodiment under non-denaturing conditions) is the absence and/or lack of any of application of a temperature higher than 40 °C, application of a temperature higher than 68 °C (heat), application of sonication, incubation under alkaline conditions equivalent to more than 0.01 mol/L NaOH in water, incubation in the presence of dimethyl sulfoxide (DMSO) at a concentration capable of breaking the intra- and intermolecular structure, incubation in the presence of formamide at a concentration capable of breaking the intra- and intermolecular structure, incubation in the presence of a chaotropic compound at a concentration capable of breaking the intra- and intermolecular structure, and a mixture thereof.

Importantly, under non-denaturing conditions each partner of the binding pair is desired not to form any intramolecular bond which would render it incapable of forming a bond with a partner of the other species. As explained before, and by way of example, in such an undesired case, intramolecular folding and stabilization of a certain fold in a partner species would lead to a secondary structure which under non-denaturing conditions would be stable enough to inhibit or prevent the desired intramolecular bonding of the two different species of the binding pair.

All-LNA ss-oligonucleotides comprising 5 or more monomers have features which cannot be reliably predicted by the present tools that are available to the skilled person, taking into account non-denaturing conditions, and specifically excluding any application of a denaturing treatment detailed herein. For practical reasons, the present study was limited to ss-oligonucleotides consisting of up to 15 LNA monomers to identify those ss-oligonucleotides which in the absence of denaturing conditions are capable of forming a hybridized duplex from two separate single-stranded species. That is to say, each member of a binding pair must be sufficiently devoid of any inter- or intramolecular structures. That such a case cannot be taken for granted regarding complementary all-LNA oligonucleotides is shown in the present report.

Accordingly, the present disclosure, in a first aspect being related to all other aspects and embodiments as disclosed herein, unexpectedly provides a pair of separate ss-oligonucleotides, each oligonucleotide consisting of 5 to 15 LNA monomers, the separate ss-oligonucleotides in aqueous solution being capable of forming with each other an antiparallel duplex in the absence of denaturing conditions prior to duplex formation. Independent from any theoretical and/or computer-implemented model with uncertain reliability, such a pair can unexpectedly be identified and provided by a method of the second aspect as follows. To the knowledge of the authors of this report, such a method has not been shown or even suggested previously.

Thus, the present disclosure, in a second aspect being related to all other aspects and embodiments as disclosed herein, provides a method for selecting and providing a binding pair of single-stranded all-LNA oligonucleotides capable of forming in aqueous solution at a temperature from 0 °C to 40 °C an antiparallel duplex with 5 to 15 consecutive base pairs, the method comprising the steps of
(a) providing a first single-stranded (= ss-) oligonucleotide consisting of 5 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 5 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 5 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

In an embodiment, specifically the steps (c) and (d) are performed in the absence of a condition specified as a "denaturing condition" as described above.

A specific embodiment of this second aspect, the specific embodiment being related to all other aspects and embodiments as disclosed herein, provides a method for selecting and providing a binding pair of single-stranded all-LNA oligonucleotides capable of forming in aqueous solution at a temperature from 0 °C to 40 °C an antiparallel duplex with 5 to 7 consecutive base pairs, the method comprising the steps of
(a) providing a first single-stranded (= ss-) oligonucleotide consisting of 5 to 7 locked nucleic acid (= LNA) monomers, each monomer comprising a nucleobase, the nucleobases of the first ss-oligonucleotide forming a first nucleobase sequence;
(b) providing a second ss-oligonucleotide consisting of 5 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 5 to 7 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

In an embodiment, specifically the steps (c) and (d) are performed in the absence of a condition specified as a "denaturing condition" as described above.

Each single-stranded oligonucleotide consists of monomers, wherein each monomer is a ribonucleoside analog, wherein in the ribose moiety of the ribonucleoside analog a methylene connects the 2'-oxygen and 4'-carbon atom and thereby locks the ribose into C3*-endo* conformation. An all-LNA ss-oligonucleotides according to the present disclosure can be chemically synthesized using building blocks such as protected phosphoramidites of single LNA nucleosides using standard techniques.

Each LNA monomer comprises a nucleobase, wherein the nucleobase is selected from a canonical or non-canonical naturally occurring nucleobase and a nucleobase which does not naturally occur. In an embodiment a LNA monomer comprises a nucleobase selected from the group consisting of N⁴-acetylcytosine, 5-acetyluracil, 4-amino-6-chloropyrimidine, 4-amino-5-fluoro-2-methoxypyrimidine, 6-amino-1-methyluracil, 5-aminoorotic acid, 5-aminouracil, 6-aminouracil, 6-azauracil, N⁴-benzoylcytosine, 5-bromouracil, 5-chlorouracil, 6-chlorouracil, 6-chloromethyluracil, 6-chloro-3-methyluracil, cytosine, 5,6-dimethyluracil, 5-ethyluracil, 5-ethynyluracil, 5-fluorocytosine, 5-fluoroorotic acid, 5-fluorouracil, 5-iodo-2,4-dimethoxypyrimidine, 5-iodouracil, isocytosine, 5-methylcytosine, 6-methyl-5-nitrouracil, 2-methylthio-4-pyrimidinol, 5-methyl-2-thiouracil, 6-methyl-2-thiouracil, 6-methyluracil, 5-nitrouracil, orotic acid, 6-phenyl-2-thiouracil, 6-propyl-2-thiouracil, 2-thiouracil, 4-thiouracil, thymine, 5-(trifluoromethyl)uracil, uracil, adenine, 8-azahypoxanthine, 8-azaguanine, allopurinol, 4-aminopyrazolo [3,4-*d*]pyrimidine, 2-aminopurine, 2-acetamido-6-hydroxypurine, 2-amino-6-chloropurine, 2-amino-6-iodopurine, azathioprine, 4-amino-6-hydroxypyrazolo [3,4-*d*]pyrimidine, aminophylline, N⁶-benzyladenine, N⁶-benzoyladenine, 6-benzyloxypurine, 8-bromotheophylline, 8-bromo-3-methylxanthine, 8-bromo-7-(2-butyn-1-yl)-3-methylxanthine, 6-chloropurine, 8-chlorotheophylline, 6-chloro-2-fluoropurine, 6-chloro-7-deazapurine, 2-chloroadenine, 6-chloro-7-iodo-7-deazapurine, 2,6-diaminopurine, 2,6-dichloropurine, 6-(dimethylamino)purine, 2,6-dichloro-7-deazapurine, 5,6-dichlorobenzimidazole hydrochloride, 7-deazahypoxanthine, 2-fluoroadenine, guanine, hypoxanthine, 9-(2-hydroxyethyl)adenine, isoguanine, 3-iodo-1*H*-pyrazolo-[3,4-d]pyrimidin-4-amine, kinetin, 6-mercaptopurine, 6-methoxypurine, 3-methylxanthine, 1-methylxanthine, 3-methyladenine, O⁶-(cyclohexylmethyl)guanine, 6-thioguanine, 2-thioxanthine, xanthine, 5-propynyl-uracil, 5-propynyl-cytidine, 7-deazaadenine, 7-deazaguanine, 7-propynyl-7-deazaadenine, 7-propynyl-7-deazaguanine, and a derivative thereof.

An all-LNA ss-oligonucleotide according to all aspects and embodiments as disclosed herein may contain a number of monomers, the number being selected from the group consisting of 5, 6, 7, 8, 9, 10, 11, 12, 13, 14, and 15. In an embodiment of all aspects and embodiments as disclosed herein, the first ss-oligonucleotide consists of 8 to 15 monomers (i.e. a number selected from 8, 9, 10, 11, 12, 13, 14, and 15 monomers). In yet another embodiment of all aspects and embodiments as disclosed herein, the first ss-oligonucleotide consists of 9 to 11 monomers (i.e. a number selected from 9, 10, and 11 monomers), and in a more specific embodiment of all aspects and embodiments as disclosed herein, the first ss-oligonucleotide consists of 9 monomers.

Initial experiments used these oligo sizes, in order to provide a combination of, firstly, a high binding specificity for the binding partners, secondly, a favourable speed with which the two partners of the binding pair hybridize and form a duplex, and, thirdly, formation of a stable duplex wich has no substantial detectable tendency to dissociate again into single strands. Surprisingly it was found, that even a binding pair of all-LNA ss-oligonucleotides with a complementary nucleobase sequence consisting of 7, 6 and even 5 consecutive LNA monomers could satisfy the criteria of sufficient specificity, pairing speed and duplex stability. Even more surprisingly, these criteria were met under specific ambient conditions which are a prerequisite for using the binding pair of all-LNA ss-oligonucleotides as a means of molecular recognition in assays for detection of a target analyte such as, but not limited to, immunoassays. So a specific embodiment that is related to all other aspects disclosed herein is a method for selecting and providing a binding pair of single-stranded all-LNA oligonucleotides capable of forming in aqueous solution at a temperature from 0 °C to 40 °C an antiparallel duplex with 5, 6 or 7 consecutive base pairs, the method being a specific embodiment as described.

Another specific embodiment that is related to all other aspects and embodiments disclosed herein is a pair of separate ss-oligonucleotides, each oligonucleotide consisting of 5 to 7 LNA monomers, the separate ss-oligonucleotides in aqueous solution being capable of forming with each other an antiparallel duplex in the absence of denaturing conditions prior to duplex formation. Another embodiment related to all other aspects is a pair of ss-oligonucleotides sharing a complementary nucleobase sequence of 5, 6, or 7 LNA monomers comprised in each ss-oligonucleotide, the binding pair being capable of forming in aqueous solution and in the absence of denaturing conditions an all-LNA duplex. As explained above, already, absence of denaturing conditions means that neither any of the ss-oligonucleotides is subjected to denaturation prior to duplex formation, nor is denaturing treatment part of the incubation after the members of the binding pairs are contacted with each other in aqueous solution.

Shorter LNA oligomers can be less complex and are more economical to synthesize, and they provide an ideal source of complementary binding partners for molecular recognition in aqueous solution.

In a particular embodiment relating to all aspects of this report, a member of the binding pair is an all-LNA ss-oligonucleotide, wherein the all-LNA ss-oligonucleotide is a fragment of a larger all-LNA ss-oligonucleotide, the larger ss-oligonucleotide being one member, partner or species of a binding pair which is selected and provided by a method for selecting and providing a binding pair of single-stranded all-LNA, according to the second aspect as provided herein. In a specific embodiment thereof, the larger oligonucleotide comprises 8 to 15 LNA monomers, and the fragment comprises a contiguous nucleobase sub-sequence of the larger fragment, wherein the fragment comprises 5 to 7 LNA monomers. Thus, a binding pair in an embodiment consists of a first and a second all-LNA ss-oligonucleotide, each comprising 5, 6 or 7 LNA monomers, the nucleobase sequences of the first and second all-LNA ss-oligonucleotide being complementary thereby being capable of forming an antiparallel duplex with 5, 6 or 7 base pairs, wherein each member of the binding pair is a fragment of a larger all-LNA ss-oligonucleotide selected and provided by a method according to the second aspect as provided herein. In a specific embodiment, the first and the second all-LNA ss-oligonucleotides consist of 5 monomers, each. In another specific embodiment, the first and the second all-LNA ss-oligonucleotides consist of 6 monomers, each. In yet another specific embodiment, the first and the second all-LNA ss-oligonucleotides consist of 7 monomers, each.

Thus, the present disclosure, in a third aspect being related to particularly the second aspect but also to all other aspects and embodiments as disclosed herein, provides a method for selecting and providing a binding pair of single-stranded all-LNA oligonucleotides capable of forming in aqueous solution at a temperature from 0 °C to 40 °C an antiparallel duplex with 5 to 7 consecutive base pairs, the method comprising the steps of
(a) providing a first single-stranded (= ss-) oligonucleotide consisting of 8 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 8 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 8 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) selecting a first contiguous nucleobase sub-sequence from an oligonucleotide of the binding pair selected in step (f), thereby creating a fragment of the oligonucleotide, the fragment consisting of 5 to 7 LNA monomers;
(h) optionally selecting a second contiguous nucleobase sub-sequence from the other oligonucleotide of the binding pair selected in step (f), wherein the second sub-sequence is complementary to the first sub-sequence of step (g), thereby creating a fragment of the other oligonucleotide, the fragment consisting of 5 to 7 LNA monomers;
(i) synthesizing separately the ss-oligonucleotide fragment of step (g) and the ss-oligonucleotide fragment of step (h);
thereby selecting and providing the binding pair of single-stranded all-LNA oligonucleotides.

In an embodiment, specifically the steps (c) and (d) are performed in the absence of a condition specified as a "denaturing condition" as described above.

The selected fragments can be readily verified concerning their property of being capable of forming in aqueous solution at a temperature from 0 °C to 40 °C an antiparallel duplex. Therefore, in a specific embodiment, the method includes the additional steps of
(k) mixing in an aqueous solution about equal molar amounts of the first and second ss-oligonucleotide fragment, wherein this step is performed at a non-denaturing temperature, more specifically at a temperature from 0 °C to 40 °C;
(l) incubating the mixture of (k) for a time interval of 20 min or less, thereby obtaining a mixture still comprising the first and second oligonucleotide fragments as ss-oligonucleotides or a mixture containing or consisting of the first and second oligonucleotide fragments as duplex;
(m) selecting the binding pair if in step (l) duplex but no ss-oligonucleotide fragment is detectably present.

The present report provides single-stranded all-LNA oligonucleotides as binding pairs, capable of replacing other binding pairs such as biotin and (strept)avidin. That is to say, related to all aspects and embodiments herein, all binding pairs of ss-oligonucleotides (including the binding pairs which are fragments thereof) which are consisting of LNA monomers and which are obtainable by a method according to the second and/or third aspect presented above, are capable of duplex formation under non-denaturing conditions, specifically. Accordingly, each such ss-oligonucleotide which is provided and optionally stored under non-denaturing conditions is capable of hybridization with its binding partner, under non-denaturing conditions, and retain this quality under non-denaturing conditions.

More specifically, a denaturing condition" in the context of the present disclosure includes as an embodiment any presence or addition of a denaturant which would be capable of lowering the melting temperature of a DNA duplex of 20 base pairs in length and with a G+C content of 50% by 15 °C or more.

Denaturing conditions exclude the conditions under which an assay for detection of a target analyte using a proteinacious analyte-specific receptor must be performed, in order to maintain the required capabilities and functions of these compounds. I.e. the required capabilities and functions of these compounds would be likely lost in the presence of a denaturing condition. At the same time, these non-denaturing conditions exclude any of a temperature higher than 68 °C, application of sonication, incubation under alkaline conditions equivalent to more than 0.01 mol/L NaOH in water, incubation in the presence of dimethyl sulfoxide (DMSO), incubation in the presence of formamide, incubation in the presence of a chaotropic compound, and a mixture thereof, wherein any of these conditions are applied to an extent being capable of breaking an intra- and intermolecular structure of a single all-LNA ss-oligonucleotide, if present, and wherein the intra- and intermolecular structure would be capable of preventing hybridization and duplex formation of the oligonucleotide with a complementary single all-LNA ss-oligonucleotide. Importantly, the all-LNA ss-oligonucleotides taught by the present report do not require any of such denaturing conditions, neither the oligonucleotides in isolated form, nor the oligonucleotides as a binding pair, i.e while one member is contacted with the other.

The first and the second ss-oligonucleotide do not need to be of equal size, i.e. need not consist of an equal number of monomers. However, an equal number of monomers making up the first and the second ss-oligonucleotide is a specific embodiment of all aspects and embodiments as disclosed herein.

Known to the skilled person, two oligonucleotides are antiparallel if they run parallel to each other but with opposite alignments. A specifc example is given by the two complementary strands of a duplex, which run in opposite directions alongside each other. As a consequence, each end of the duplex comprises the 5' end of the first strand next to/aligned with the 3'end of the opposite second strand. Similar to DNA and RNA, LNA exhibits Watson-Crick base pairing (Koshkin, A. A.et al. J Am Chem Soc 120 (1998) 13252-13260).

Specific Watson-Crick base pairing involving hydrogen-bridge forming bases on complementary opposite strands is a feature well known to the skilled person and widely published in the art. Examples include the canonical base pairs adenine:thymine, adenine:uracil, and cytosine:guanine. Other Watson-Crick base pairs include 5-methylcytosine:guanine, 5-hydroxymethylcytosine:guanine, 7-deazaguanine:cytosine, and 5-chlorouracil:7-deazaadenine. Many more are known to the art.

In order to join the two members of the binding pair, one has to be contacted with the other. Following the contacting step, the two members can interact with each other and form a duplex. No denaturing conditions are necessary to this end, as explained above. Importantly, after contacting the two different (i.e. first and second) ss-oligonucleotides (see e.g. step (c) according to the second aspect disclosed above), step (d) of the method as reported herein specifies incubation for a time interval of 20 min or less. That is to say, duplex formation is rapid and, to the extent duplex can be formed, this process is substantially completed within 20 min or less. It should be noted in this regard that in all aspects and embodiments as disclosed herein, the single-stranded all-LNA binding partners (oligonucleotides) are capable of binding to each other under conditions comparable to biotin and (strept)avidin, with specific reference to molecular recognition and binding. In a specific embodiment of all aspects and embodiments as disclosed herein, the time interval for duplex formation (i.e. following the contacting step) is selected from the group consisting of 1 s to 20 min, 1 s to 15 min, 1 s to 10 min, 1 s to 5 min, 1 s to 1 min, 1 s to 30 s, 1 s to 20 s, 1 s to 10 s, and 1 s to 5 s. A very much desired and advantageous time interval is selected from 1 s to 10 s, and 1 s to 5 s.

Importantly, prior to step (c) the first ss-oligonucleotide and the second ss-oligonucleotide do not require denaturing treatment but can be stored or kept under non-denaturing conditions, specifically at a non-denaturing temperature and at the same time undesired consequences are avoided. Particularly, the ss-oligonucleotides as reported in here are characterized by an exceptionally low, if not absent, tendency to stably fold into secondary structures which could interfere with the capability of complementary all-LNA oligonucleotides to form a duplex. In an embodiment of all aspects and embodiments as disclosed herein, the first ss-oligonucleotide and the second ss-oligonucleotide are stored and/or kept at a temperature from -80 °C to 40 °C, specifically from 0 °C to 40 °C, more specifically from 25 °C to 37 °C. A non-denaturing temperature for a single-stranded all-LNA oligonucleotide comprising 5 to 15 monomers is a temperature lower than 68 °C, more specifically lower than 40 °C.

In a specific embodiment of all (specifically the second and third) aspects and embodiments as disclosed herein in step (c) and/or in step (d) the temperature is lower than 68 °C, more specifically the temperature is lower than 40 °C, even more specifically the temperature is from 0 °C to 37 °C. In a method of the second or the third aspect, in step (c) the temperature is selected independently from the temperature in step (d), and vice versa. In a specific embodiment of all aspects and embodiments as disclosed herein, the temperatures in step (c) and (d) do not differ by more than 5 °C, or both steps are performed at the same temperature. In an even more specific embodiment of all aspects and embodiments as disclosed herein in step (c) and/or in step (d) the temperature is from 25 °C to 40 °C, yet more specifically from 25 °C to 37 °C. In another specific embodiment of all aspects and embodiments as disclosed herein, prior to step (c) the first ss-oligonucleotide and the second ss-oligonucleotide are stored and/or kept at a temperature from -80 °C to 40 °C, specifically from 0 °C to 40 °C, more specifically from 25 °C to 37 °C.

In another embodiment of all (specifically the second and third) aspects and embodiments as disclosed herein, prior to step (c) the first ss-oligonucleotide and the second ss-oligonucleotide are stored and/or kept in aqueous solution comprising a buffer maintaining the pH of the solution from pH 6 to pH 8, more specifically from pH 6.5 to pH 7.5.

In yet another embodiment of all (specifically the second and third) aspects and embodiments as disclosed herein, in step (c) the aqueous solution contains a buffer maintaining the pH of the solution from pH 6 to pH 8, more specifically from pH 6.5 to pH 7.5. In another embodiment of all (specifically the second and third) aspects and embodiments as disclosed herein, in step (c) the aqueous solution contains an aggregate amount of dissolved substances from 10 mmol/L to 500 mmol/L, more specifically from 200 mmol/L to 300 mmol/L, more specifically from 10 mmol/L to 150 mmol/L, more specifically from 50 mmol/L to 200 mmol/L.

The conditions described herein which are applied during the steps of mixing (step (c)) and incubating (step (d)) likewise apply to the conditions under which the separate ss-oligonucleotides are kept. Thus, in an embodiment each ss-oligonucleotide of any of the steps (a) and (b) is kept in the absence of denaturing conditions prior to step (c). This includes any embodiment in which each ss-oligonucleotide of any of the steps (a) and (b) is pre-incubated in the absence of denaturing conditions prior to step (c). In an embodiment prior to step (c) each ss-oligonucleotide of any of the steps (a) and (b) is kept in aqueous solution at a temperature from -80 °C to 40 °C, specifically from 0 °C to 40 °C, more specifically from 25 °C to 37 °C . In a further embodiment prior to step (c) each ss-oligonucleotide of any of the steps (a) and (b) is kept in aqueous solution in the absence of a denaturant compound, specifically in the absence of any of formamide and DMSO.

The incubation of step (d) provides conditions of duplex formation with the proviso that the two complementary all-LNA ss-oligonucleotides are in fact capable of molecularly recognizing each other. Further above inter- and intramolecular structures within one or both species of a suspected binding pair are discussed. In case there are e.g. secondary structures in one species prior to step (c), duplex formation in step (d) may be inhibited. The following step (e) is required to find out whether or not duplex formation has occurred in the absence of denaturation. Step (e) detecting and quantifying in the mixture obtained in step (d) ss-oligonucleotides and duplex oligonucleotides. In an embodiment of all aspects and embodiments as disclosed herein, step (e) comprises subjecting the incubated mixture of step (d) to column chromatography with an aqueous solvent as mobile phase. Thus, column chromatography can be used advantageously to separate duplex molecules from ss-oligonucleotides. Suitable column chromatography methods such as HPLC are well known to the skilled person in this regard. However, any alternative method capable of differentiating and quantifying ss-oligonucleotides and duplex is suitable, too. Such an alternative method includes SPR (Surface plasmon resonance; e.g. Biacore) and electrophoresis.

In case inter- and intramolecular structures are present in one or both ss-oligonucleotides prior to the step (c) (which is mixing in an aqueous solution about equal molar amounts of the first and second ss-oligonucleotide), duplex formation is inhibited. If inhibition is complete, only ss-oligonucleotides will be detectably present after step (d) (which is incubating the mixture). However, inhibition may be incomplete. Thus, depending on the strength of the inter- and intramolecular structures, these may temporarily undergo "unfolding", i.e. certain changes causing the nucleobases of an inhibited first ss-oligonucleotide become sufficiently exposed. In an unfolded form, the first ss-oligonucleotide is then capable of forming a duplex with the complementary second ss-oligonucleotide. The same requirement of unfolding may apply to the complementary second ss-oligonucleotide, too. In any such case, the amount of duplex formed reflects the number of unfolding events during the time of incubation, i.e. the incubation time applied in step (d). In the ideal case, following step (d), substantially no ss-oligonucleotides are detectably present, anymore, and only duplex is detectable.

The K_{d} value is the equilibrium dissociation constant between the first and the second member of the binding pair. This value provides a quantitative measurement characterizing the affinity of the binding partners of a binding pair. The equilibrium dissociation constant K_{d} is the ratio of k_{off}/kₒₙ, between the first and the second member of the binding pair. K_{d} and affinity are inversely related. The K_{d} value relates to the concentration of a member that is still sufficient to molecularly interact and bind to the other member; the lower the K_{d} value (lower concentration), the higher the affinity of the first member for the other member. In an embodiment, the affinity K_{d} of the binding partners as selected by a method herein, the binding partners consisting of a first and a second single-stranded oligonucleotide, each consisting of 5 to 15 locked nucleic acid monomers for each other is from < 1x 10⁻¹⁵ M to > 1x 10⁻⁵ M. In a further embodiment, the affinity K_{d} of the binding partners as selected by a method herein, the binding partners consisting of a first and a second single-stranded oligonucleotide, each consisting of 5 to 15 locked nucleic acid monomers for each other is from < 1x 10⁻¹² M to > 1x 10⁻⁵ M. In a further embodiment, the affinity K_{d} of the binding partners as selected by a method herein, the binding partners consisting of a first and a second single-stranded oligonucleotide, each consisting of 8 to 15 locked nucleic acid monomers for each other is from from 2x 10⁻¹² M to 1x10⁻¹⁵ M.

In a further embodiment, the affinity K_{d} of the binding partners as selected by a method herein, the binding partners consisting of a first and a second single-stranded oligonucleotide, each consisting of 5 to 7 locked nucleic acid monomers for each other is from 1x10⁻¹² M to 2x 10⁻⁵ M.

Notably, the K_{d} of the binding partners is substantially influenced by the G+C content in the binding pair. That is to say, for a binding pair with a given number of complementary base pairs generally the affinity of the binding partners becomes with an increasing G+C content.

In order to exclude any interference of a naturally occurring oligonucleotide or polynucleotide with the molecular recognition (i.e. duplex formation) of a binding pair consisting of LNA oligonucleotides, stereoisomers of LNA monomers are advantageously used as building blocks in the synthesis of all-LNA ss-oligonucleotides. The rationale of this approach is to select those stereoisomeric monomers which render the ss-oligonucleotides incapable of forming a duplex with a naturally occurring oligonucleotide or polynucleotide, specifically incapable of forming a Watson-Crick duplex with a naturally occurring oligonucleotide or polynucleotide.

In an embodiment of all aspects and embodiments as disclosed herein, the first and second ss-oligonucleotides (and any fragments thereof) consist of beta-D-LNA monomers. That is to say, the first ss-oligonucleotide entirely consists of beta-D-LNA monomers, and the second ss-oligonucleotide entirely consists of beta-D-LNA monomers. In yet another embodiment of all aspects and embodiments as disclosed herein, the first and second ss-oligonucleotides (and any fragments thereof) consist of beta-L-LNA monomers. That is to say, the first ss-oligonucleotide entirely consists of beta-L-LNA monomers, and the second ss-oligonucleotide entirely consists of beta-L-LNA monomers. Use of beta-L-LNA revealed to be advantageous as duplex formation is not disturbed in the presence of naturally occurring nucleic acids. In this regard it is noted that presently the wealth of technical experience with hybridization conditions for all-D-LNA and all-L-LNA oligonucleotide pairs is rather limited.

The present report discloses a pair of separate ss-oligonucleotides each oligonucleotide consisting of 5 to 15 LNA monomers, the separate ss-oligonucleotides in aqueous solution being capable of forming with each other an antiparallel duplex in the absence of denaturing conditions prior to duplex formation or during duplex formation. The teachings of the present report conveniently and advantageously allow to select and provide such ss-oligonucleotides, the monomers of which are LNA monomers. To the knowledge of the authors of this report the methods described herein represent the first successful approach to overcome the limitations of the algorithms known to the art in predicting hybridization features of all-LNA ss-oligonucleotides. The nucleobase sequence of the first ss-oligonucleotide is complementary to the nucleobase sequence of the second ss-oligonucleotide, in order to allow pairing of the two ss-oligonucleotides in an antiparallel orientation to be capable of forming a duplex.

In an embodiment related to all other aspects and embodiments, there is provided a pair of separate ss-oligonucleotides, each oligonucleotide consisting of 5 to 15 LNA monomers, the separate ss-oligonucleotides in aqueous solution being capable of forming with each other an antiparallel duplex in the absence of denaturing conditions prior to duplex formation or during duplex formation, wherein the pair of ss-oligonucleotides is obtainable and/or obtained by performing a method for selecting and providing a binding pair of single-stranded all-LNA oligonucleotides capable of forming in aqueous solution at a temperature from 0 °C to 40 °C an antiparallel duplex with 5 to 15 consecutive base pairs, the method comprising the steps of
(a) providing a first single-stranded (= ss-) oligonucleotide consisting of 5 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 5 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 5 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

In an embodiment related to all other aspects and embodiments, there is provided a pair of separate ss-oligonucleotides, each oligonucleotide consisting of 5 to 7 LNA monomers, the separate ss-oligonucleotides in aqueous solution being capable of forming with each other an antiparallel duplex in the absence of denaturing conditions prior to duplex formation or during duplex formation, wherein the pair of ss-oligonucleotides is obtainable and/or obtained by performing a method for selecting and providing a binding pair of single-stranded all-LNA oligonucleotides capable of forming in aqueous solution at a temperature from 0 °C to 40 °C an antiparallel duplex with 5 to 7 consecutive base pairs, the method comprising the steps of
(a) providing a first single-stranded (= ss-) oligonucleotide consisting of 8 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 8 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 8 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) selecting a first contiguous nucleobase sub-sequence from an oligonucleotide of the binding pair selected in step (f), thereby creating a fragment of the oligonucleotide, the fragment consisting of 5 to 7 LNA monomers;
(h) optionally selecting a second contiguous nucleobase sub-sequence from the other oligonucleotide of the binding pair selected in step (f), wherein the second sub-sequence is complementary to the first sub-sequence of step (g), thereby creating a fragment of the other oligonucleotide, the fragment consisting of 5 to 7 LNA monomers;
(i) synthesizing separately the ss-oligonucleotide fragment of step (g) and the ss-oligonucleotide fragment of step (h);
thereby selecting and providing the binding pair of 5 to 7 single-stranded all-LNA oligonucleotides. In an embodiment, specifically the steps (c) and (d) are performed in the absence of a condition specified as a "denaturing condition" as described above. The selected fragments can be readily verified concerning their property of being capable of forming in aqueous solution at a temperature from 0 °C to 40 °C an antiparallel duplex. Therefore, in a specific embodiment, the method includes the additional steps of
(k) mixing in an aqueous solution about equal molar amounts of the first and second ss-oligonucleotide fragment, wherein this step is performed at a non-denaturing temperature, more specifically at a temperature from 0 °C to 40 °C;
(l) incubating the mixture of (k) for a time interval of 20 min or less, thereby obtaining a mixture still comprising the first and second oligonucleotide fragments as ss-oligonucleotides or a mixture containing or consisting of the first and second oligonucleotide fragments as duplex;
(m) selecting the binding pair if in step (l) duplex but no ss-oligonucleotide fragment is detectably present.

Each single-stranded all-LNA oligonucleotide can contain four different nucleobases. In an embodiment of all aspects and embodiments as disclosed herein, each ss-oligonucleotide contains three different nucleobases. In another embodiment of all aspects and embodiments as disclosed herein, each ss-oligonucleotide contains two different nucleobases. In yet another embodiment of all aspects and embodiments as disclosed herein, each ss-oligonucleotide contains just one nucleobase. In this latter embodiment, all nucleobases in a ss-oligonucleotide are the same.

In an embodiment of all aspects and embodiments as disclosed herein, the nucleobases in each ss-oligonucleotide the G+C content is lower than 75%. In a specific embodiment, the G+C content is lower than a value selected from 74%, 73%, 72%, 71%, and 70%. In yet another embodiment of all aspects and embodiments as disclosed herein, each LNA monomer in the binding pair comprises a nucleobase selected from the group consisting of adenine, thymine, uracil, guanine, cytosine, and 5-methylcytosine. In a more specific embodiment, among the nucleobases in each ss-oligonucleotide each cytosine is replaced by a 5-methylcytosine.

In an embodiment of all aspects and embodiments as disclosed herein, a binding pair of two separate compatible binding partners is a pair of all-LNA ss-oligonucleotides selected from the group consisting of
5' tgctcctg 3' (SEQ ID NO:1) and 5' caggagca 3' (SEQ ID NO:2),
5' tgctcctgt 3' (SEQ ID NO:9) and 5' acaggagca 3' (SEQ ID NO:10),
5' gtgcgtct 3' (SEQ ID NO:11) and 5' agacgcac 3' (SEQ ID NO:12), and
5' gttggtgt 3' (SEQ ID NO:13) and 5' acaccaac 3' (SEQ ID NO:14).

In a specific embodiment, the monomers of the ss-oligonucleotides in any selected pair of the foregoing group are all beta-D-LNA monomers. In yet another specific embodiment, the monomers of the ss-oligonucleotides in any selected pair of the foregoing group are all beta-L-LNA monomers.

Several such pairs of single-stranded all-LNA oligonucleotides have been found and are reported herein as exemplary embodiments, i.e. non-limiting examples illustrating pairs of separate ss-oligonucleotides which are capable of binding to each other by way of hybridization and duplex formation, under non-denaturing conditions. Table 1 provides a non-limiting compilation thereof. It is understood that the listed sequences denote all-LNA nucleosides, i.e. oligonucleotides containing only LNA monomers. Sequences are given in the conventional orientation, i.e. from the 5' terminus to the 3' terminus.

**Table 1**

| SEQ ID NO: | first member | SEQ ID NO: | second member |
|---|---|---|---|
| 33 | cttcc | 34 | ggaag |
| 42 | gctcc | 43 | ggagc |
| 16 | gttggt | 46 | ccaac |
| 16 | gttggt | 19 | caccaac |
| 16 | gttggt | 17 | caacacaccaac |
| 16 | gttggt | 18 | acacaccaac |
| 16 | gttggt | 14 | acaccaac |
| 16 | gttggt | 20 | accaac |
| 40 | ctgtca | 41 | tgacag |
| 44 | tgctcc | 45 | ggagca |
| 35 | tcttcc | 36 | ggaaga |
| 21 | gttggtg | 17 | caacacaccaac |
| 21 | gttggtg | 18 | acacaccaac |
| 21 | gttggtg | 14 | acaccaac |
| 21 | gttggtg | 19 | caccaac |
| 21 | gttggtg | 20 | accaac |
| 1 | tgctcctg | 2 | caggagca |
| 11 | gtgcgtct | 12 | agacgcac |
| 13 | gttggtgt | 17 | caacacaccaac |
| 13 | gttggtgt | 18 | acacaccaac |
| 13 | gttggtgt | 14 | acaccaac |
| 13 | gttggtgt | 19 | caccaac |
| 13 | gttggtgt | 20 | accaac |
| 22 | gttggtgtg | 17 | caacacaccaac |
| 22 | gttggtgtg | 18 | acacaccaac |
| 22 | gttggtgtg | 14 | acaccaac |
| 22 | gttggtgtg | 19 | caccaac |
| 22 | gttggtgtg | 20 | accaac |
| 9 | tgctcctgt | 15 | caggagc |
| 9 | tgctcctgt | 10 | acaggagca |
| 9 | tgctcctgt | 2 | caggagca |
| 22 | gttggtgtg | 30 | cacaccaac |
| 37 | ttctcttcc | 38 | ggaagagaa |
| 23 | gttggtgtgttg | 17 | caacacaccaac |
| 23 | gttggtgtgttg | 18 | acacaccaac |
| 23 | gttggtgtgttg | 14 | acaccaac |
| 23 | gttggtgtgttg | 19 | caccaac |
| 23 | gttggtgtgttg | 20 | accaac |
| 31 | gttggtgtgttggtg | 32 | caccaacacaccaac |
| 28 | aaaaaaaaa | 24 | ttttttttt |
| 28 | aaaaaaaaa | 25 | tttttttt |
| 28 | aaaaaaaaa | 26 | ttttttt |
| 28 | aaaaaaaaa | 27 | tttttt |
| 39 | aaaaaa | 27 | tttttt |

The binding pairs given in Table 1 reflect specific embodiments. It is understood that any reference to a "first" and a "second" member of the binding pair is arbitrary in that the "second" member can equally be regarded as the first member, as long as the second member is in such a case replaced by the "first" member. I.e. references as "first" and "second" presented in the table are arbitrary and the way how they are denoted can be changed. Accordingly and exemplarily, a binding pair (SEQ ID NO:16) : (SEQ ID NO:20) is the same as (SEQ ID NO:20) : (SEQ ID NO:16). In an embodiment of all aspects and embodiments as disclosed herein, a binding pair of two separate compatible binding partners is a pair of all-LNA ss-oligonucleotides selected from the group consisting of
(SEQ ID NO: 1) : (SEQ ID NO:2),
(SEQ ID NO:9) : (SEQ ID NO: 10),
(SEQ ID NO: 11) : (SEQ ID NO: 12),
(SEQ ID NO: 13) : (SEQ ID NO: 14),
(SEQ ID NO:9) : (SEQ ID NO:15),
(SEQ ID NO: 16) : (SEQ ID NO:20),
(SEQ ID NO:21) : (SEQ ID NO: 18),
(SEQ ID NO:21) : (SEQ ID NO:20),
(SEQ ID NO:21) : (SEQ ID NO: 19),
(SEQ ID NO:23) : (SEQ ID NO: 17),
(SEQ ID NO:25) : (SEQ ID NO:28).

In an embodiment of all aspects and embodiments as disclosed herein, the binding pair of two separate compatible binding partners is the pair of all-LNA ss-oligonucleotides of SEQ ID NO:16 and SEQ ID NO:20. Thus, the binding pair is gttggt : accaac.

In a specific embodiment, the monomers of the ss-oligonucleotides in any selected pair of the foregoing group are beta-D-LNA monomers. In yet another specific embodiment, the monomers of the ss-oligonucleotides in any selected pair of the foregoing group are beta-L-LNA monomers.

In contrast, pairs of single-stranded all-LNA oligonucleotides have been found which under non-denaturing conditions are not or not sufficiently capable of duplex formation. Table 2 provides a non-limiting compilation thereof.

**Table 2**

| SEQ ID NO: | first member | SEQ ID NO: | second member |
|---|---|---|---|
| 3 | gcctgacg | 4 | cgtcaggc |
| 5 | ctgcctgacg | 6 | cgtcaggcag |
| 7 | gactgcctgacg | 8 | cgtcaggcagtc |
| 47 | cgtcaggcagttcag | 48 | ctgaactgcctgacg |
| 29 | tttt | 39 | aaaaaa |

The pair given by SEQ ID NO:29 and SEQ ID NO:39 exemplifies a case in which the sequence complexity is minimal, and in which one member of the binding pair comprises only 4 monomers. The binding properties characterizing this particular binding pair were found to be insufficient. One possible interpretation could be that a 4-mer is simply too short and thus only provides insufficient intramolecular interaction with the corresponding single strand. This finding is in marked contrast to the setting in which the 4-mer is replaced by a 6-mer (SEQ ID NO:27 combined with SEQ ID NO:39).

Concerning the other pairs of all-LNA oligonucleotides presented in Table 2, it was found that there is always one binding partner comprising or consisting of the sequence "gcctgacg" (SEQ ID NO:3). So it appears that this particular sequence and its complement negatively influence the capability of such all-LNA oligonucleotides to form a duplex under non-denaturing conditions. This particularly surprising finding can indeed guide the skilled person towards selecting advantageous oligonucleotide pairs of single strands with 8 monomers or more. Thus, an embodiment of all other aspects and embodiments provided herein is a pair of separate ss-oligonucleotides, each oligonucleotide consisting of 8 to 15 LNA monomers, the separate ss-oligonucleotides in aqueous solution being capable of forming with each other an antiparallel duplex in the absence of denaturing conditions prior to duplex formation or during duplex formation, wherein the pair of ss-oligonucleotides is obtainable and/or obtained by performing a method for selecting and providing a binding pair of single-stranded all-LNA oligonucleotides capable of forming in aqueous solution at a temperature from 0 °C to 40 °C an antiparallel duplex with 8 to 15 consecutive base pairs, the method comprising the steps of
(a) providing a first single-stranded (= ss-) oligonucleotide consisting of 8 to 15 (i.e. a number selected from any of 8, 9, 10, 11, 12, 13, 14, and 15) locked nucleic acid (= LNA) monomers, each monomer comprising a nucleobase, the nucleobases of the first ss-oligonucleotide forming a first nucleobase sequence, wherein the first nucleobase sequence does not consist of or comprise a sequence selected from 5' gcctgacg 3' (SEQ ID NO:3) and 5' cgtcaggc 3' (SEQ ID NO:4);
(b) providing a second ss-oligonucleotide consisting of 8 to 15 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 8 to 15 consecutive base pairs, wherein the two bases of each base pair are bound to each other by hydrogen bonds, wherein the second nucleobase sequence does not consist of or comprise a sequence selected from 5' gcctgacg 3' (SEQ ID NO:3) and 5' cgtcaggc 3' (SEQ ID NO:4);
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

By way of a method of the second aspect or a method of the third aspect as disclosed in here, and also by making use of any of its embodiments including but not limited to the binding pairs shown in Table 1, and alternatively or additionally by way of selecting binding pairs excluding a sequence selected from 5' gcctgacg 3' (SEQ ID NO:3) and 5' cgtcaggc 3' (SEQ ID NO:4) when the number of monomers of one or both of the oligonucleotide(s) is 8 to 15, the present disclosure provides an antiparallel all-LNA duplex which is formed, obtainable and/or obtained under non-denaturing conditions at a pre-selected temperature from 25 °C to 40 °C from a non-denatured pair of complementary single-stranded all-LNA oligonucleotides. Such a duplex, in aqueous solution, can be considered a fourth aspect of the present report. Each oligonucleotide strand in the duplex comprises LNA monomers, the number of LNA monomers being selected from the group consisting of 5, 6, 7, 8, 9, 10, 11, 12, 13, 14, and 15, more specifically the number being selected the group consisting of 5, 6, and 7. The duplex is formed by complementary Watson-Crick base pairing, and the number of base pairs in the duplex is a number selected from the group consisting of 5, 6, 7, 8, 9, 10, 11, 12, 13, 14, and 15, more specifically a number selected either selected from any of 8 to 15, or from any of 5 to 7.

The present disclosure, in a fifth aspect being related to all other aspects and embodiments as disclosed herein, provides a liquid composition comprising an aqueous solvent and a binding pair consisting of a first single-stranded oligonucleotide and a second single-stranded oligonucleotide, wherein each oligonucleotide consists of 5 to 15 locked nucleic acid (= LNA) monomers, each monomer comprising a nucleobase, the nucleobases of the monomers forming a first nucleobase sequence of the first oligonucleotide and a second nucleobase sequence of the second oligonucleotide, wherein the first nucleobase sequence and the second nucleobase sequence are selected that the first oligonucleotide and the second oligonucleotide are capable of forming an antiparallel duplex of 5 to 15 consecutive Watson-Crick base pairs at a temperature from 0 °C to 40 °C, and wherein the binding pair is obtainable by a method according to a method of the second or third aspect as disclosed herein.

In a specific embodiment of all aspects and embodiments as disclosed herein, there is provided a liquid composition comprising an aqueous solvent and a binding pair consisting of a first single-stranded oligonucleotide and a second single-stranded oligonucleotide, wherein each oligonucleotide consists of 5 to 15, specifically 5 to 7 or 8 to 15 locked nucleic acid (= LNA) monomers, each monomer comprising a nucleobase, the nucleobases of the monomers forming a first nucleobase sequence of the first oligonucleotide and a second nucleobase sequence of the second oligonucleotide, wherein the first nucleobase sequence and the second nucleobase sequence are selected that the first oligonucleotide and the second oligonucleotide are capable of forming an antiparallel duplex of 5 to 15, specifically 5 to 7 or 8 to 15 consecutive Watson-Crick base pairs at a temperature from 0 °C to 40 °C, and wherein the binding pair is obtainable by a method according to a method of the second or third aspect as disclosed herein.

In another embodiment of all aspects and embodiments as disclosed herein, each oligonucleotide consists of 5 to 15 LNA monomers, wherein the first nucleobase sequence and the second nucleobase sequence are selected that the first oligonucleotide and the second oligonucleotide are capable of forming an antiparallel duplex of 5 or more consecutive Watson-Crick base pairs at a temperature from 0 °C to 40 °C, and wherein the binding pair is obtainable or obtained by a method according to a method of the second or third aspect and an embodiment thereof.

As a different embodiment it was further surprisingly discovered that even longer complementary all-LNA ss-oligonucleotides exist which are capable of forming antiparallel duplex under non-denaturing conditions. Thus, the present disclosure provides a method for selecting and providing a binding pair of single-stranded all-LNA oligonucleotides capable of forming in aqueous solution at a temperature from 0 °C to 40 °C an antiparallel duplex with 16 to 20 consecutive base pairs, the method comprising the steps of
(a) providing a first single-stranded (= ss-) oligonucleotide consisting of 16 to 20 locked nucleic acid (= LNA) monomers, each monomer comprising a nucleobase, the nucleobases of the first ss-oligonucleotide forming a first nucleobase sequence;
(b) providing a second ss-oligonucleotide consisting of 16 to 20 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 16 to 20 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

In an embodiment, specifically the steps (c) and (d) are performed in the absence of a condition specified as a "denaturing condition" as described above.

A specific embodiment of this second aspect, the specific embodiment being related to all other aspects and embodiments as disclosed herein, provides a method for selecting and providing a binding pair of single-stranded all-LNA oligonucleotides capable of forming in aqueous solution at a temperature from 0 °C to 40 °C an antiparallel duplex with 5 to 15 consecutive base pairs, the method comprising the steps of
(a) providing a first single-stranded (= ss-) oligonucleotide consisting of 5 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 16 to 20 LNA monomers, the second ss-oligonucleotide consisting of at least the number of monomers as the first ss-oligonucleotide, each monomer of the second ss-oligonucleotide comprising a nucleobase, the nucleobases of the second ss-oligonucleotide forming a second nucleobase sequence of the second ss-oligonucleotide, the second nucleobase sequence comprising or consisting of a nucleobase sequence complementary to the first nucleobase sequence in antiparallel orientation and, by way of complementarity, predicting the capability of the first and second ss-oligonucleotide to form with each other an antiparallel duplex, the predicted duplex comprising or consisting of 5 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

In an embodiment, specifically the steps (c) and (d) are performed in the absence of a condition specified as a "denaturing condition" as described above.

Exemplary binding pairs of a first and second all-LNA ss-oligonucleotide were identified as reflected in Table 3 as follows. These binding pairs are specific embodiments.

**Table 3**

| SEQ ID NO: | first member | SEQ ID NO: | second member |
|---|---|---|---|
| 59 | cagtggacgacgatagacat | 60 | atgtctatcgtcgtccactg |
| 61 | agaggatcgaggagtacagg | 62 | cctgtactcctcgatcctct |
| 63 | agaaatggacgagatgctaa | 64 | ttagcatctcgtccatttct |
| 65 | actgaacttgtgagaaacgc | 66 | gcgtttctcacaagttcagt |
| 67 | atggagagtcaggcaagttt | 68 | aaacttgcctgactctccat |
| 69 | tgaagatgcgagtgatgaac | 70 | gttcatcactcgcatcttca |

As it turns out, none of the sequences shown in Table 3 contains the subsequence gcctgacg (SEQ ID NO:3) or its complement which are discussed further above.

In an embodiment of all aspects and embodiments as disclosed herein, one ss-oligonucleotide of the binding pair is attached (covalently or non-covalently attached) to a solid phase selected from the group consisting of magnetic bead, paramagnetic bead, synthetic organic polymer (latex) bead, polysaccharide bead, test tube, microwell plate cavity, cuvette, membrane, scaffolding molecule, quartz crystal, film, filter paper, disc and chip. In another embodiment of all aspects and embodiments as disclosed herein, one ss-oligonucleotide of the binding pair is connected (covalently or non-covalently attached) to a molecule selected from the group consisting of peptide, polypeptide, oligonucleotide, polynucleotide, sugar, glycan, hapten, and dye. In yet another embodiment of all aspects and embodiments as disclosed herein, a ss-oligonucleotide is attached covalently to a linker. In yet another embodiment of all aspects and embodiments as disclosed herein, a ss-oligonucleotide is attached covalently to an analyte-specific receptor useful in a receptor-based assay such as, but not limited to, an immunoassay.

In very general terms, an immunoassay provides one or more receptors which are capable of specifically binding to a target analyte. Such receptors can be exemplified by analyte-specific immunoglobulins; hence the name immunoassay. However, for the purpose of the present disclosure, any other type of analyte-specific receptor is considered, too. Thus, the more general term receptor-based assay is appropriate and used synonymously with the term immunoassay.

Thus a sixth aspect related to all other aspects and embodiments as disclosed herein is the use of a pair of single-stranded all-LNA oligonucleotides of the first aspect in a receptor-based assay for determining an analyte, the receptor-based assay comprising an analyte-specific receptor and a solid phase for immobilizing the analyte on the solid phase, wherein the first ss-oligonucleotide of the pair is coupled to the analyte-specific receptor and the second ss-oligonucleotide of the pair is coupled to the solid phase. An embodiment of this aspect is any such use of a pair of single-stranded all-LNA oligonucleotides, wherein the pair of single-stranded all-LNA oligonucleotides is obtainable by means of an embodiment according to any of the second or third aspect of the present report.

In an receptor-based assay an analyte becomes bound to an analyte-specific receptor. The receptor with the bound analyte can be immobilized on the solid phase by way of a duplex that is formed by the pair of single-stranded all-LNA oligonucleotides. Thus, as a result a complex is formed, the complex comprising the solid phase, the duplex, the receptor, and the analyte. In a further step, immobilized analyte can be detected, e.g. using a further analyte-specific binding agent which itself is labeled or whose presence can be detected by other means. In a competitive assay format no further binding agent is necessary. Instead, a pre-determined amount of labeled analyte is added and competes for binding with unlabeled analyte, the presence and/or concentration of which is to be detected.

Typically, the analyte is comprised in a sample, wherein the sample is a complex mixture of different molecules. For the purpose of the present disclosure, a liquid sample is considered. The liquid sample comprises a liquid phase, i.e. it comprises a liquid solvent which usually is an aqueous solvent. In the aqueous solvent a plurality of molecules are present in dissolved state. Thus, in a specific embodiment the sample is in a liquid state of aggregation, and it is a monophasic homogeneous mixture. In another specific embodiment, the sample contains insoluble parts and therefore is a heterophasic mixture, and the analyte is homogeneously distributed in the sample. Typically, the analyte is comprised in the mixture in dissolved form, and in addition one or more further molecules are present in the mixture in dissolved form.

With regards to detection of a target analyte which is present in the liquid sample, or which is suspected to be present therein, in an essential step, the analyte is specifically bound. Specific binding implies that an analyte-specific receptor is present or is added, wherein the receptor has a binding affinity and binding specificity for the analyte which are high for the target analyte and low or absent for the further molecules which are also present in the sample. In a specific embodiment (and exemplifying a large number of existing assays), a compound comprising a receptor capable of specifically binding to the analyte is added to the sample. Importantly, the mixture of the sample and the compound comprising the receptor must provide conditions which are permissive to the specific interaction of the receptor and the target analyte in the sample. This includes that in the mixture the conditions must be permissive to the actual binding of the analyte by the receptor, and they are desired to stabilize the receptor with the bound target analyte. At the same time, the mixture of the sample and compound is desired not to favor or stabilize unspecific binding of further molecules to the receptor, or to the compound comprising the receptor as a whole.

Subsequently, the analyte is immobilized. Immobilization is an important step in the detection process as it allows to separate the analyte from the surrounding complex mixture, specifically from the further molecules of the sample. Immobilization requires a solid phase to which the target analyte becomes attached. Once immobilized, the analyte can be separated from the mixture by way of phase separation. Separated from the mixture (i.e. purified) the analyte is then detected.

Considering a receptor-based assay and the immobilization step there is the need to provide a solid phase and to build a connection between the solid phase and the target analyte. It is desired that the connection builds up in a self-assembly process.

Immunoassays are well-established bioanalytical methods, specific embodiments, in which detection or quantitation of an analyte depends on the reaction of the analyte and at least one analyte-specific receptor, thus forming an analyte:receptor complex. A non-limiting example is the reaction between an antigen and an antibody, respectively.

Thus a seventh aspect related to all other aspects and embodiments as disclosed herein is a method of performing a receptor-based assay for determining an analyte, the method comprising the steps of contacting the analyte with an analyte-specific receptor having attached thereto a first member of a pair of separate ss-oligonucleotides of the first aspect as disclosed herein, and with a solid phase having attached thereto a second member of the pair, incubating thereby forming a complex comprising the solid phase, the analyte-specific receptor bound to the solid phase and the analyte bound to the analyte-specific receptor, wherein an antiparallel duplex is formed, the duplex consisting of the first and the second member of the pair, wherein the duplex connects the analyte-specific receptor and the solid phase in the complex, followed by detecting analyte bound in the complex, thereby determining the analyte. In an embodiment the latter step of detecting can be made, e.g., using a labeled analyte-specific antibody capable of binding to analyte in the complex, also known to the art as a "sandwich" assay.

The specific embodiment of a "sandwich" immunoassay can be used for analytes possessing a plurality of recognition epitopes (i.e. more than one recognition epitopes). Thus, a sandwich assay requires at least two receptors that attach to nonoverlapping epitopes on the analyte. In a "heterogeneous sandwich immunoassay" one of the receptors has the functional role of an analyte-specific capture receptor; this receptor is or (during the course of the assay) becomes immobilized on a solid phase. A second analyte-specific receptor is supplied in dissolved form in the liquid phase. A sandwich complex is formed once the respective analyte is bound by a first and a second receptor (receptor-1:analyte:receptor-2). The sandwich complex is also referred to as "detection complex". Within the detection complex the analyte is sandwiched between the receptors, i.e. in such a complex the analyte represents a connecting element between the first receptor and a second receptor.

The term "heterogeneous" (as opposed to "homogeneous") denotes two essential and separate steps in the assay procedure. In the first step a detection complex containing label is formed and immobilized, however with unbound label still surrounding the complexes. Prior to determination of a label-dependent signal unbound label is washed away from immobilized detection complex, thus representing the second step. In contrast, a homogeneous assay produces an analyte-dependent detectable signal by way of single-step incubation and does not require a washing step.

In a heterogeneous assay the solid phase is functionalized such that it may have bound to its surface the functional capture receptor (the first receptor), prior to being contacted with the analyte; or the surface of the solid phase is functionalized in order to be capable of anchoring a first receptor, after it has reacted with (i.e. bound to) the analyte. In the latter case the anchoring process must not interfere with the receptor's ability to specifically capture and bind the analyte. A second analyte-specific receptor present in the liquid phase is used for detection of bound analyte, i.e. analyte that has been immobilized or which becomes immobilized on the solid phase. Thus, in a immunoassay the analyte is allowed to bind to the first (capture) and second (detector) receptors. Thereby a "detection complex" is formed wherein the analyte is sandwiched between the capture receptor and the detector receptor. In a typical embodiment the detector receptor is labeled prior to being contacted with the analyte; alternatively a label is specifically attached to the detector receptor after analyte binding. With the detection complexes being immobilized on the solid phase the amount of label detectable on the solid phase corresponds to the amount of sandwiched analyte. After removal of unbound label with a washing step, immobilized label indicating presence and amount of analyte can be detected.

Any washing step(s) necessary in a heterogeneous immunoassay require(s) the non-covalent connection of the first binding partner and the second binding partner to be sufficiently stable. However, the extent of required stability of the connection depends on the strength of the washing step(s) to be applied. Importantly and unexpectedly, a binding pair as demonstrated herein is exceptionally well suited to facilitate the immobilization step in an immunoassay. That is to say, in an immunoassay a first binding partner of the binding pair of all-LNA oligonucleotides which is attached to a solid phase, and a second binding partner of the binding pair which is attached to the analyte-specific capture receptor are well suited to facilitate immobilization of the receptor on the solid phase. Likewise such immobilization advantageously works for the capture receptor having bound a target analyte, and for a detection complex, too.

Another well-known embodiment is a competitive immunoassay which in its simplest form differs from the sandwich-type format by the lack of a second detector receptor. In contrast, the sample with the analyte is mixed with an artificially produced labeled analogon that is capable of cross-reacting with the analyte-specific receptor. In the assay the analyte and the analogon compete for binding to a capture receptor which is or becomes immobilized. Following the binding step, the higher the amount of immobilized label, the smaller the amount of the non-labeled analyte that was capable of competing for the capture receptor. Immobilized label is determined after a washing step. So the amount of label that is detectable on the solid phase inversely corresponds to the amount of analyte that was initially present in the sample.

In all aspects and embodiments disclosed herein, the binding force in the duplex formed by the pair of all-LNA oligonucleotides will exceed the any of the binding forces holding together the analyte and any of the analyte-specific (capture and/or detection) receptors which are used in an assay for analyte detection (such as, but not limited to, an immunoassay). In the event that the binding force in the duplex needs to be fine-tuned, binding pairs can be selected such that Watson-Crick pairing regions of different lengths and/or with different base pair composition can be provided. In more general terms, pairs of complementary single-stranded all-LNA oligonucleotides can be provided and selected according to specific technical needs, with respect to a first and a second component to be attached to one another by means of an all-LNA binding pair.

The binding forces in a duplex of Watson-Crick-paired complementary all-LNA oligonucleotides with a given number of paired nucleobases can be fine-tuned by changing individual base pairs in the duplex (e.g. by replacing an A:T base pair with a C:G base pair), or by lengthening or shortening the duplex. In any case, the fine-tuned pair of single-stranded all-LNA needs to be subjected to a method for selecting and providing a binding pair of single-stranded all-LNA oligonucleotides capable of forming in aqueous solution at a temperature from 0 °C to 40 °C an antiparallel duplex with 5 to 15 consecutive base pairs, as disclosed elsewhere in this document.

Yet, an eighth aspect related to all other aspects and embodiments as disclosed herein is a kit for performing a receptor-based assay for determining an analyte, the kit comprising in a first container an analyte-specific receptor having attached thereto a first member of a pair of separate ss-oligonucleotides according to the first aspect as disclosed herein, or a first member of a pair of separate ss-oligonucleotides obtained by a method according to the secod or the third aspect as disclosed herein, the kit further comprising in a second container a solid phase having attached thereto a second member of the pair.

Even in more general terms, a kit for non-covalently connecting a first and a second component is provided, wherein the kit comprises in a first separate compartment the first component having attached thereto a first member of a pair of separate ss-oligonucleotides according to the first aspect as disclosed herein, or a first member of a obtained by a method according to the secod or the third aspect as disclosed herein, the kit further comprising in a second container the second component having attached thereto a second member of the pair.

It is understood that the binding pairs disclosed in the present report can serve as a general replacement for established binding pairs such as biotin:(strept)avidin. Thus, the all-LNA binding pairs can serve to connect a first and a second component attached respectively to a first and a second member of a complementary pair of ss-oligonucleotides capable of forming a duplex under non-denaturing conditions. The skilled person is well aware of a plethora of applications which go beyond analyte detection assays as described herein in more detail, buit which also extend to e.g. in situ analysis of target antigens in tissue samples.

In principle, the binding pairs of the present disclosure also make it possible to provide separate different binding pairs in the same aqueous solution, thereby opening the way to multiplexed assays. By virtue of having identified different binding pairs of oligonucleotides which do not share complementary base sequences many different additional applications become feasible.

Specific aspects and embodiments include the following more formalized list of items. This list of numbered items forms part of the original disclosure of the present report.
1. A method for selecting and providing a binding pair of single-stranded all-LNA oligonucleotides capable of forming in aqueous solution at a temperature from 0 °C to 40 °C an antiparallel duplex with 5 to 15 consecutive base pairs, the method comprising the steps of
   (a) providing a first single-stranded (= ss-) oligonucleotide consisting of 5 to 15 locked nucleic acid (= LNA) monomers, each monomer comprising a nucleobase, the nucleobases of the first ss-oligonucleotide forming a first nucleobase sequence;
   (b) providing a second ss-oligonucleotide consisting of 5 to 15 LNA monomers, the second ss-oligonucleotide comprising at least the number of monomers as the first ss-oligonucleotide, each monomer of the second ss-oligonucleotide comprising a nucleobase, the nucleobases of the second ss-oligonucleotide forming a second nucleobase sequence, the second nucleobase sequence comprising or consisting of a nucleobase sequence complementary to the first nucleobase sequence in antiparallel orientation and predicting the capability of the first and second ss-oligonucleotide to form with each other an antiparallel duplex, the predicted duplex comprising or consisting of 5 to 15 consecutive base pairs, wherein the two bases of each base pair are bound to each other by hydrogen bonds;
   (c) mixing in an aqueous solution about equal molar amounts of the first and second ss-oligonucleotide, wherein this step is performed at a non-denaturing temperature, more specifically at a temperature from 0 °C to 40 °C;
   (d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
   (e) detecting and quantifying in the mixture obtained in step (d) ss-oligonucleotides and duplex oligonucleotides; followed by
   (f) selecting the binding pair if in step (e) duplex is detectably present, and the molar amount of duplex is higher than the molar amount of ss-oligonucleotides;
   (g) optionally synthesizing separately the first and the second ss-oligonucleotide of a binding pair selected in step (f);
   thereby selecting and providing the binding pair of single-stranded all-LNA oligonucleotides.
2. The method of item 1, wherein in step (a) the first nucleobase sequence does not consist of or comprise a sequence selected from 5' gcctgacg 3' (SEQ ID NO:3) and 5' cgtcaggc 3' (SEQ ID NO:4) when the number of monomers of the first oligonucleotide is 8 to 15.
3. The method of any of the items 1 and 2, wherein the second nucleobase sequence does not consist of or comprise a sequence selected from 5' gcctgacg 3' (SEQ ID NO:3) and 5' cgtcaggc 3' (SEQ ID NO:4) when the number of monomers of the second oligonucleotide is 8 to 15.
4. The method of any of the items 1 to 3, wherein prior to step (e) the mixture obtained in step (d) is subjected to the additional step of separating ss-oligonucleotides and duplex oligonucleotides.
5. The method of any of the items 1 to 4, wherein steps (c) and (d) are performed at a non-denaturing temperature, specifically at a temperature selected from the group consisting of 0 °C to 5 °C, 5 °C to 10 °C, 10 °C to 15 °C, 15 °C to 20 °C, 20 °C to 25 °C, 25 °C to 30 °C, 30 °C to 35 °C, and 35 °C to 40 °C.
6. The method of any of the items 1 to 5, wherein steps (c) and (d) are performed at a temperature from 25 °C to 37 °C.
7. The method of any of the items 1 to 6, wherein prior to step (c) each ss-oligonucleotide of any of the steps (a) and (b) is kept in the absence of denaturing conditions.
8. The method of item 7, wherein prior to step (c) each ss-oligonucleotide of any of the steps (a) and (b) is kept in aqueous solution at a temperature from -80 °C to 40 °C, specifically from 0 °C to 40 °C, more specifically from 25 °C to 37 °C.
9. The method of item 7, wherein prior to step (c) each ss-oligonucleotide of any of the steps (a) and (b) is kept in aqueous solution in the absence of a denaturant compound capable of lowering the melting temperature of a DNA duplex of 20 base pairs in length and with a G+C content of 50% by at least 15 °C, specifically in the absence of any of formamide and DMSO.
10. The method of any of the items 1 to 9, wherein in step (d) the time interval is selected from the group consisting of 1 s to 20 min, 1 s to 5 min, 1 s to 60 s, and 1 s to 30 s.
11. The method of any of the items 1 to 10, wherein the mixture of step (c) comprises a buffer maintaining the pH of the mixture from pH 6 to pH 8, more specifically from pH 6.5 to pH 7.5.
12. The method of any of the items 1 to 11, wherein the mixture of step (c) contains an amount of dissolved substances from about 10 mmol/L to about 1000 mmol/L, specifically from about 10 mmol/L to about 500 mmol/L, more specifically from about 200 mmol/L to about 300 mmol/L.
13. The method of any of the items 1 to 12, wherein steps (c) and (d) are performed in the absence of a denaturant compound capable of lowering the melting temperature of a DNA duplex of 20 base pairs in length and with a G+C content of 50% by at least 15 °C, more specifically in the absence of any of formamide and dimethyl sulfoxide.
14. The method of any of the items 1 to 13, wherein each LNA monomer comprises a nucleobase selected from the group consisting of N⁴-acetylcytosine, 5-acetyluracil, 4-amino-6-chloropyrimidine, 4-amino-5-fluoro-2-methoxypyrimidine, 6-amino-1-methyluracil, 5-aminoorotic acid, 5-aminouracil, 6-aminouracil, 6-azauracil, N⁴-benzoylcytosine, 5-bromouracil, 5-chlorouracil, 6-chlorouracil, 6-chloromethyluracil, 6-chloro-3-methyluracil, cytosine, 5,6-dimethyluracil, 5-ethyluracil, 5-ethynyluracil, 5-fluorocytosine, 5-fluoroorotic acid, 5-fluorouracil, 5-iodo-2,4-dimethoxypyrimidine, 5-iodouracil, isocytosine, 5-methylcytosine, 6-methyl-5-nitrouracil, 2-methylthio-4-pyrimidinol, 5-methyl-2-thiouracil, 6-methyl-2-thiouracil, 6-methyluracil, 5-nitrouracil, orotic acid, 6-phenyl-2-thiouracil, 6-propyl-2-thiouracil, 2-thiouracil, 4-thiouracil, thymine, 5-(trifluoromethyl)uracil, uracil, adenine, 8-azahypoxanthine, 8-azaguanine, allopurinol, 4-aminopyrazolo [3,4-d]pyrimidine, 2-aminopurine, 2-acetamido-6-hydroxypurine, 2-amino-6-chloropurine, 2-amino-6-iodopurine, azathioprine, 4-amino-6-hydroxypyrazolo [3,4-d]pyrimidine, aminophylline, N⁶-benzyladenine, N⁶-benzoyladenine, 6-benzyloxypurine, 8-bromotheophylline, 8-bromo-3-methylxanthine, 8-bromo-7-(2-butyn-1-yl)-3-methylxanthine, 6-chloropurine, 8-chlorotheophylline, 6-chloro-2-fluoropurine, 6-chloro-7-deazapurine, 2-chloroadenine, 6-chloro-7-iodo-7-deazapurine, 2,6-diaminopurine, 2,6-dichloropurine, , 6-(dimethylamino)purine, 2,6-dichloro-7-deazapurine, 5,6-dichlorobenzimidazole hydrochloride, 7-deazahypoxanthine, 2-fluoroadenine, guanine, hypoxanthine, isoguanine, 3-iodo-1*H*-pyrazolo-[3,4-*d*]pyrimidin-4-amine, kinetin, 6-mercaptopurine, 6-methoxypurine, 3-methylxanthine, 1-methylxanthine, 3-methyladenine, O⁶-(cyclohexylmethyl)guanine, 6-thioguanine, 2-thioxanthine, xanthine, 5-propynyl-uracil, 5-propynyl-cytidine, 7-deazaadenine, 7-deazaguanine, 7-propynyl-7-deazaadenine, 7-propynyl-7-deazaguanine, and a derivative thereof.
15. The method of item 14, wherein each LNA monomer comprises a nucleobase selected from the group consisting of adenine, thymine, uracil, guanine, cytosine, and 5-methylcytosine,
16. The method of any of the items 1 to 15, wherein prior to step (e) the step of subjecting the incubated mixture of step (d) to the step of separating ss-oligonucleotides and duplex oligonucleotides.
17. The method of item 16, wherein the incubated mixture of step (d) is subjected to column chromatography and/or electrophoresis.
18. The method of any of the items 1 to 17, wherein the monomers of the ss-oligonucleotides of any of the steps (a) and (b) are beta-D-LNA monomers.
19. The method of any of the items 1 to 17, wherein the monomers of the ss-oligonucleotides of any of the steps (a) and (b) are beta-L-LNA monomers.
20. A pair of separate complementary ss-oligonucleotides, each ss-oligonucleotide consisting of 5 to 15 LNA monomers, the separate ss-oligonucleotides in aqueous solution being capable of forming with each other an antiparallel duplex in the absence of denaturing conditions prior to duplex formation or during duplex formation.
21. A pair of separate complementary ss-oligonucleotides, each ss-oligonucleotide consisting of 8 to 15 LNA monomers, the separate ss-oligonucleotides in aqueous solution being capable of forming with each other an antiparallel duplex in the absence of denaturing conditions prior to duplex formation or during duplex formation, wherein each of the ss-oligonucleotides does not contain a sequence selected from the group consisting of 5' gcctgacg 3' (SEQ ID NO:3) and 5' cgtcaggc 3' (SEQ ID NO:4).
22. A pair of separate complementary ss-oligonucleotides, each ss-oligonucleotide consisting of 5 to 7 LNA monomers, the separate ss-oligonucleotides in aqueous solution being capable of forming with each other an antiparallel duplex in the absence of denaturing conditions prior to duplex formation or during duplex formation.
23. The pair of separate complementary ss-oligonucleotides of any of the items 20 to 22, wherein the pair of ss-oligonucleotides is obtained by performing a method of any of the items 1 to 19.
24. A pair of separate complementary ss-oligonucleotides , wherein the pair is selected from the group consisting of
   (SEQ ID NO: 1) : (SEQ ID NO:2),
   (SEQ ID NO:9) : (SEQ ID NO: 10),
   (SEQ ID NO: 11) : (SEQ ID NO: 12),
   (SEQ ID NO: 13) : (SEQ ID NO: 14),
   (SEQ ID NO:9) : (SEQ ID NO: 15),
   (SEQ ID NO: 16) : (SEQ ID NO:20),
   (SEQ ID NO:21) : (SEQ ID NO:18),
   (SEQ ID NO:21) : (SEQ ID NO:20),
   (SEQ ID NO:21) : (SEQ ID NO:19),
   (SEQ ID NO:23) : (SEQ ID NO:17),
   (SEQ ID NO:25) : (SEQ ID NO:28).
25. The pair of separate complementary ss-oligonucleotides of item 24, wherein the pair is selected from the group consisting of,
   (SEQ ID NO:1) : (SEQ ID NO:2),
   (SEQ ID NO:28) : (SEQ ID NO:24),
   (SEQ ID NO:16) : (SEQ ID NO:20).
26. A pair of separate complementary ss-oligonucleotides of any of the items 20 to 25, wherein the first ss-oligonucleotide of the pair is attached to a first target, and the second ss-oligonucleotide is attached to a second target.
27. The pair of separate complementary ss-oligonucleotides of item 26, wherein a ss-oligonucleotide is covalently or non-covalently attached to the respective target.
28. The pair of separate complementary ss-oligonucleotides of any of the items 26 and 27, wherein the target is independently selected from the group consisting of a solid phase, a biomolecule, and a chemically synthesized compound.
29. The pair of separate complementary ss-oligonucleotides of any of the items 26 to 28, wherein the target is independently selected from the group consisting of an amino acid or analog thereof, a peptide, a polypeptide, a protein, a nucleobase, a nucleoside, an oligonucleotide, a nucleic acid, a lipid, and an analyte-specific receptor, the receptor including an antibody, an antibody derivative, and an antibody fragment.
30. The pair of separate complementary ss-oligonucleotides of any of the items 26 to 28, wherein the target is independently selected from the group consisting of a peptide, a polypeptide, a protein, a steroid or non-steroid hormone, a hapten, and conjugates thereof.
31. The pair of separate complementary ss-oligonucleotides of any of the items 26 to 28, wherein a target comprises a conjugate consisting of a plurality of different molecules selected from the group consisting of a peptide, a polypeptide, a protein, a steroid or non-steroid hormone, a hapten, a nucleobase, a nucleoside, an oligonucleotide, a nucleic acid, a lipid, an analyte-specific receptor, an analyte a cross-linking agent, and a mixture thereof.
32. The pair of separate complementary ss-oligonucleotides of any of the items 26 to 28, wherein a target comprises a solid phase.
33. The pair of separate complementary ss-oligonucleotides of any of the items 26 to 28, wherein a target comprises a detectable label.
34. A method of forming an antiparallel all-LNA duplex in the absence of denaturing conditions, the method comprising the steps of
   (a) providing separately the first and the second member of a pair of single-stranded all-LNA oligonucleotides of any of the items 20 to 33, wherein each single-stranded all-LNA oligonucleotide is separately dissolved in aqueous solution in the absence of a denaturant and kept at a temperature from 0 °C to 40 °C;
   (b) contacting the single-stranded all-LNA oligonucleotides of the pair with each other at a temperature from 0 °C to 40 °C in the absence of a denaturant;
   thereby forming the antiparallel all-LNA duplex.
35. An antiparallel duplex formed by a first and the second member of a pair of single-stranded all-LNA oligonucleotides, wherein the antiparallel duplex is obtained by a method of item 34.
36. Use of a pair of single-stranded all-LNA oligonucleotides of any of the items 20 to 33 in a liquid aqueous medium to connect a first and a second component in a complex, wherein the first component is attached to the first member of the pair, and the second component is attached to the second member of the pair.
37. Use of a pair of single-stranded all-LNA oligonucleotides of any of the items 20 to 33 in a receptor-based assay for determining an analyte, the receptor-based assay comprising an analyte-specific receptor and a solid phase for immobilizing the analyte on the solid phase, wherein the first ss-oligonucleotide of the pair is coupled to the analyte-specific receptor and the second ss-oligonucleotide of the pair is coupled to the solid phase.
38. A kit for performing a receptor-based assay for determining an analyte, the kit comprising in a first container an analyte-specific receptor having attached thereto a first member of a pair of separate ss-oligonucleotides of any of the items 20 to 33, the kit further comprising in a second container a solid phase having attached thereto a second member of the pair.
39. A method of performing a receptor-based assay for determining an analyte, the method comprising the steps of contacting the analyte with an analyte-specific receptor having attached thereto a first member of a pair of separate ss-oligonucleotides of any of the items 20 to 33, and with a solid phase having attached thereto a second member of the pair, incubating thereby forming a complex comprising the solid phase, the analyte-specific receptor bound to the solid phase and the analyte bound to the analyte-specific receptor, wherein an antiparallel duplex is formed, the duplex consisting of the first and the second member of the pair, wherein the duplex connects the analyte-specific receptor and the solid phase in the complex, followed by detecting analyte bound in the complex, thereby determining the analyte.

Concerning all aspects and embodiments as disclosed herein, the skilled person appreciates that an analyte-specific receptor specifically includes an antibody or an antibody fragment.

The following examples and figures are provided to aid the understanding of the present invention, the true scope of which is set forth in the appended claims. It is understood that modifications can be made in the procedures set forth without departing from the spirit of the invention.

### Description of the Figures

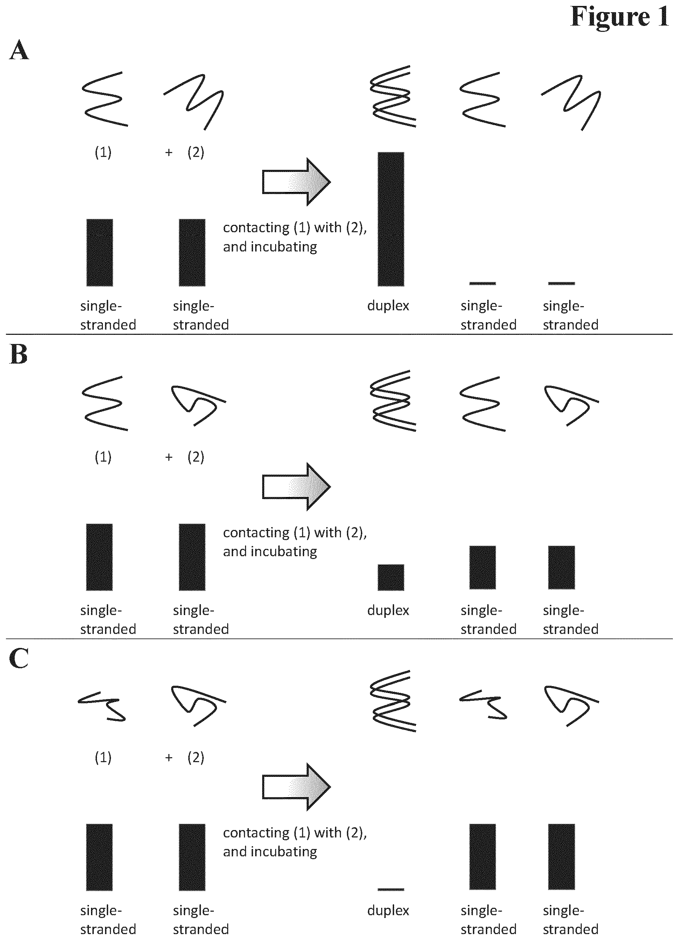

- **Figure 1**: Schematic view depicting the screening approach for compatible all-LNA oligonucleotide binding pairs (Example 2). Black bars represent quantities of the respective single-stranded molecules, duplex molecules or other complex molecules.
The first (1) and a second (2) single-stranded oligonucleotides are contacted with each other.
A: None of the two oligonucleotides is characterized by inter- or intramolecular secondary structures, both are capable of unencumbered molecular recognition of the respective partner; as a result, duplex is the main abundant resulting product, and single strands are either undetectable or present at insignificant amounts (desired outcome).
B: At least one of the two oligonucleotides is characterized by inter- or intramolecular secondary structures; as a result, duplex is less abundant and the majority of single strands are still present; duplex is formed, however at a reduced rate (not desired outcome).
C: Both oligonucleotides are characterized by inter- or intramolecular secondary structures; as a result, no duplex or an insignificant amount of duplex is formed and the single strands are still present, even after prolonged incubation (not desired outcome).
- **Figure 2**: HPLC analysis of single-stranded LNA 1 (SEQ ID NO:1; Example 2); the indicated retention time of the main peak is 3.353 min.
- **Figure 3**: HPLC analysis of single-stranded LNA 2 (SEQ ID NO:2; Example 2); the indicated retention time of the minor peak is 6.440 min, the indicated retention time of the main peak is 7.145 min.
- **Figure 4**: HPLC analysis of mixed LNA 1 and LNA 2, immediate injection into HPLC system (Example 2); the indicated retention time of the minor peak is 1.671 min, the indicated retention time of the main peak is 6.641 min.
- **Figure 5**: HPLC analysis of mixed LNA 1 and LNA 2 after thermal denaturation prior to injection (Example 2); positive control: duplex formation; the indicated retention time of the minor peak is 1.710 min, the indicated retention time of the main peak is 6.656 min.
- **Figure 6**: HPLC analysis of single-stranded LNA 3 (SEQ ID NO:5; Example 2); the indicated retention time of the main peak is 3.353 min.
- **Figure 7**: HPLC analysis of single-stranded LNA 4 (SEQ ID NO:6; Example 2); the indicated retention time of the minor peak is 6.440 min, the indicated retention time of the main peak is 7.145 min.
- **Figure 8**: HPLC analysis of mixed LNA 3 and LNA 4, immediate injection into HPLC system (Example 2); slow duplex formation (ratio < 0.05); the indicated retention time of the first peak is 3.387 min, the indicated retention time of the main peak is 7.157 min.
- **Figure 9**: HPLC analysis of mixed LNA 3 and LNA 4, injection after 50 min (Example 2); slow duplex formation (ratio = 0.05); the indicated retention time of the first peak is 3.365 min, the indicated retention time of the second peak is 6.871 min, the indicated retention time of the third peak is 7.148 min.
- **Figure 10**: HPLC analysis of mixed LNA 3 and LNA 4 after thermal denaturation prior to injection (Example 2); positive control: duplex formation; the indicated retention time of the main peak is 6.882 min.
- **Figure 11**: HPLC analysis of single stranded LNA 5'-Bi-Heg-accaac-3' (5' modified SEQ ID NO:20); the indicated retention time of the main peak is 6.184 min.
- **Figure 12**: HPLC analysis of single-stranded LNA 5'-gttggt-3' (SEQ ID NO:16); the indicated retention time of the minor peak is 1.496, and the indicated retention time of the main peak is 1.865 min.
- **Figure 13**: HPLC analysis of mixed LNAs 5'-Bi-Heg-accaac-3' (SEQ ID NO:20) and 5'-gttggt-3' (SEQ ID NO:16) (mixing at r.t. and immediate injection); the indicated retention time of the main peak is 6.568 min. Fast duplex formation (100% duplex formation: ratio 1.0).
5'-gttggt-3' (SEQ ID NO:14) used up for duplex formation, some residual single stranded LNA 5'-Bi-Heg-accaac-3' (SEQ ID NO:20) detectable.
- **Figure 14**: HPLC analysis of mixed LNAs 5'-Bi-Heg-accaac-3' (SEQ ID NO:20) and 5'-gttggt-3' (SEQ ID NO:16) (storage and mixing at +4°C to + 6°C and immediate injection); the indicated retention time of the main peak is 6.588 min, another retention time of 7.056 min is indicated. Fast duplex formation (100% duplex formation: ratio 1.0). 5'-gttggt-3' (SEQ ID NO:16) used up for duplex formation, minor amount of residual single stranded LNA 5'-Bi-Heg-accaac-3' (SEQ ID NO:20) detectable.
- **Figure 15**: HPLC analysis of mixed LNAs 5'-Bi-Heg-accaac-3' (SEQ ID NO:20) and 5'-gttggt-3' (SEQ ID NO:16) (storage and mixing at 0°C (ice bath) and immediate injection); the indicated retention time of the main peak is 6.552 min. Fast duplex formation (100% duplex formation: ratio 1.0). 5'-gttggt-3' (SEQ ID NO: 16) used up for duplex formation, minor amount of residual single stranded LNA 5'-Bi-Heg-accaac-3' (SEQ ID NO:20) detectable.
- Figure 16: HPLC analysis of mixed LNAs 5'-Bi-Heg-accaac-3' (SEQ ID NO:20) and 5'-gttggt-3' (SEQ ID NO: 16) (storage and mixing at - 10°C (magnesium chloride / ice bath) and immediate injection); the indicated retention time of the main peak is 6.547 min. Fast duplex formation (100% duplex formation: ratio 1.0). 5'-gttggt-3' (SEQ ID NO:16) used up for duplex formation, minor amount of residual single stranded LNA 5'-Bi-Heg-accaac-3' (SEQ ID NO:20) detectable.
- **Figure 17**: HPLC analysis of mixed LNAs 5'-Bi-Heg-accaac-3' (SEQ ID NO:20) and 5'-gttggt-3' (SEQ ID NO:16) after thermal denaturation and annealing prior to injection; the indicated retention time of the main peak is 6.583 min, another retention time of 6.967 min is indicated. Positive control for duplex formation.
- **Figure 18**: HPLC analysis of single stranded LNA 5'-Bi-Heg-cgtcaggcagttcag-3' (5' modified SEQ ID NO:47); the indicated retention time of the main peak is 6.840 min.
- **Figure 19**: HPLC analysis of single-stranded LNA 5'-ctgaactgcctgacg-3' (SEQ ID NO:48); the indicated retention time of the main peak is 3.488 min.
- **Figure 20**: HPLC analysis of mixed LNAs 5'-Bi-Heg- cgtcaggcagttcag-3' (SEQ ID NO:47) / 5'-ctgaactgcctgacg-3' (SEQ ID NO:48) (mixing at room temperature and immediate injection); the indicated retention time of the first peak is 3.594 min, the indicated second retention time of the respective peak is 6.580 min. Identification of a sequence pair with slow duplex formation. Slow duplex formation (ratio < 0.5).
- **Figure 21**: HPLC analysis of mixed LNAs 5'-Bi-Heg- cgtcaggcagttcag-3' (SEQ ID NO:47) / 5'-ctgaactgcctgacg-3' (SEQ ID NO:48) (storage and mixing at 0°C (ice bath) and immediate injection); the indicated retention time of the first peak is 3.541 min, the indicated second retention time of the respective peak is 6.853 min. Slow duplex formation (ratio < 0.5).
- **Figure 22**: HPLC analysis of mixed LNAs 5'-Bi-Heg- cgtcaggcagttcag-3' (SEQ ID NO:47) / 5'-ctgaactgcctgacg-3' (SEQ ID NO:48) (storage and mixing at -10°C (magnesium chloride / ice bath) and immediate injection); the indicated retention time of the first peak is 3.516 min, the indicated second retention time of the respective peak is 6.848 min. Slow duplex formation (ratio < 0.5).
- **Figure 23**: HPLC analysis of mixed LNAs 5'-Bi-Heg- cgtcaggcagttcag-3' (SEQ ID NO:47) / 5'-ctgaactgcctgacg-3' (SEQ ID NO:48) after thermal denaturation and annealing prior to injection; the indicated retention time of the main peak is 6.580 min. Positive control: duplex formation.
- **Figure 24**: A: Schematic depiction of the Biacore sensor used in Example 4.
B: Individual components as depicted in A.
   1: Sensor surface
   2: Streptavidin attached to the sensor surface
   3: biotin
   4: linker molecule attaching covalently the first ss-oligonucleotide to biotin.
   5: first ss-oligonucleotide
   6: second ss-oligonucleotide
      a: depicted is the situation when the second ss-oligonucleotide is contacted with the sensor having attached the first ss-oligonucleotide
      b: depicted is the outcome wherein the two ss-oligonucleotide are compatible and a duplex is formed under non-denaturing conditions; as a result, the bound second ss-oligonucleotide causes a change that can be detected by the Biacore instrument.

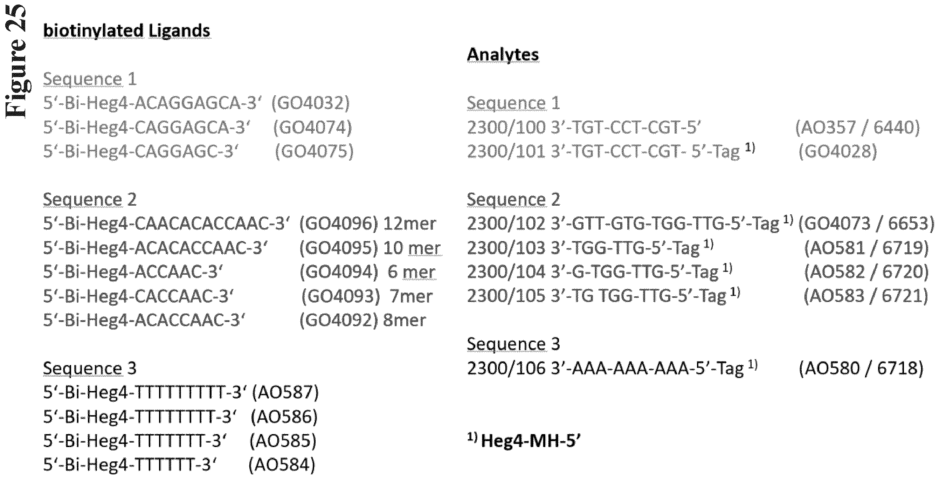

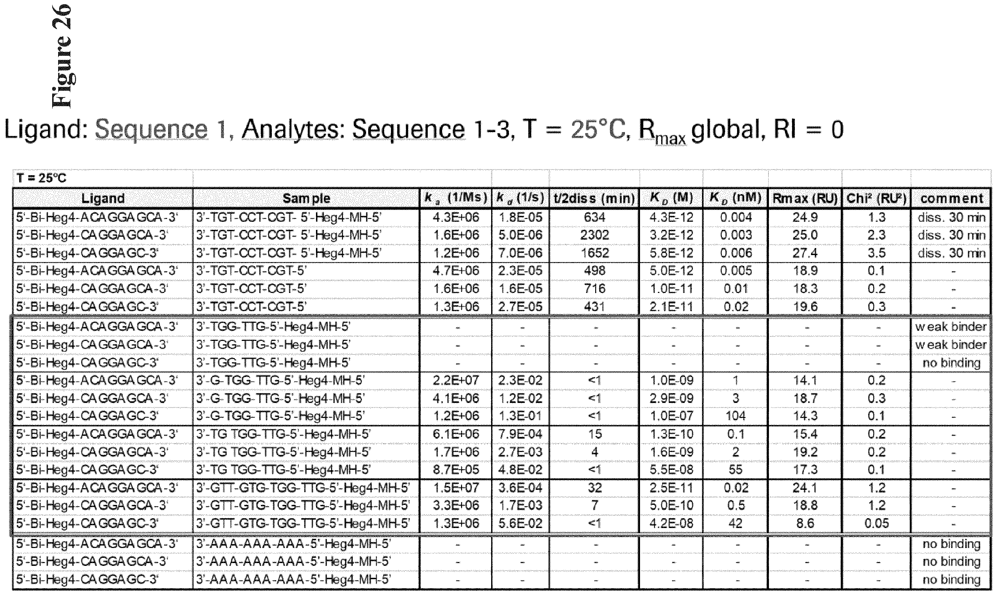

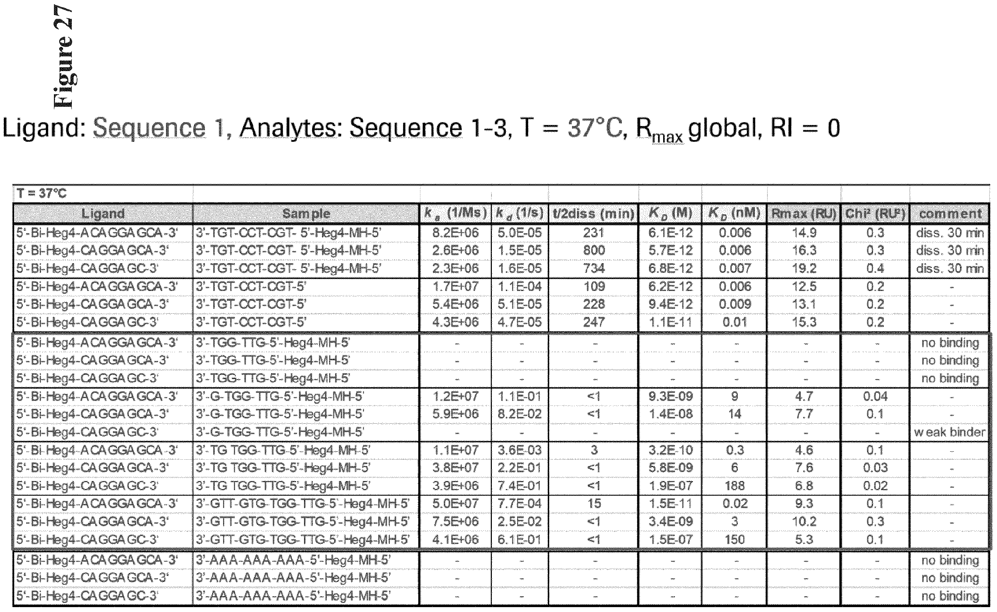

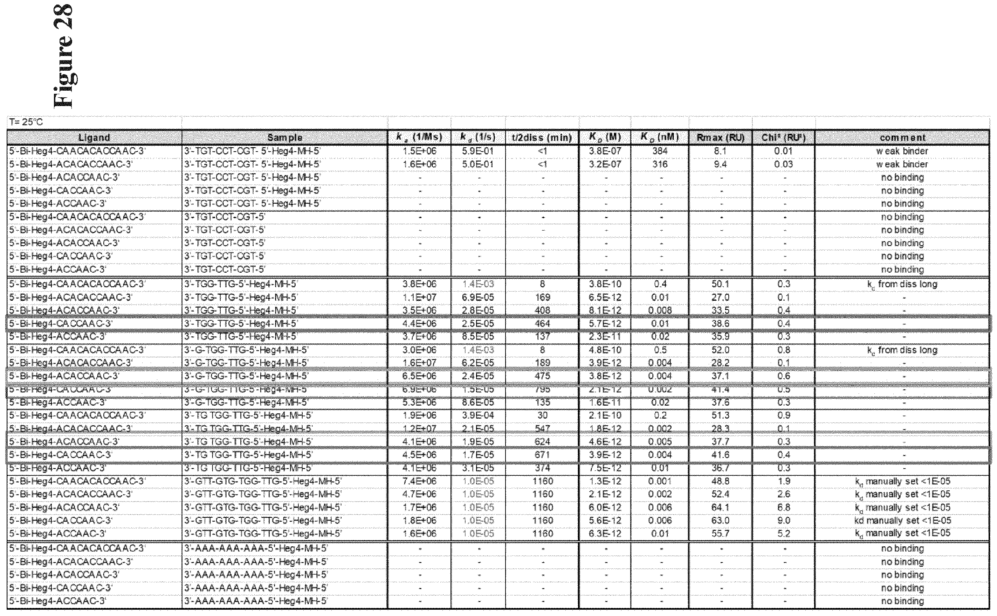

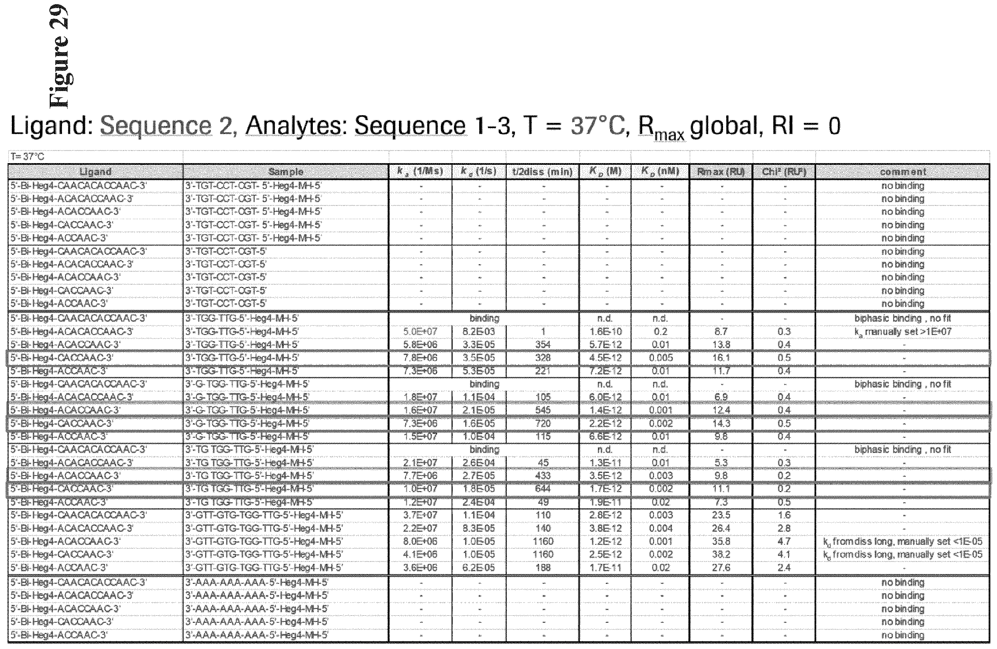

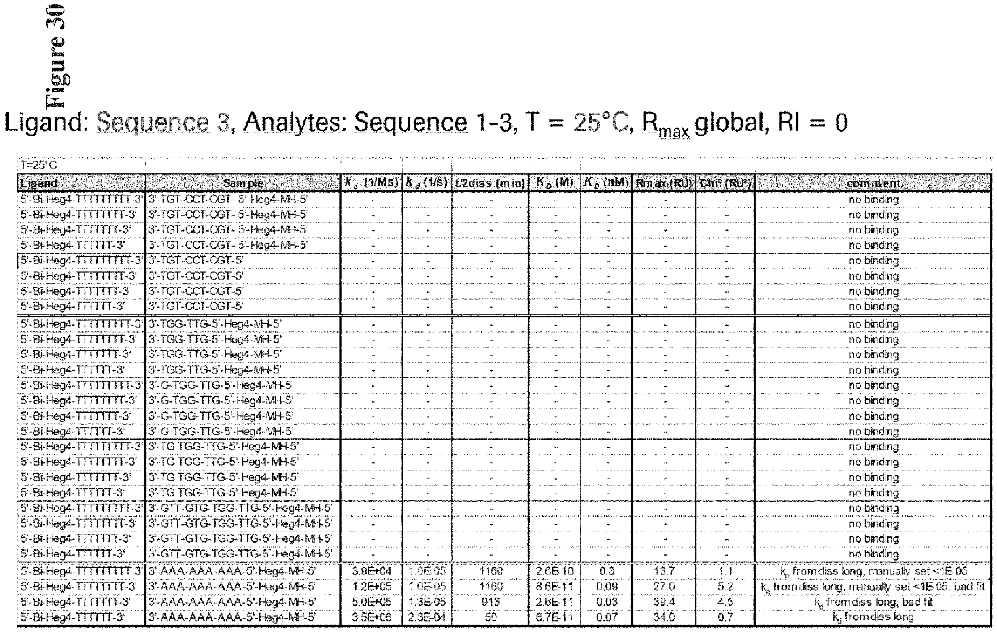

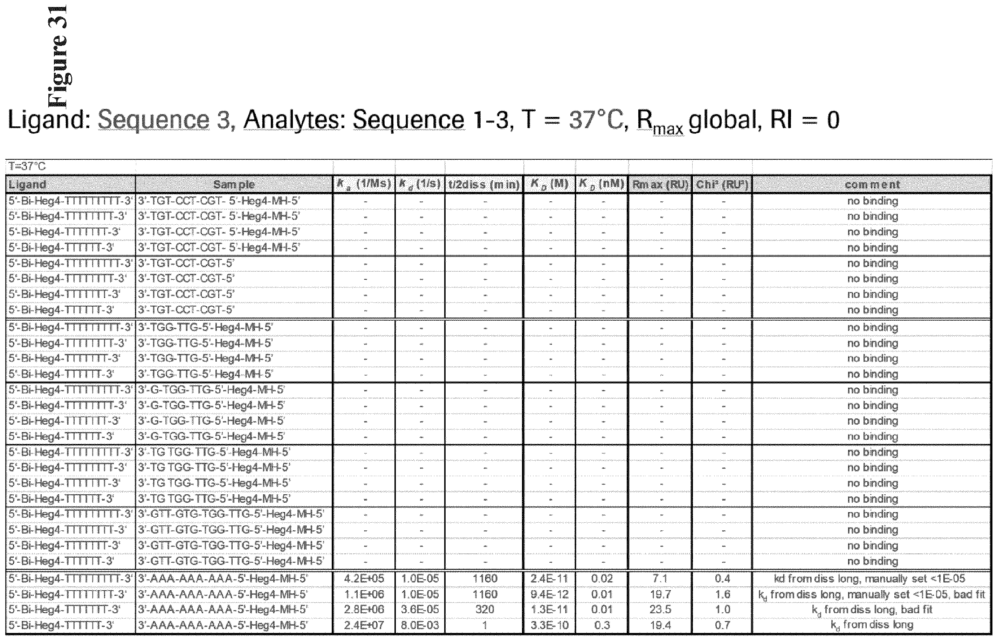

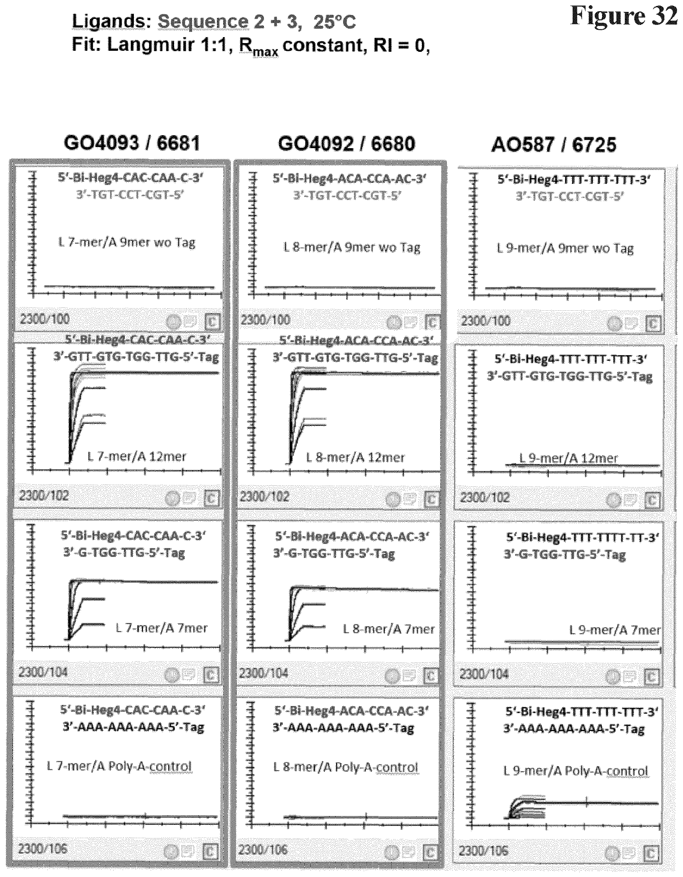

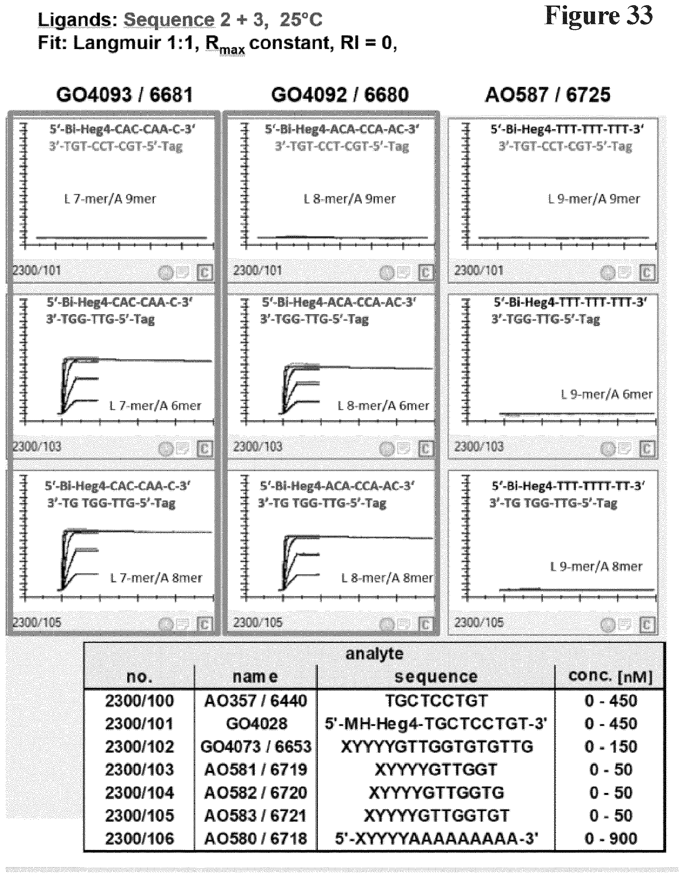

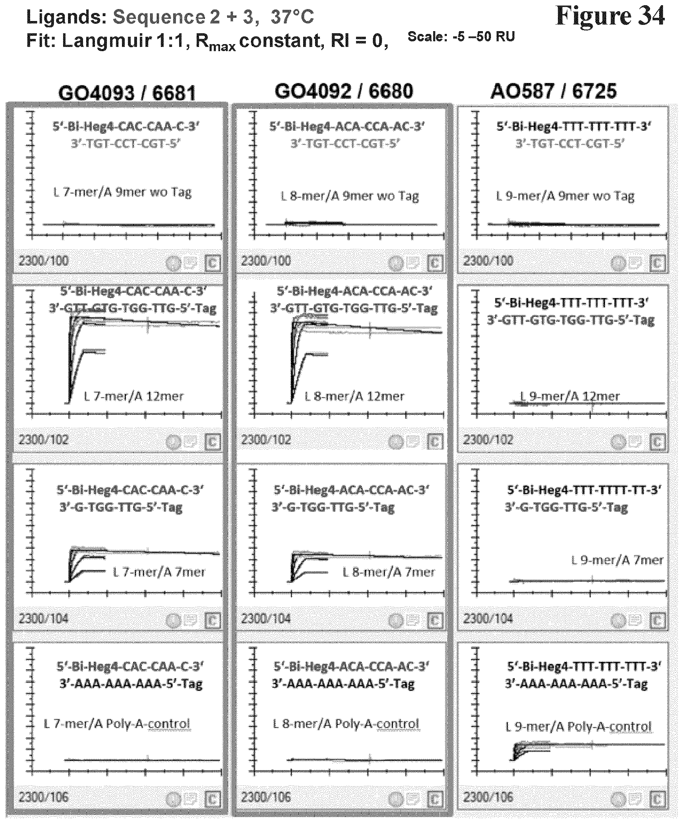

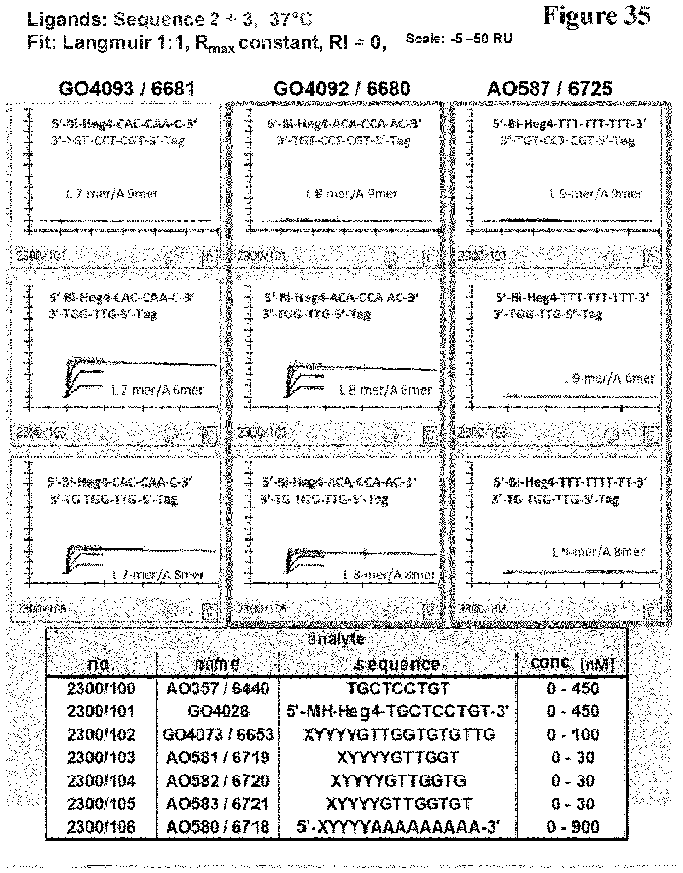

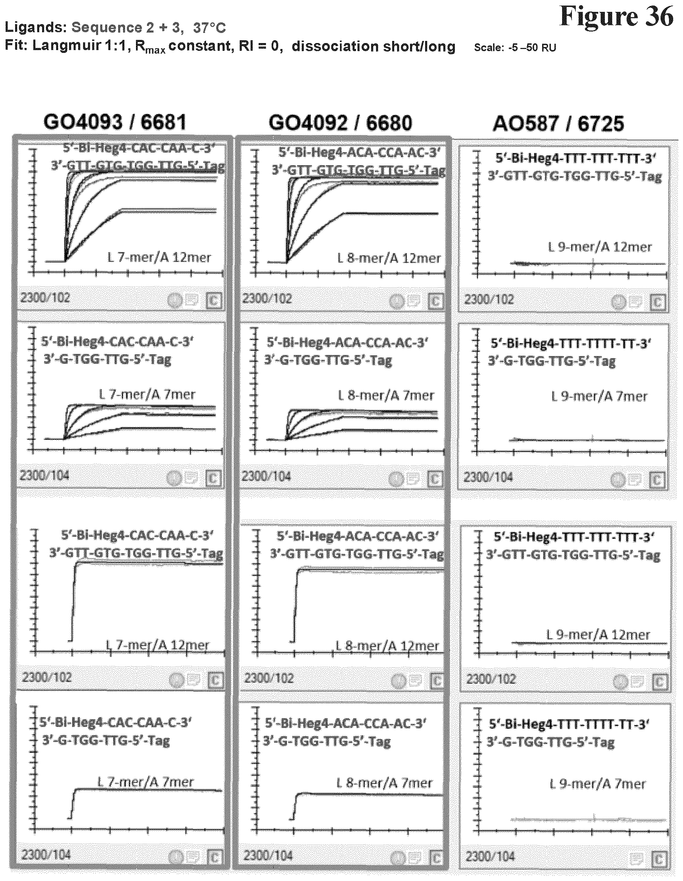

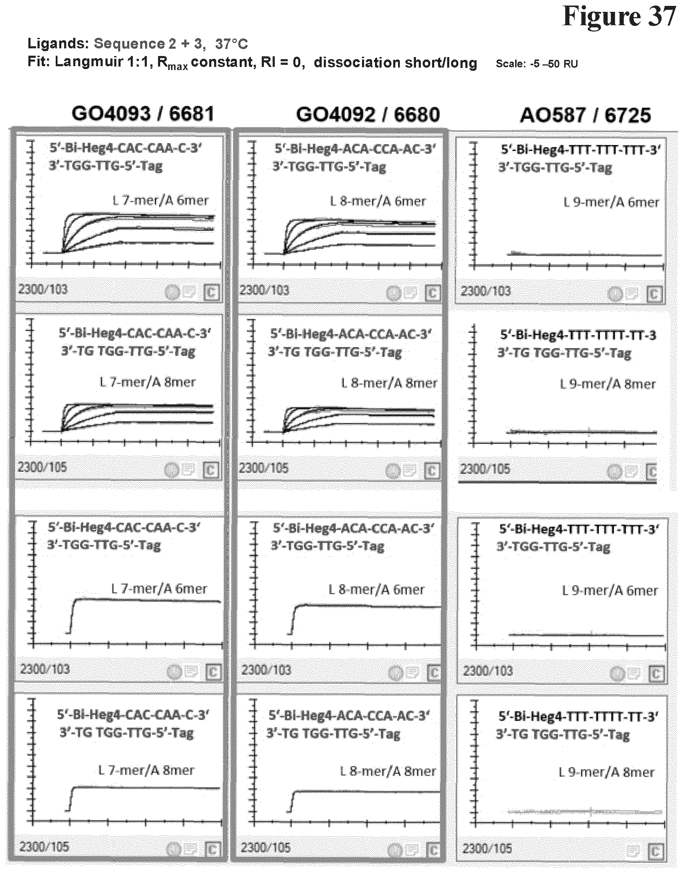

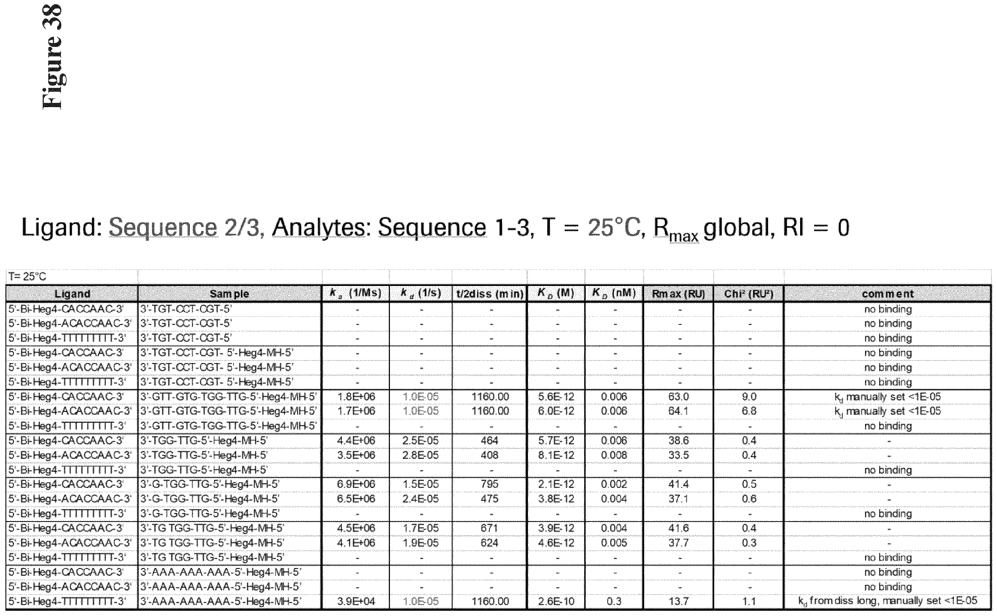

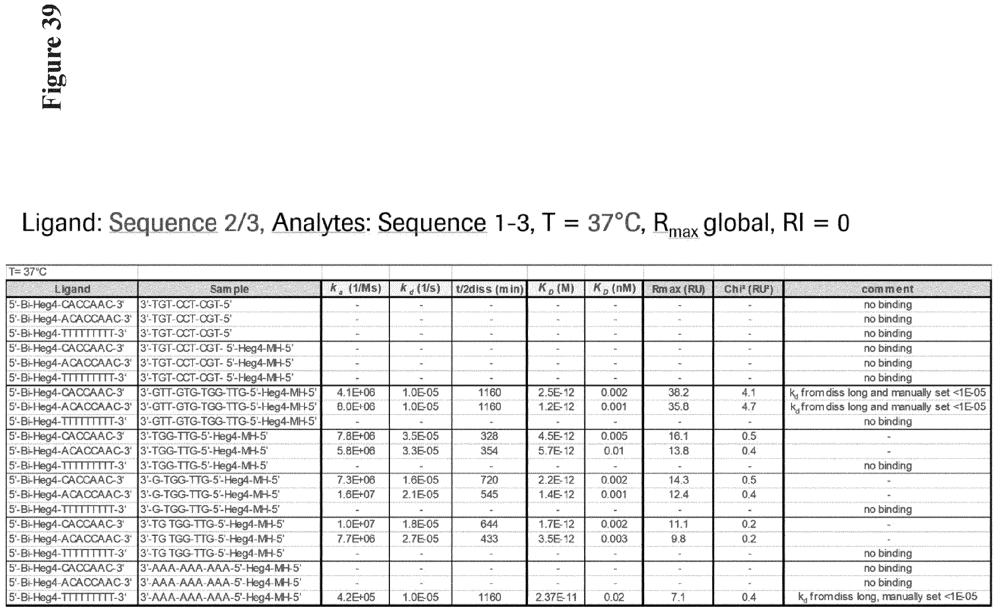

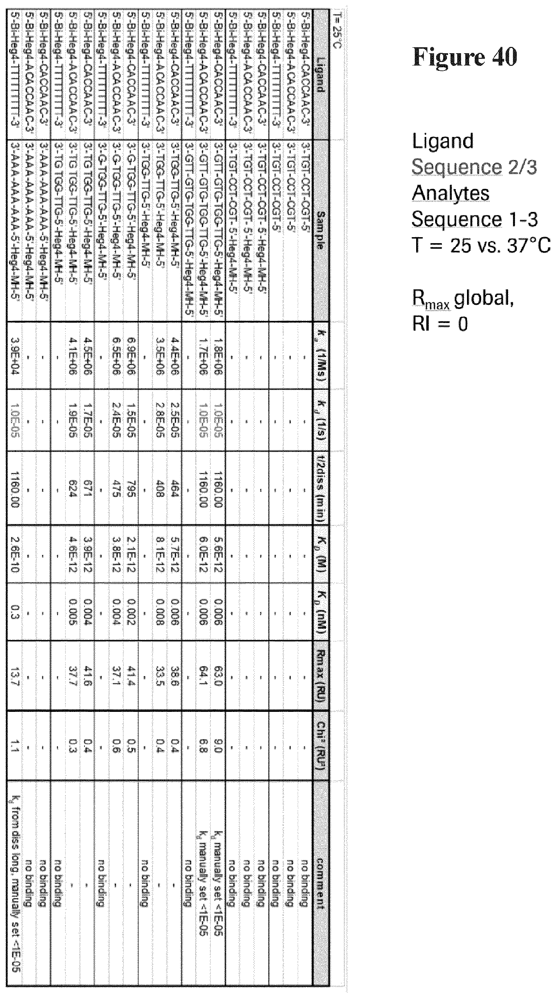

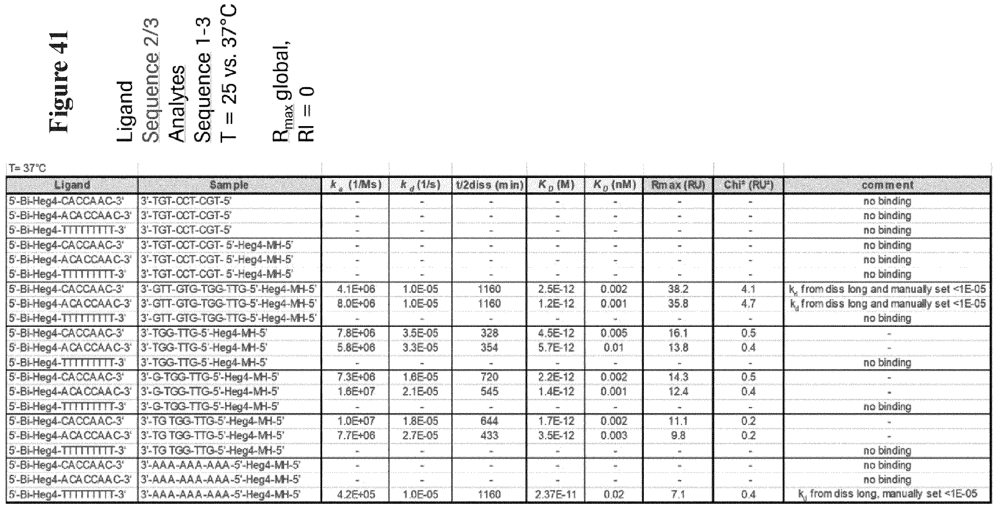

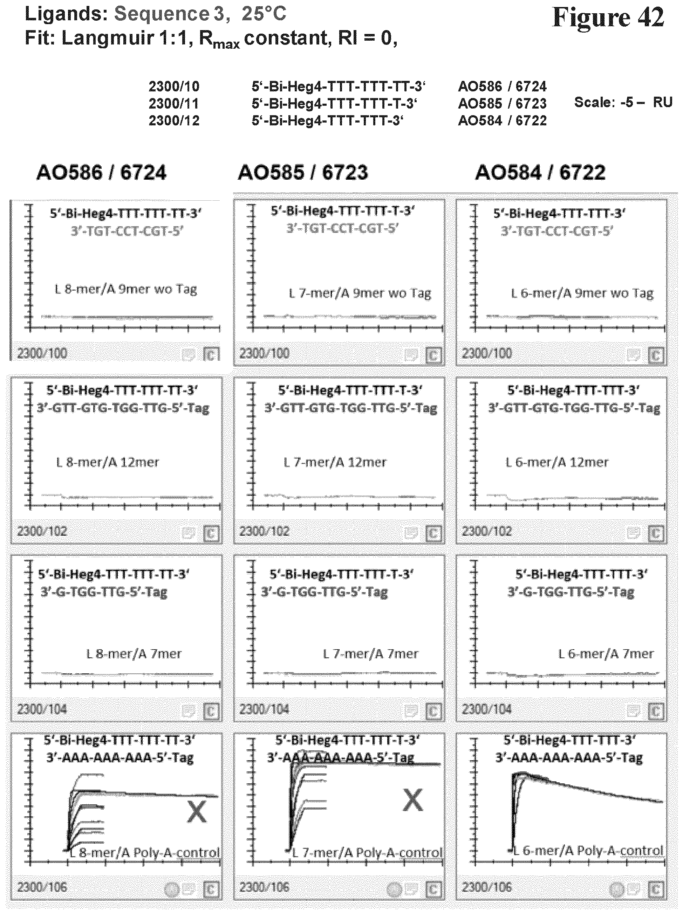

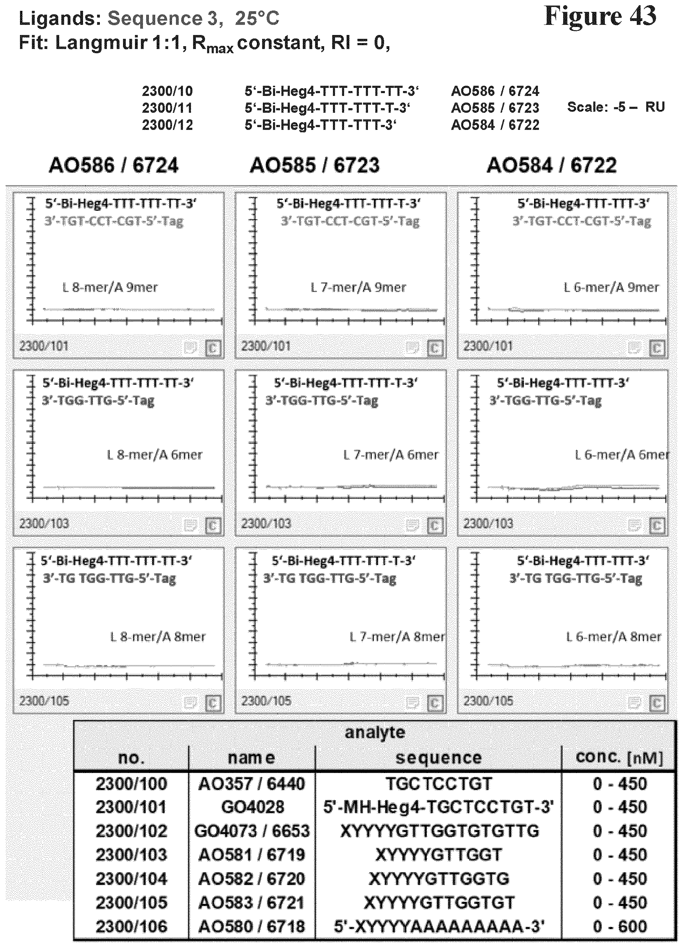

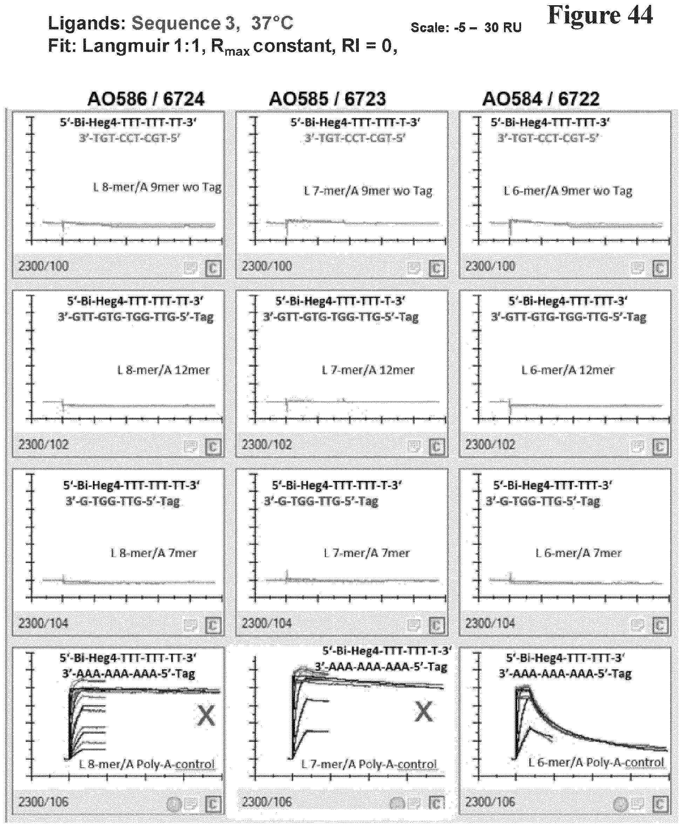

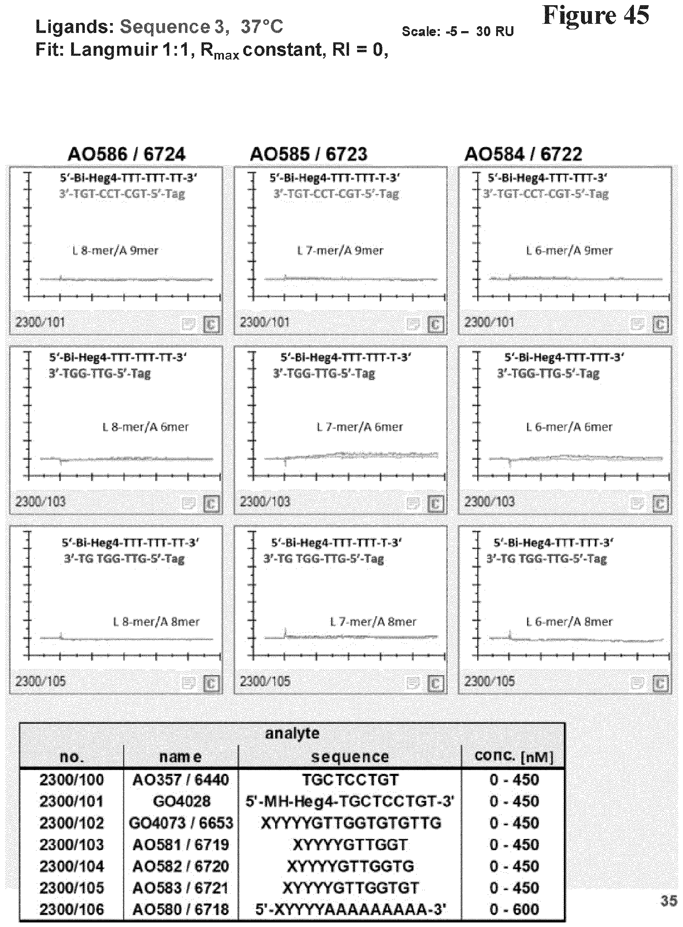

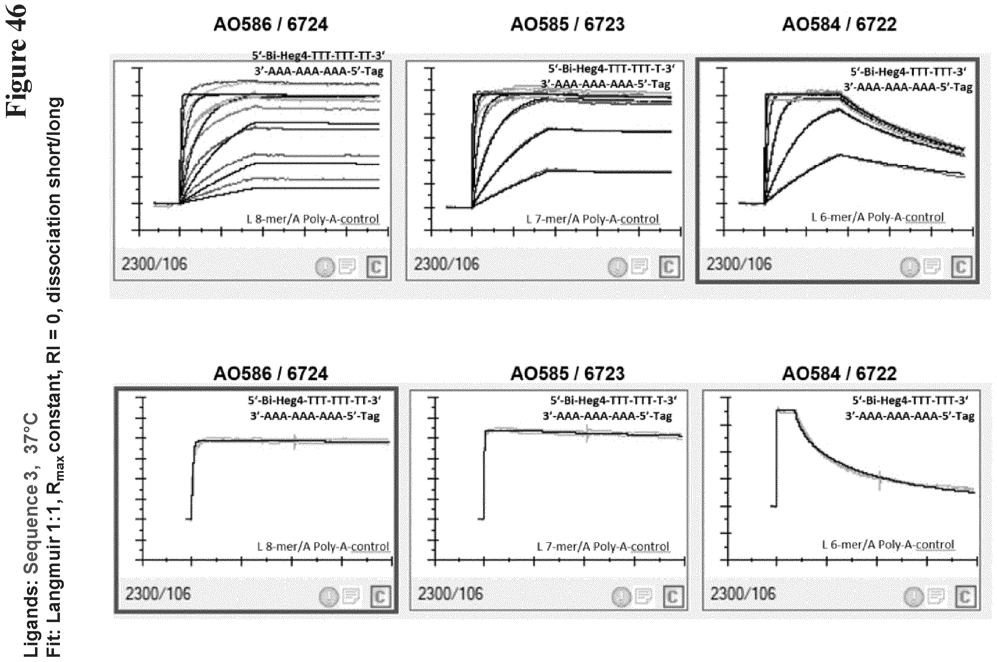

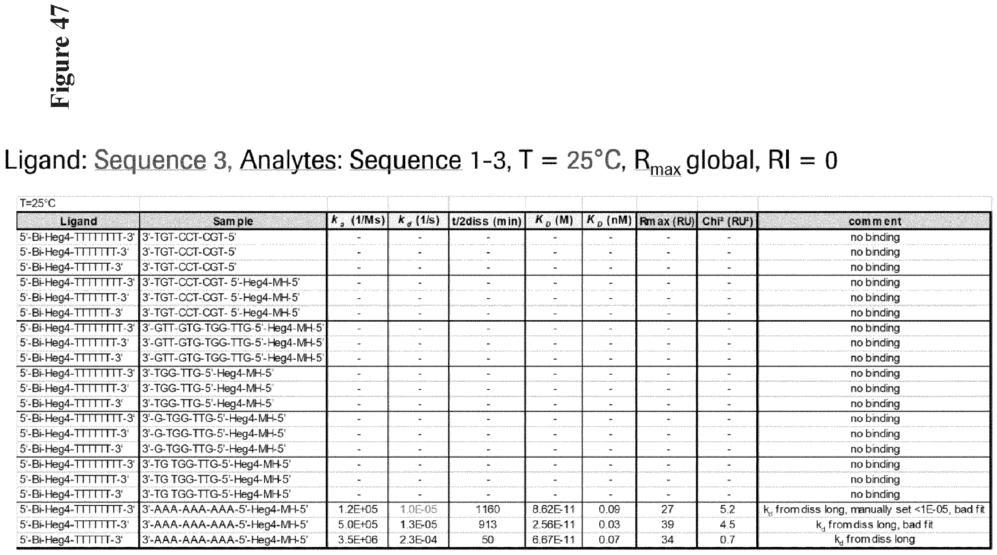

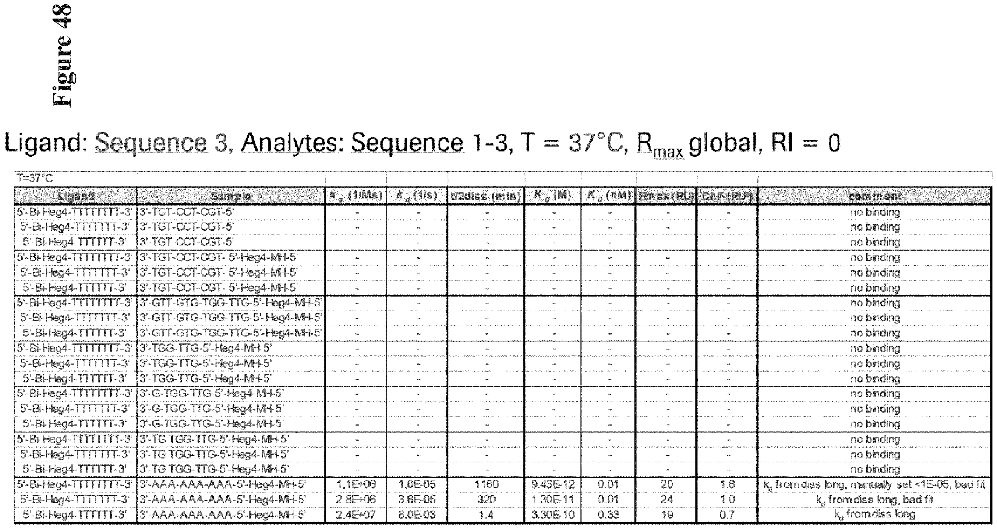

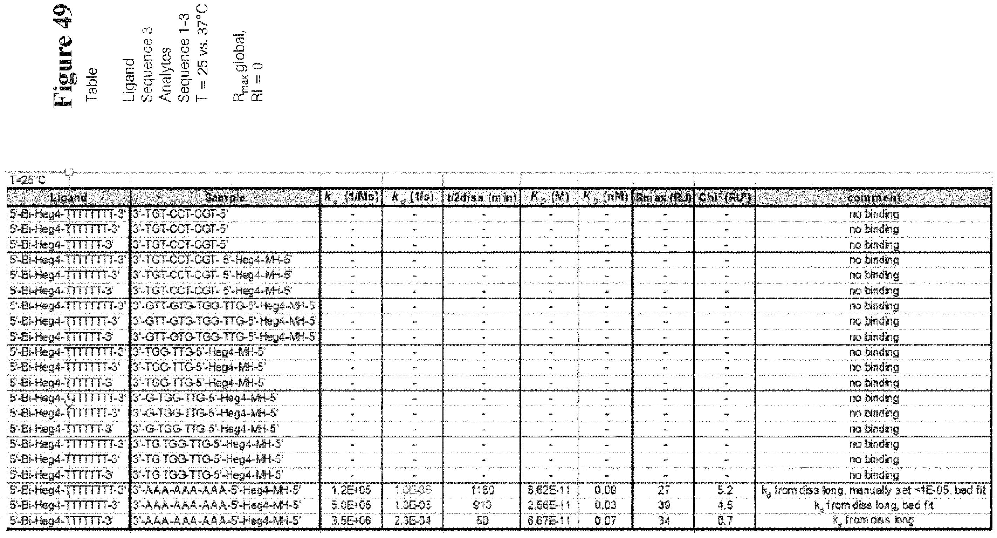

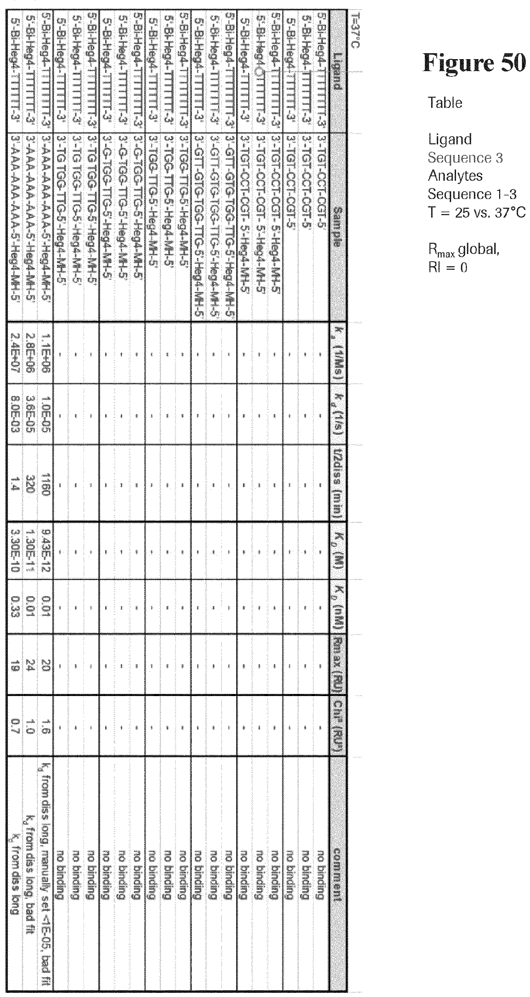

- **Figures 25 - 50**: Results of Example 4.

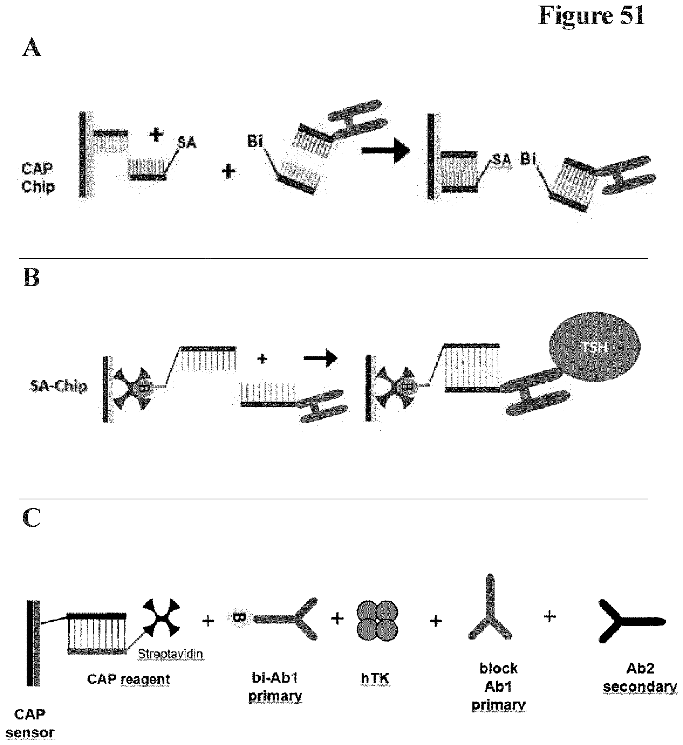

- **Figure 51**: Schematic depiction of the Biacore experiments in Example 5.

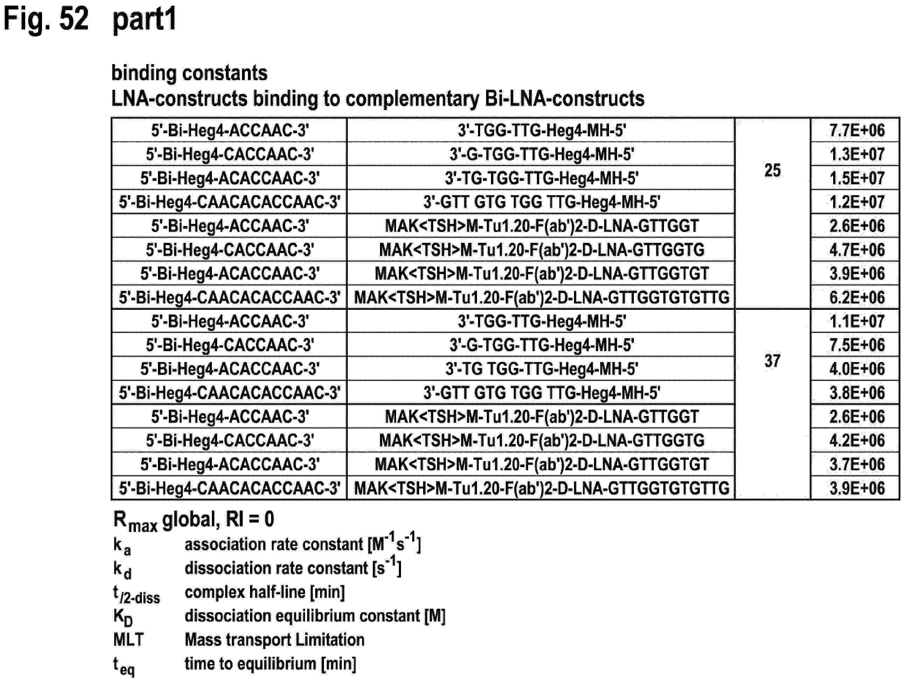

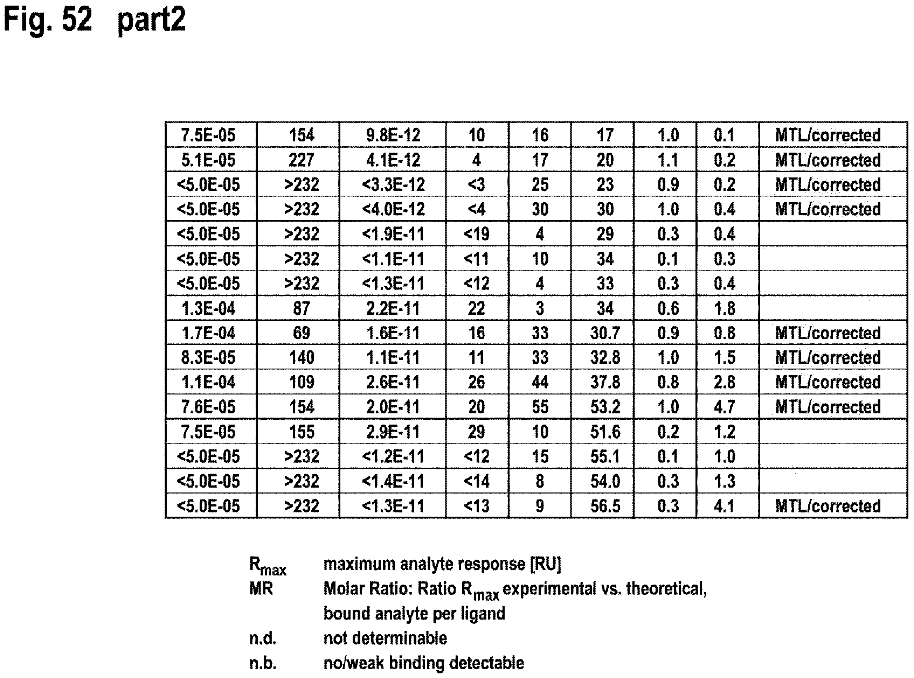

- **Figures 52 - 53**: Results of Example 5.

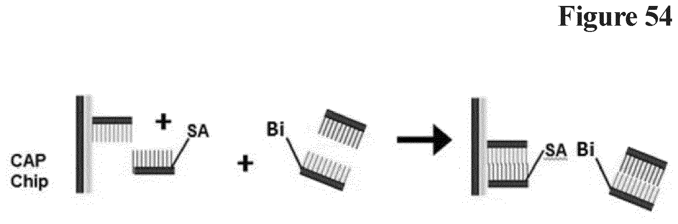

- **Figure 54**: Schematic depiction of the Biacore experiments in Example 6.

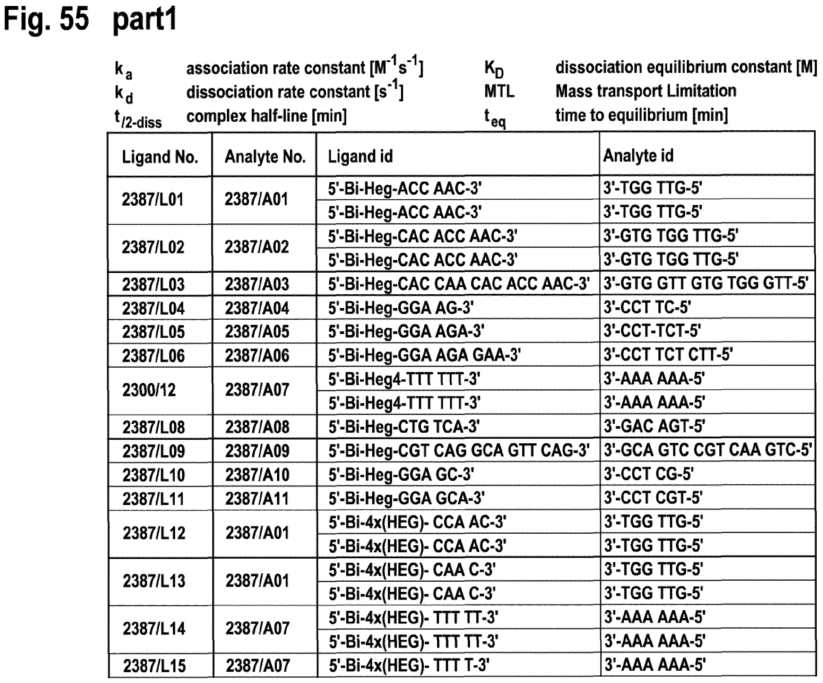

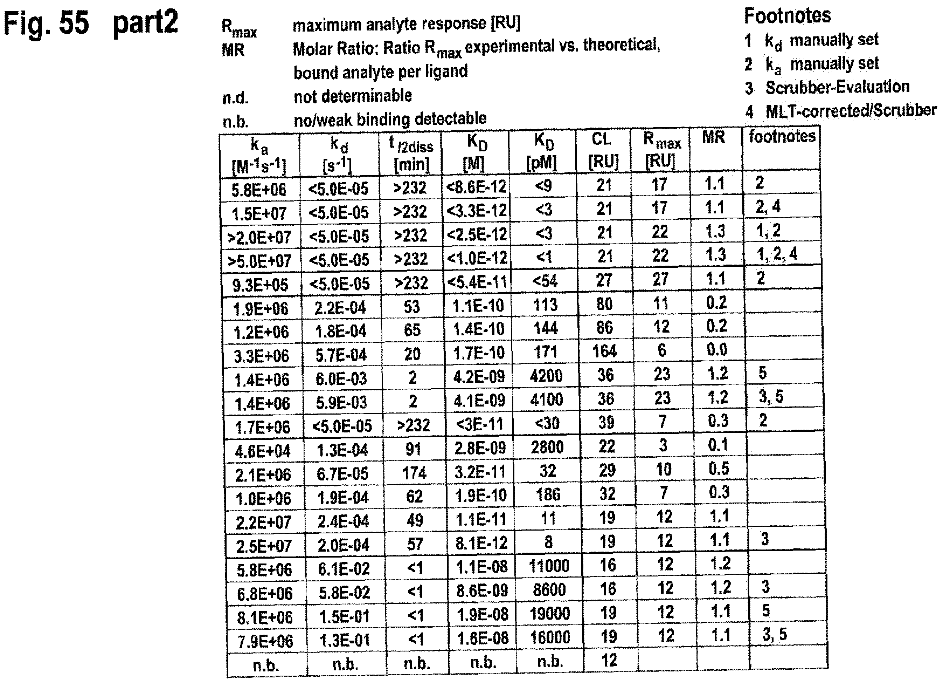

- **Figure 55**: LNA-constructs binding to Bi-LNA-constructs; binding constants at 25 °C

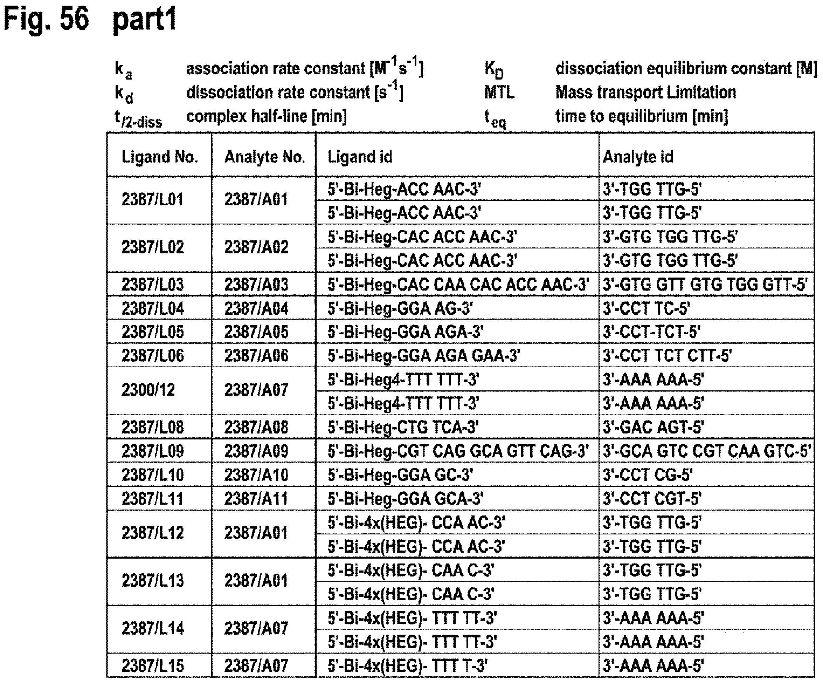

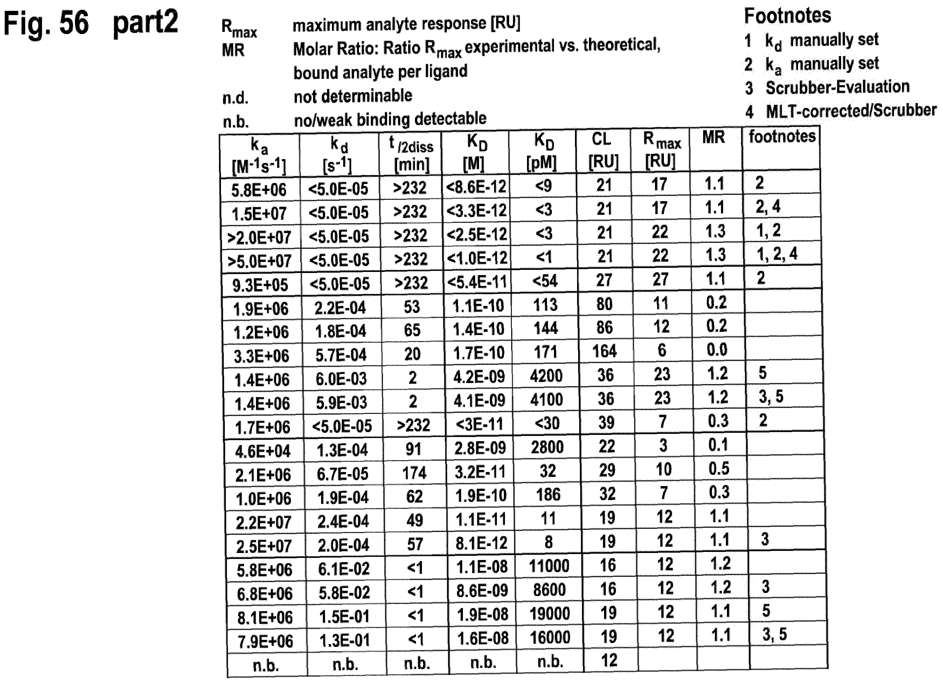

- **Figure 56**: LNA-constructs binding to Bi-LNA-constructs; binding constants at 37 °C

### Example 1

### Synthesis of LNA oligonucleotides

LNA oligonucleotides were synthesized in a 1 µmole scale synthesis on an ABI 394 DNA synthesizer using standard automated solid phase DNA synthesis procedure and applying phosphoramidite chemistry. Glen UnySupport PS (Glen Research cat no. 26-5040) and LNA phosphoramidites (Qiagen/Exiqon cat. No. 33970 (LNA-A(Bz), 339702 (LNA-T), 339705 (LNA-mC(Bz) and 339706 (LNA-G(dmf); beta-L-LNA analogues were synthesized analogously to D-beta-LNA phosphoramidites starting from L-glucose (Carbosynth, cat. No. MG05247) according to A.A. Koshkin et al., J.Org. Chem 2001, 66, 8504-8512) as well as spacer phosphoramidte 18 (Glen Research cat. No. 10-1918) and 5'-Biotin phosphoramidte (Glen Research cat. No. 10-5950) were used as building blocks. All phosphoramidites were applied at a concentration of 0.1 M in DNA grade acetonitrile. Standard DNA cycles with extended coupling time (180 sec), extended oxidation (45 sec) and detritylation time (85 sec) and standard synthesis reagents and solvents were used for the assembly of the LNA oligonucleotides. 5'-biotinylated LNA oligonucleotides were synthesized DMToff, whereas unmodified LNA oligonucleotides were synthesized as DMTon. Then, a standard cleavage program was applied for the cleavage of the LNA oligonucleotides from the support by conc. ammonia. Residual protecting groups were cleaved by treatment with conc. ammonia (8 h at 56 °C). Crude LNA oligonucleotides were evaporated and purified by RP HPLC (column: PRP-1, 7 µm, 250 x 21.5 mm (Hamilton, part no. 79352) or XBridge BEH C18 OBD, 5 µm, 10 x 250 mm (Waters part no. 186008167) using a 0.1 M triethylammonium acetate pH 7 / acetonitrile gradient. Product fractions were combined and desalted by dialysis (MWCO 1000, SpectraPor 6, part no. 132638) against water for 3 days, thereby also cleaving DMT group of DMTon purified oligonucleotides. Finally, the LNA oligonucleotides were lyophilized.

Yields ranged from 85 to 360 nmoles.

LNA oligonucleotides were analyzed by RP18 HPLC (Chromolith RP18e, Merck part no. 1.02129.0001) using a 0.1 M triethylammonium acetate pH 7 / acetonitrile gradient. Typical purities were ≥ 90%. Identity of LNA oligonucleotides were confirmed by LC-MS analysis.

Each species of oligonucleotide was synthesized and kept separately.

### Example 2

### Identification of LNA oligonucleotide sequences capable of forming duplex without prior denaturation applying RP-HPLC analysis

### a) general method:

LNA oligonucleotides from example 1 were dissolved in buffer (0.01 M Hepes pH 7.4, 0.15 M NaCl) and analyzed on RP18 HPLC (Chromolith RP18e, Merck part no. 1.02129.0001) using a 0.1 M triethylammonium acetate pH 7 / acetonitrile gradient (8-24% acetonitrile in 10 min; detection at 260 nm).

Strand and corresponding counterstrand LNA oligonucleotides were mixed at equimolar concentration at r.t. (room temperature) and immediately analyzed on RP18 HPLC (Chromolith RP18e, Merck part no. 1.02129.0001) using a 0.1 M triethylammonium acetate pH 7 / acetonitrile gradient (8-24% B in 10 min; detection at 260 nm).

In a first control experiment strand and corresponding counterstrand LNA oligonucleotides were mixed at equimolar concentration at r.t., incubated 1 h at r.t. and thereafter analyzed on RP18 HPLC (Chromolith RP18e, Merck part no. 1.02129.0001) using a 0.1 M triethylammonium acetate pH 7 / acetonitrile gradient (8-25% acetonitrile in 10 min; detection at 260 nm).

In a second control experiment to show duplex formation (positive control) strand and corresponding counterstrand LNA oligonucleotides were mixed at equimolar concentration at r.t., thermally denaturated at 95°C (10 min), and after having reached r.t. again analyzed on RP18 HPLC (Chromolith RP18e, Merck part no. 1.02129.0001) using a 0.1 M triethylammonium acetate pH 7 / acetonitrile gradient (8-24% acetonitrile in 10 min; detection at 260 nm).

Duplex formation can be detected if new peak at different retention time compared to the individual single stranded LNA oligonucleotides is formed. In the positive control mixed strand and counterstrand are thermally denaturated prior to injection yielding duplex. By time dependent injection after mixing strand and counterstrand LNA at r.t. without prior denaturation kinetics of duplex formation can be monitored.

LNA sequences are determined to be capable of quickly forming duplex if the HPLC% ratio of formed duplex and one of both single stranded LNA (corrected by extinction coefficient; in case both strands are not exactly equimolar higher ratio value is considered) is ≥ 0.9 after tempering 5-60 min at r.t. without prior denaturation (HPLC% corrected by extinction coefficients; hyperchromicity of duplex not considered).

### b) identification of sequence which forms duplex fast

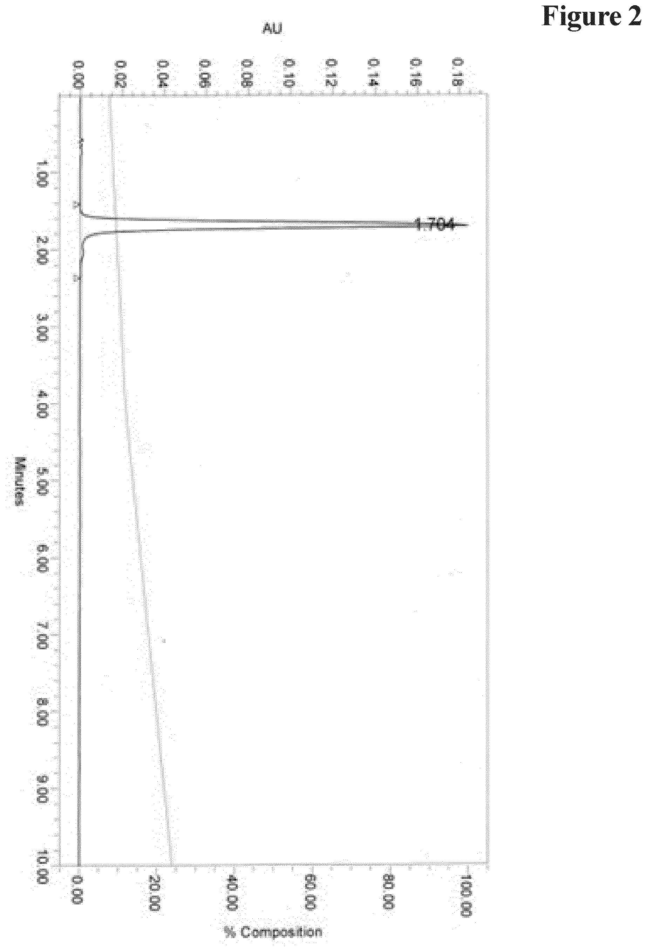

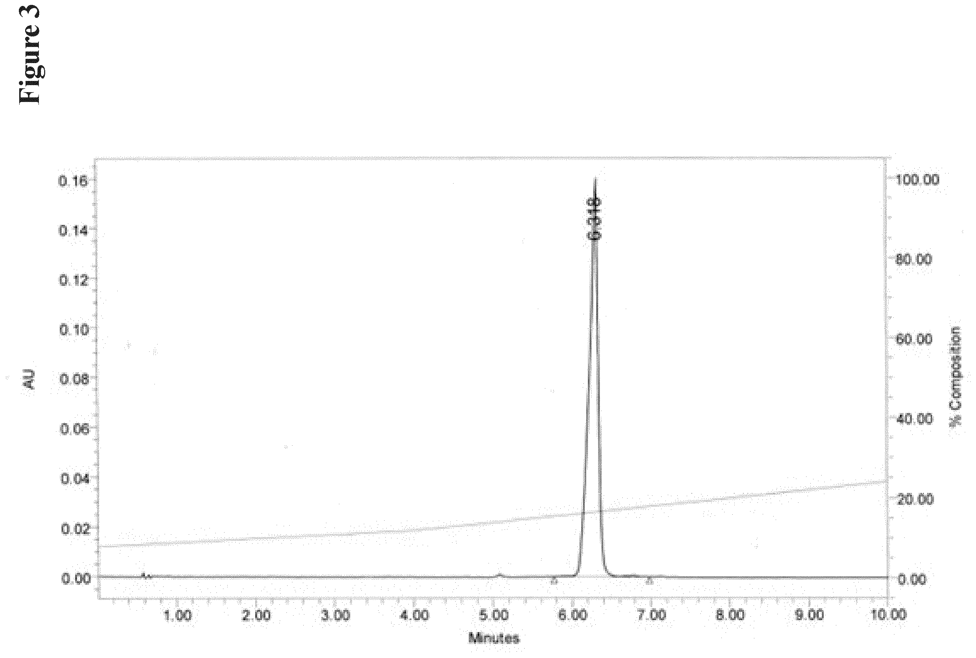

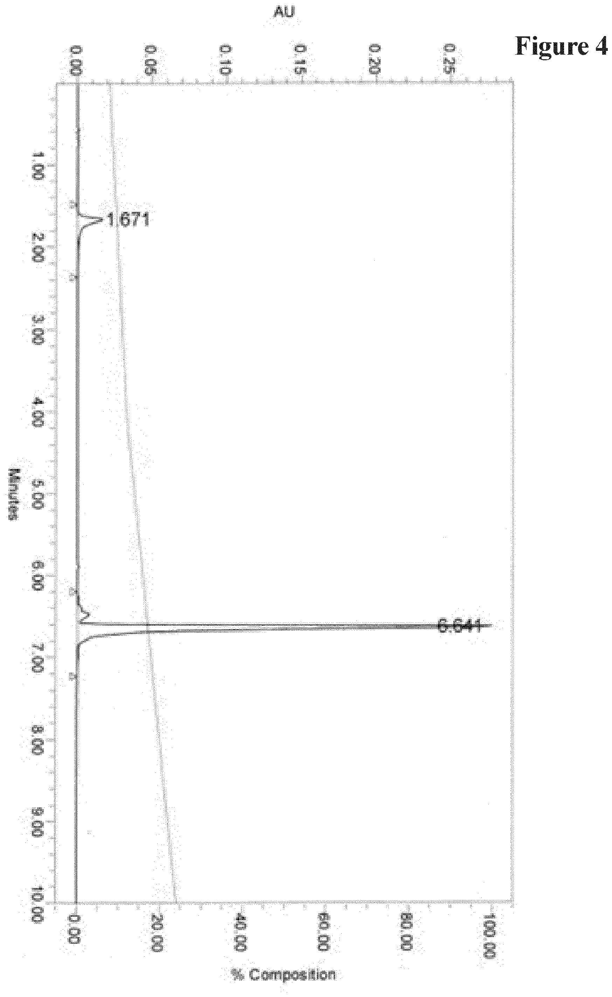

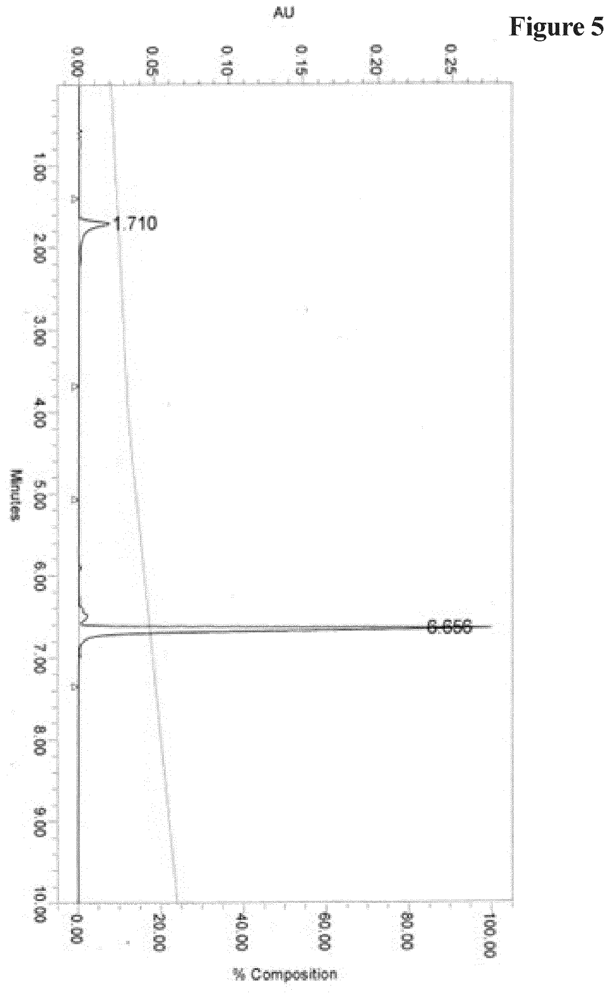

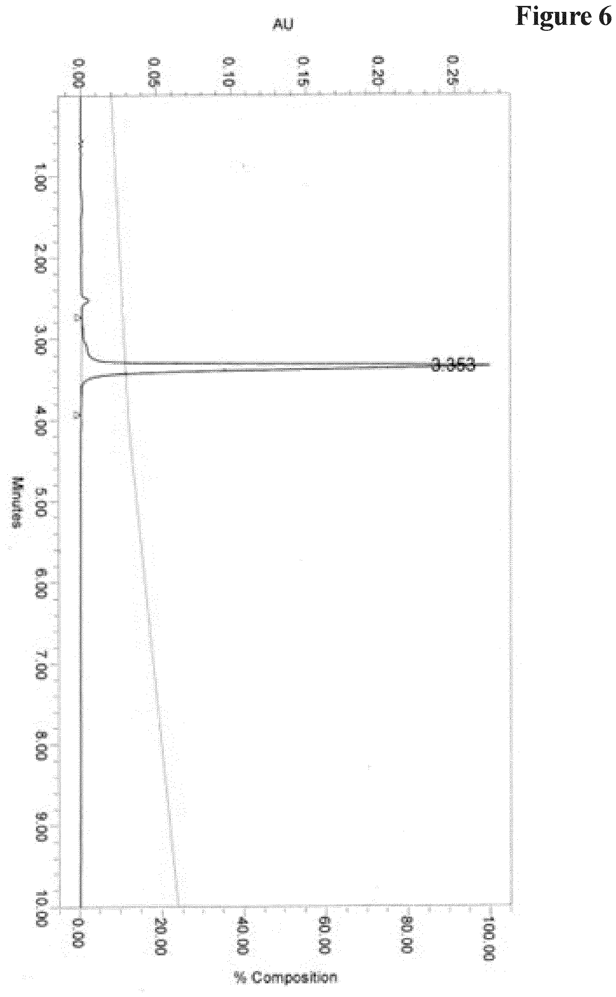

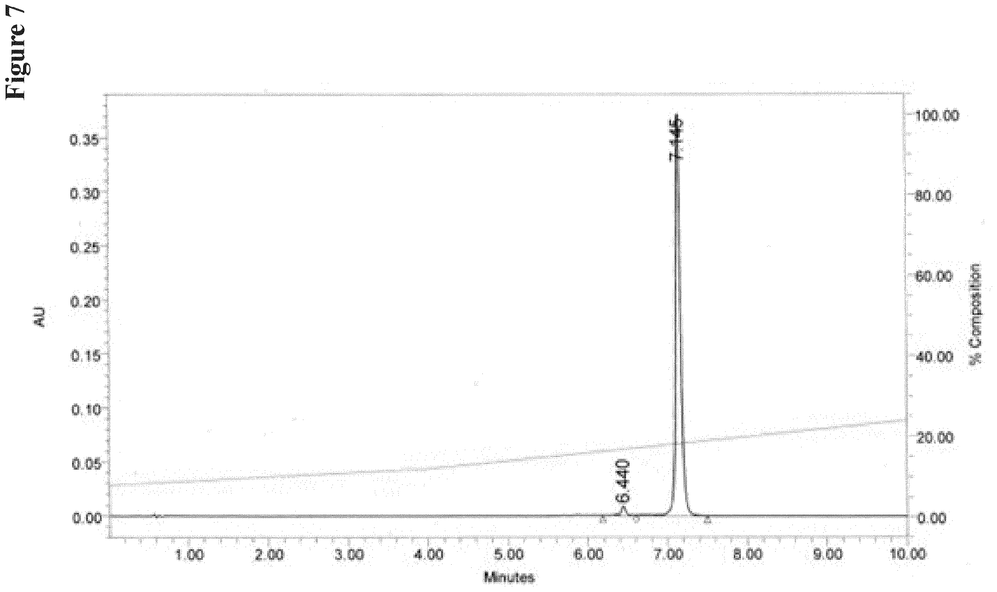

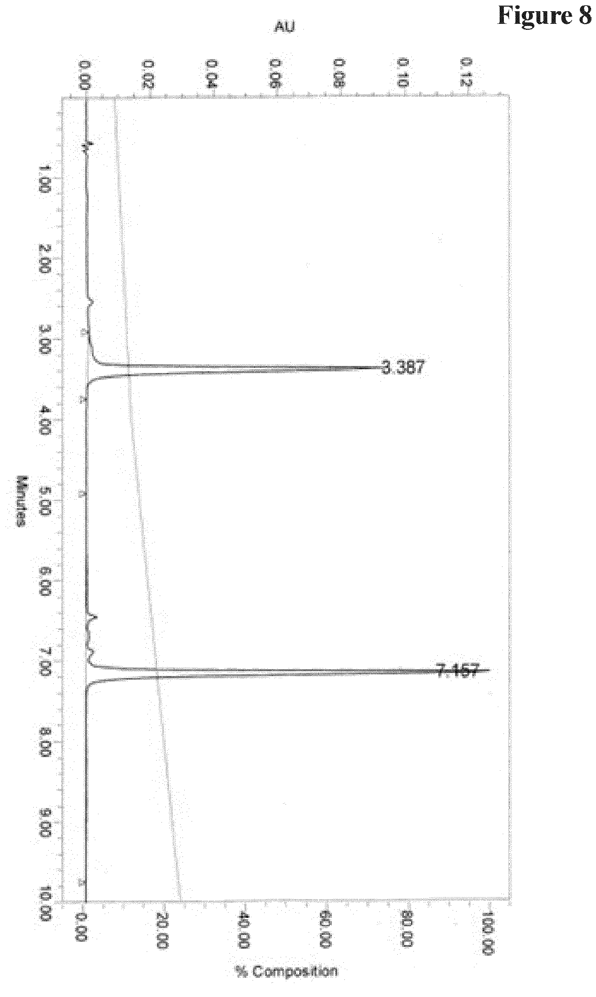

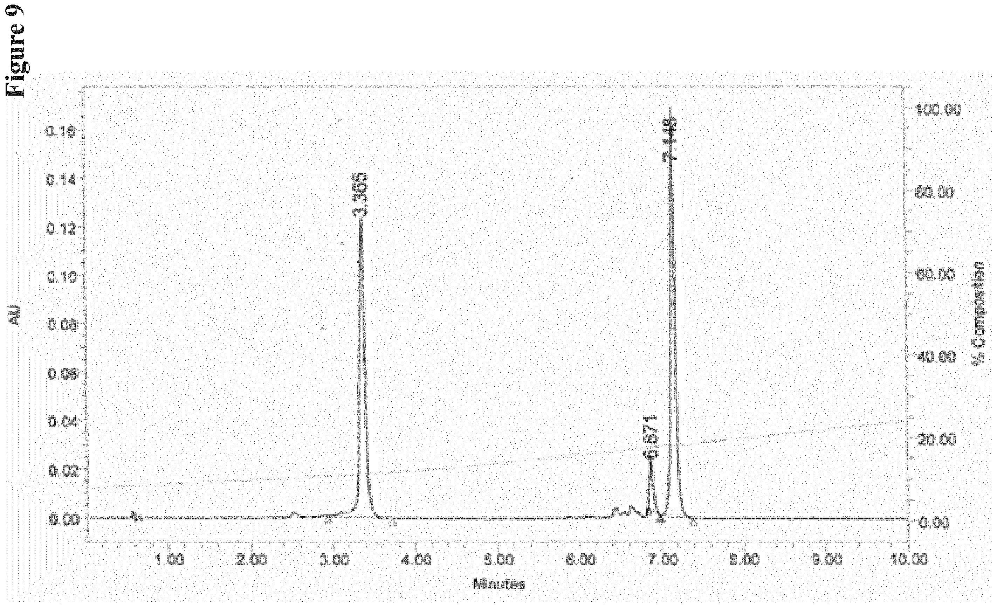

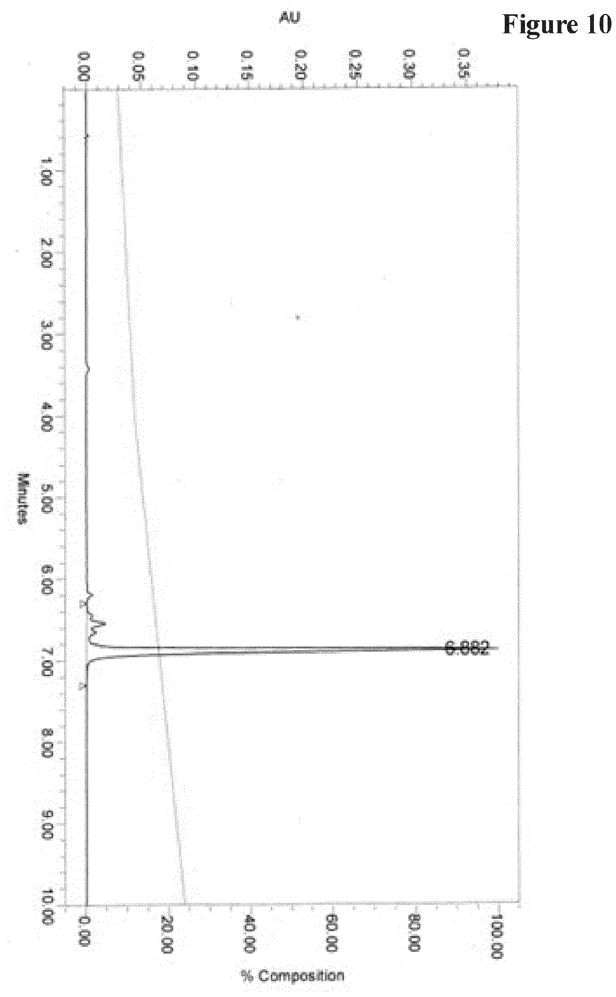

LNA 1: 5'-tgctcctg-3' (SEQ ID NO 1)
LNA 2: 5'-Bi-Heg-caggagca-3' (5'-modified SEQ ID NO 2)
Heg = hexaethyleneglycol
Bi = biotin label attached via the carboxy function of the valeric acid moiety of biotin The results are displayed in Figures 2-10.

### c) identification of sequence which forms duplex slowly

For the 10-bp hybridization experiment the following calculation of ratio was made LNA 3: 5'-ctgcctgacg-3'

LNA 4 (conjugate): 5'-Bi-Heg-cgtcaggcag-3'

| **LNA** | **retention time [min]** | **HPLC%** | **extinction coefficient (ε)** | **HPLC% * ε⁻¹ * 1000** |
|---|---|---|---|---|
| | | | **[l*mol⁻¹*cm⁻¹]** | |
| LNA 3 single strand | 3.365 | 45.14 | 98900 | 0.456 |
| LNA 4 single strand | 7.148 | 49.98 | 109300 | 0.457 |
| LNA 3/LNA 4 double strand | 6.871 | 4.88 | 208200 | 0.023 |

HPLC% * ε⁻¹ * 1000 (LNA 3/LNA 4 double strand) / HPLC% * ε⁻¹ * 1000 (LNA 3 single strand) = 0.023/0.456 = 0.05

HPLC% * ε⁻¹ * 1000 (LNA 3/LNA 4 double strand) / HPLC% * ε⁻¹ * 1000 (LNA 4 single strand) = 0.023/0.457 = 0.05

### Example 3

### Identification of LNA oligonucleotide sequences capable of forming duplex without prior denaturation applying RP-HPLC analysis

### a) general method:

LNA oligonucleotides from Example 1 were dissolved in buffer (0.01 M Hepes pH 7.4, 0.15 M NaCl) and analyzed on RP18 HPLC (Chromolith RP18e, Merck part no. 1.02129.0001) using a 0.1 M triethylammonium acetate pH 7 / acetonitrile gradient (8-24% acetonitrile in 10 min; detection at 260 nm).

Strand and corresponding counterstrand LNA oligonucleotides (i.e. first and second oligonucleotides) were mixed at equimolar concentration at r.t. (room temperature) or at a temperature selected from 0 °C to 40 °C, and immediately analyzed on RP18 HPLC (Chromolith RP18e, Merck part no. 1.02129.0001) using a 0.1 M triethylammonium acetate pH 7 / acetonitrile gradient (8-24% B in 10 min; detection at 260 nm).

In one type of experiment, strand and corresponding counterstrand LNA oligonucleotides were mixed at equimolar concentration at r.t., incubated 1 h at r.t. and thereafter analyzed on RP18 HPLC (Chromolith RP18e, Merck part no. 1.02129.0001) using a 0.1 M triethylammonium acetate pH 7 / acetonitrile gradient (8-25% acetonitrile in 10 min; detection at 260 nm). Other experiments were made at different temperatures. Temperatures were selected from 0 °C to 70°C, and more specifically from 0 °C to 5 °C, 0 °C to 5 °C, 0 °C to 10 °C, 0 °C to 20 °C, 0 °C to 30 °C, 5 °C to 10 °C, 10 °C to 15 °C, 15 °C to 20 °C, 20 °C to 25 °C, 25 °C to 30 °C, 30 °C to 35 °C, and 35 °C to 40 °C. Chromatographic analysis was performed at room temperature.

In a control experiment to show duplex formation (positive control), strand and corresponding counterstrand LNA oligonucleotides were mixed at equimolar concentration at r.t., thermally denaturated at 95 °C (10 min), and, after having cooled down again to room temperature, analyzed on RP18 HPLC (Chromolith RP18e, Merck part no. 1.02129.0001) using a 0.1 M triethylammonium acetate pH 7 / acetonitrile gradient (8-24% acetonitrile in 10 min; detection at 260 nm).

Duplex formation is detected if a new peak appears, at a different retention time compared to a peak corresponding to individual single stranded LNA oligonucleotides. In the positive control (control experiment, see above), mixed strand and counterstrand were thermally denaturated prior to injection, threby disrupting any structures that could be poresent and capable of preventing duplex formation. Thus, following thermal denaturation, duplex was obtained. By time dependent injection after mixing strand and counterstrand LNA at room temperature without prior denaturation kinetics of duplex formation was monitored.

LNA sequences were analyzed regarding their capability of quickly forming duplex. In an exemplary but non-limiting case, a positive result was obtained if the HPLC% ratio of formed duplex and one of both single stranded LNA (corrected by extinction coefficient; in case both strands are not exactly equimolar higher ratio value is considered) was ≥ 0.9 after tempering 5-60 min at r.t. without prior denaturation (HPLC% corrected by extinction coefficients; hyperchromicity of duplex not considered).

### b) identification of an exemplary sequence pair which is capable of fast duplex formation under non-denaturing conditions

An initial experiment yielded a first LNA oligonucleotide 5'-tgctcctg-3' (SEQ ID NO: 1), and a second LNA oligonucleotide Bi-Heg-5'-caggagca-3' (5' modified SEQ ID NO:2).
Heg = hexaethyleneglycol
Bi = biotin label attached via the carboxy function of the valeric acid moiety of biotin. These results and others are reflected in Figures.

The presence of the HEG moiety was found to be without impact on the hybridization properties of the respective oligonucleotide. Regarding nucleobase sequences and hybridization properties, no differences were found with respect to beta-D-LNA oligonucleotide pairs and beta-L-LNA oligonucleotide pairs.

The following list presents further exemplary results of oligonucleotide pairs which can be kept separately under non-denaturing conditions, and when contacted with each other are capable of forming a duplex under non-denaturing conditions.

The 15-mer 5' caccaacacaccaac 3' (SEQ ID NO 32, tested as 5'-modified Bi-Heg molecule) and 15-mer 5' gttggtgtgttggtg 3' (SEQ ID NO 31) showed fast duplex formation upon being contacted with each other.

### Number of Watson-Crick complementary base pairs: 5

5' ggaag 3' / 5' cttcc 3' were found with fast duplex formation ;
(SEQ ID NOs: 34 and 33, respectively).
5' ggagc 3' / 5' gctcc 3' were found with fast duplex formation ;
(SEQ ID NOs: 43 and 42, respectively).

### Number of Watson-Crick complementary base pairs: 6

5' accaac 3' / 5' gttggt 3' were found with fast duplex formation ;
(SEQ ID NOs: 20 and 16, respectively).
5' tttttt 3' / 5' aaaaaa 3' were found with fast duplex formation ;
(SEQ ID NOs: 27 and 39, respectively).
5' ggagca 3' / 5' tgctcc 3' were found with fast duplex formation ;
(SEQ ID NOs: 45 and 44, respectively).
5' ctgtca 3' / 5' tgacag 3' were found with fast duplex formation ;
(SEQ ID NOs: 40 and 41, respectively).
5' ggaaga 3' / 5' tcttcc 3' were found with fast duplex formation ;
(SEQ ID NOs: 36 and 35, respectively).

### Number of Watson-Crick complementary base pairs: 8

5' caggagca 3' / 5' tgctcctg 3' were found with fast duplex formation ;
(SEQ ID NOs: 2 and 1, respectively).

### Number of Watson-Crick complementary base pairs: 9

5' ggaagagaa 3' / 5' ttctcttcc 3' were found with fast duplex formation ;
(SEQ ID NOs: 38 and 37, respectively).

### Number of Watson-Crick complementary base pairs: 15

5' caccaacacaccaac 3' / 5' gttggtgtgttggtg 3' were found with fast duplex formation ;
(SEQ ID NOs: 32 and 31, respectively).

Typically, the first oligo of a binding pair as mentioned in the foregoing was used as a Bi-Heg conjugate as described for the initial experiments.

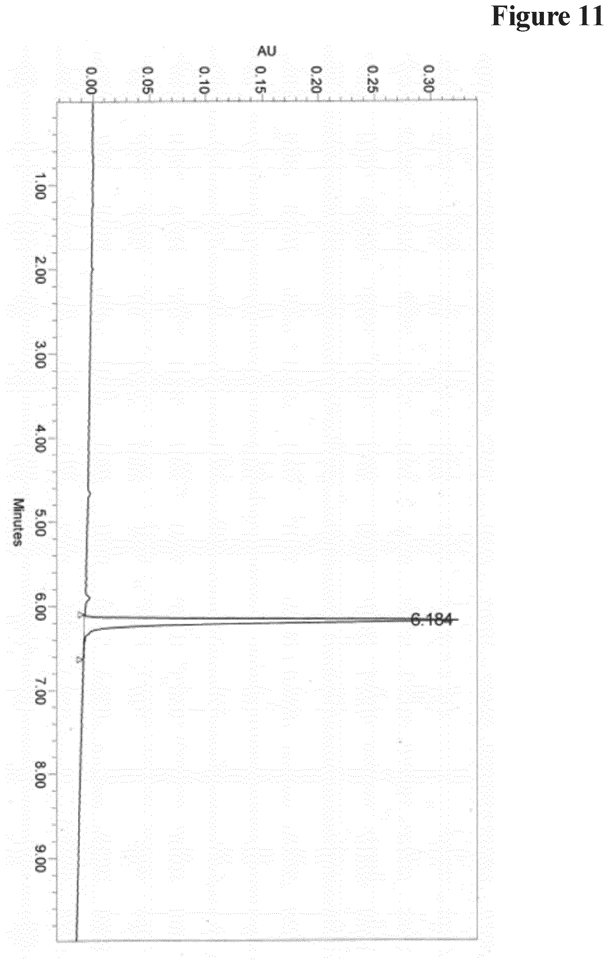

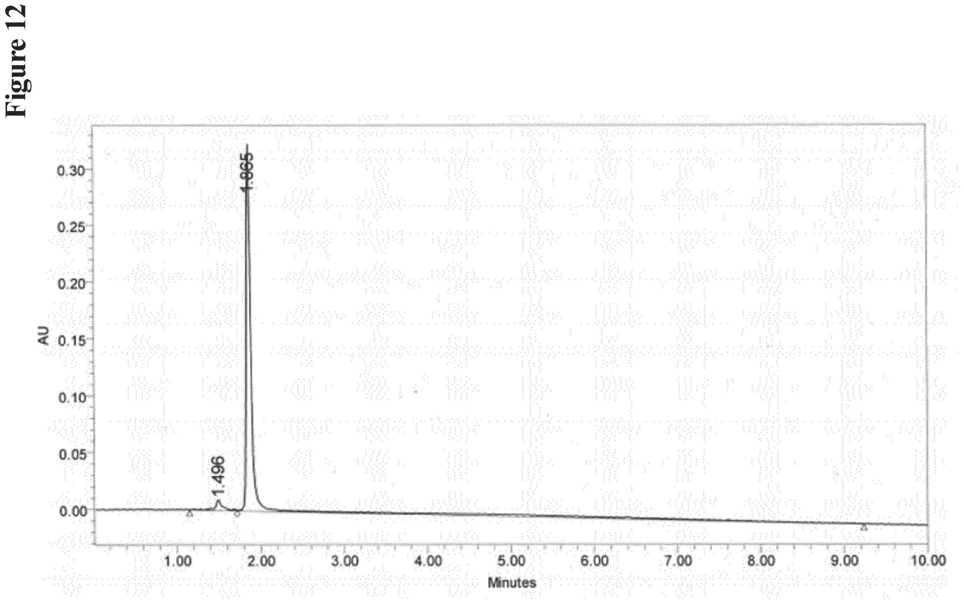

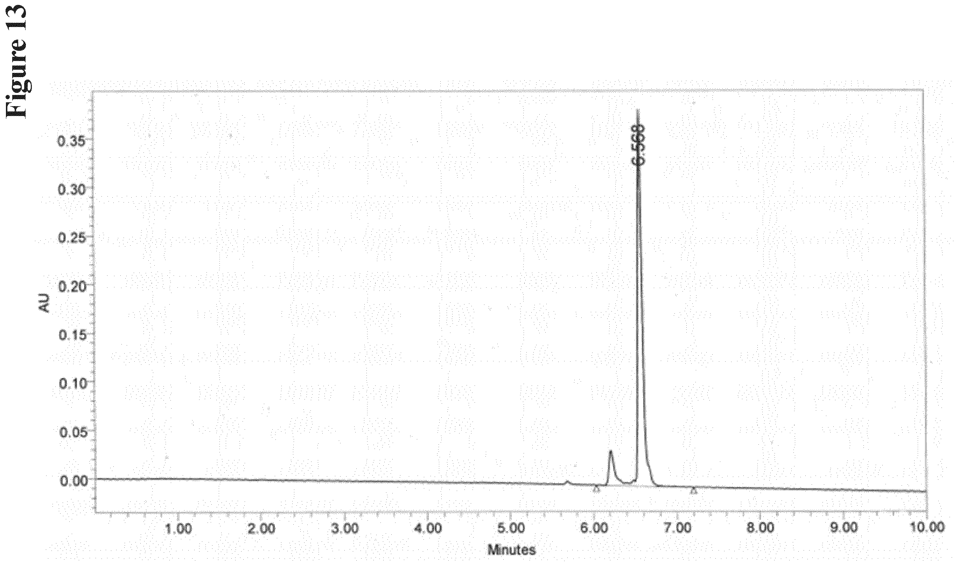

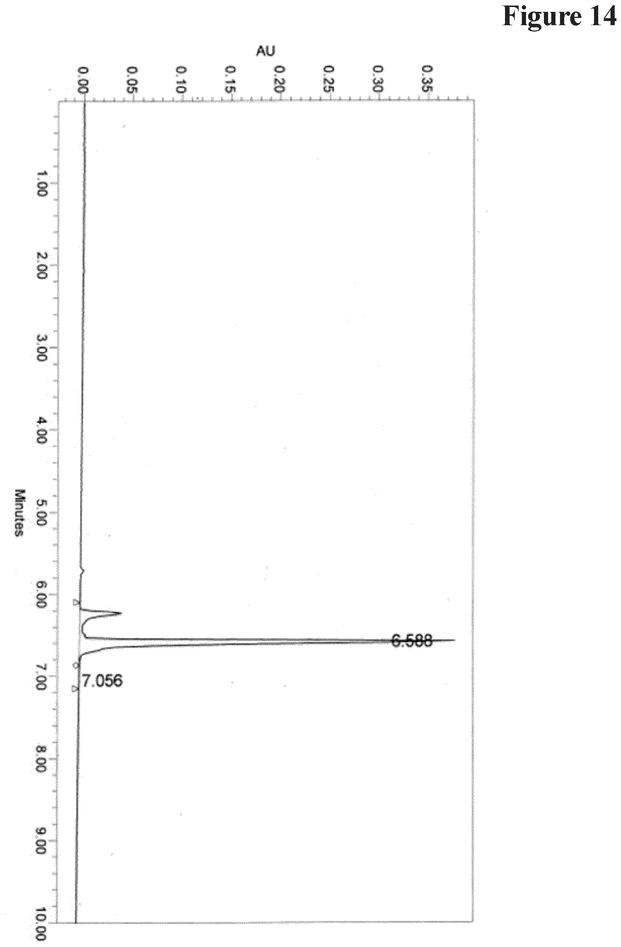

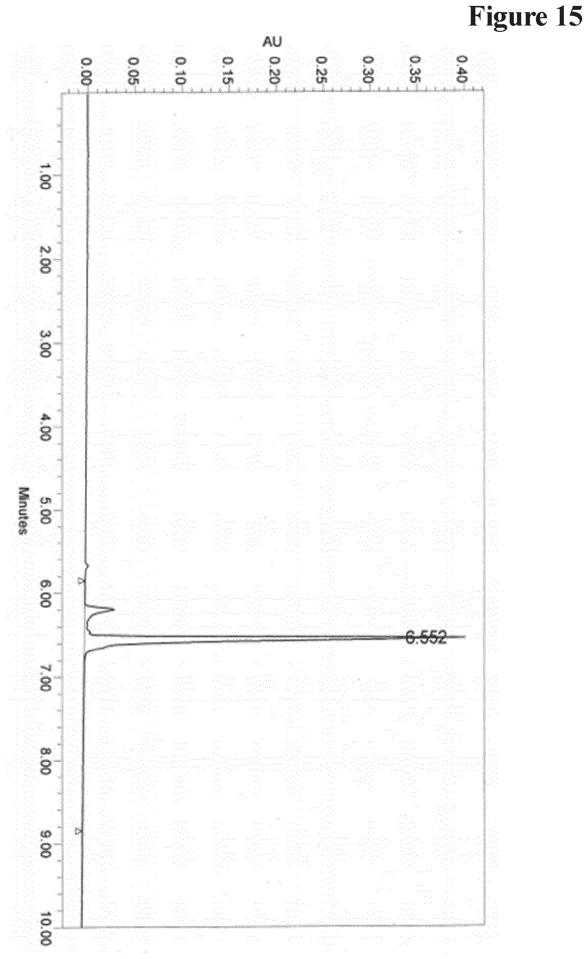

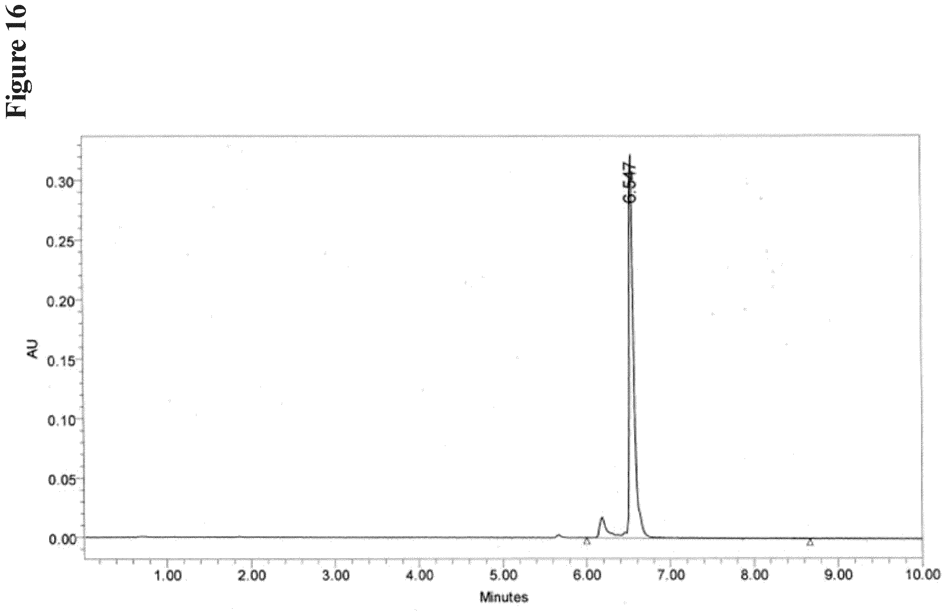

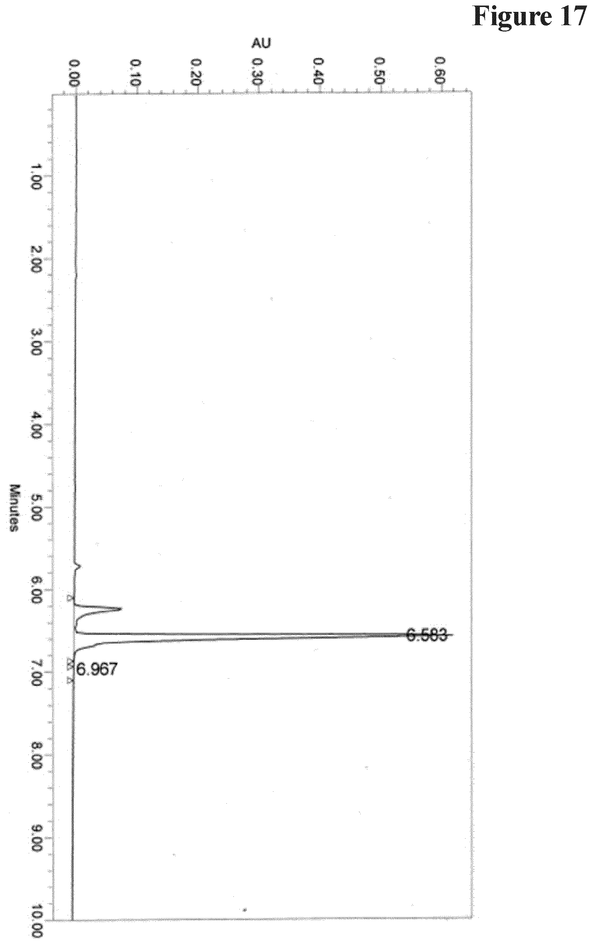

See Figures 11-17 for illustration.

### c) identification of sequence which forms duplex slowly

### Number of possible Watson-Crick complementary base pairs: 10

5' cgtcaggcag 3' / 5' ctgcctgacg 3' were found with slow duplex formation ;
(SEQ ID NOs: 6 and 5, respectively).

### Number of possible Watson-Crick complementary base pairs: 15

5' cgtcaggcagttcag 3' / 5' ctgaactgcctgacg 3' were found with slow duplex formation ; (SEQ ID NOs: 47 and 48, respectively).

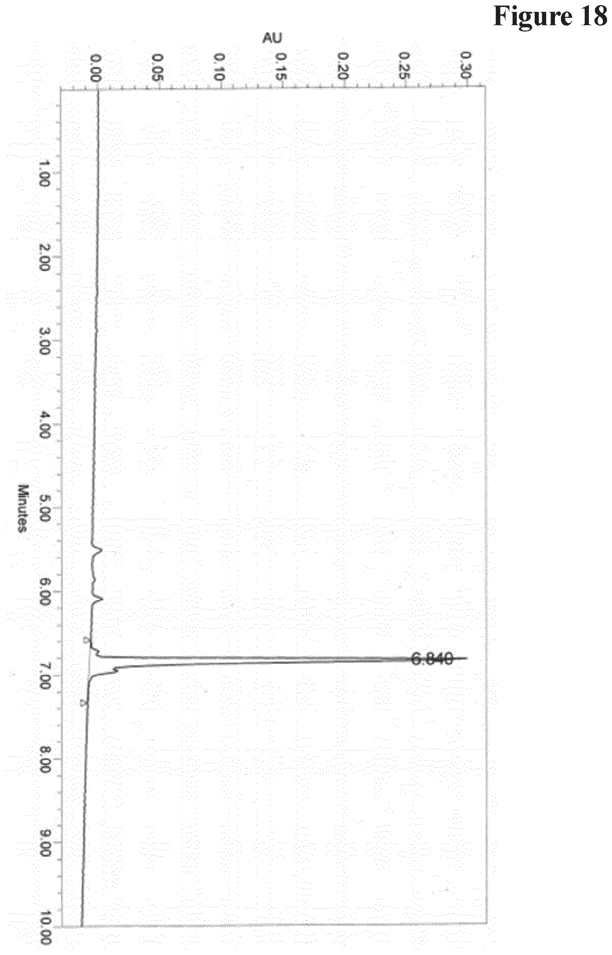

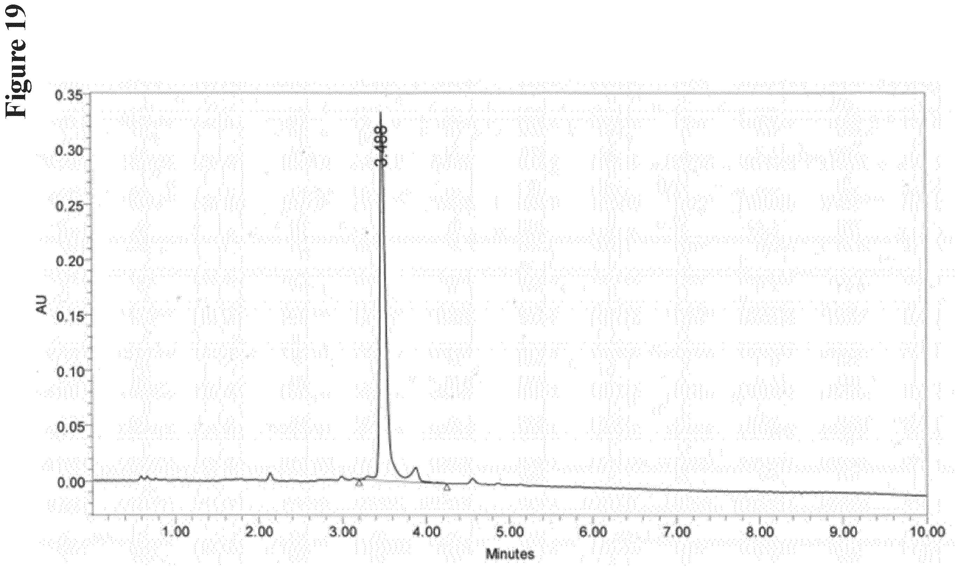

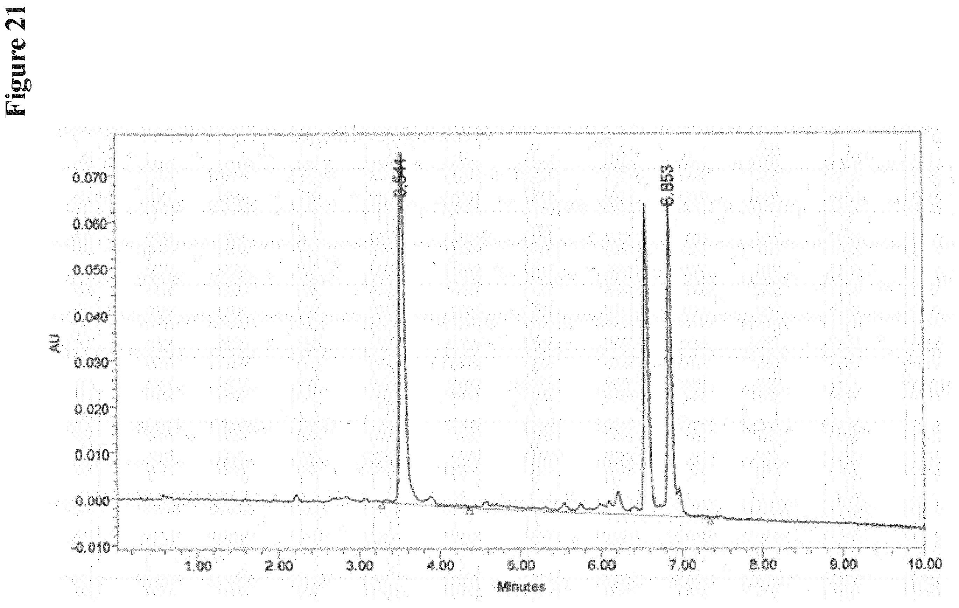

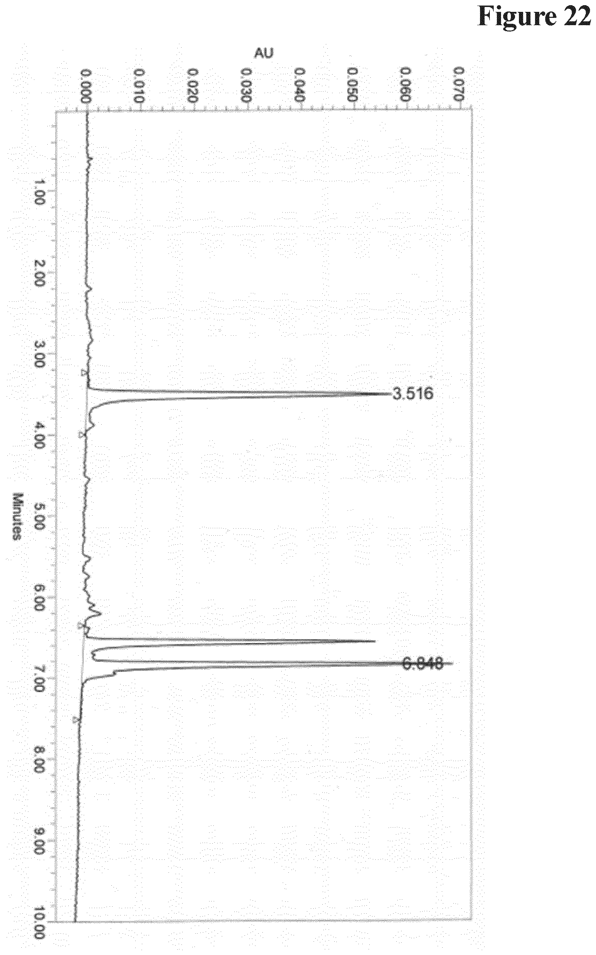

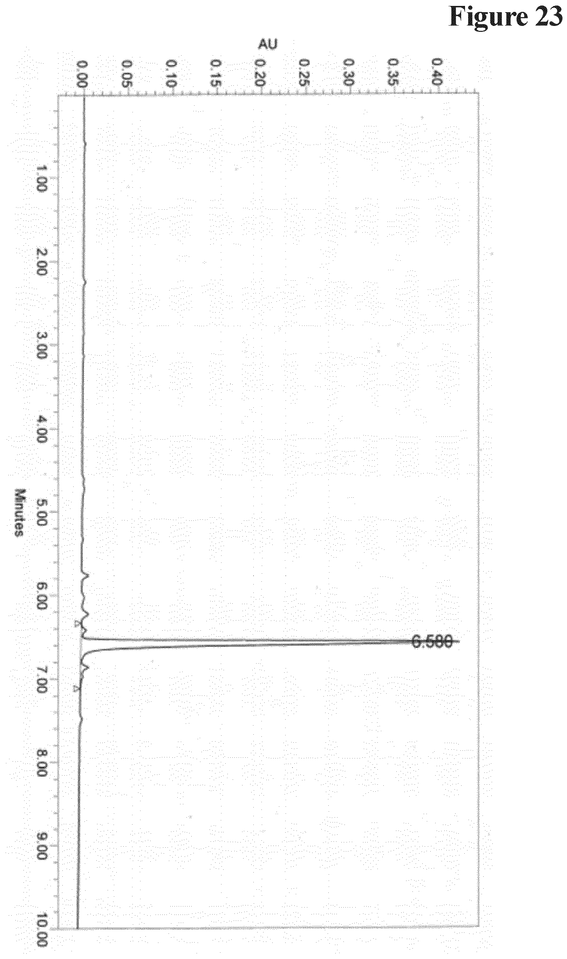

See Figures 18-20 and Figures 21-23 for illustration.

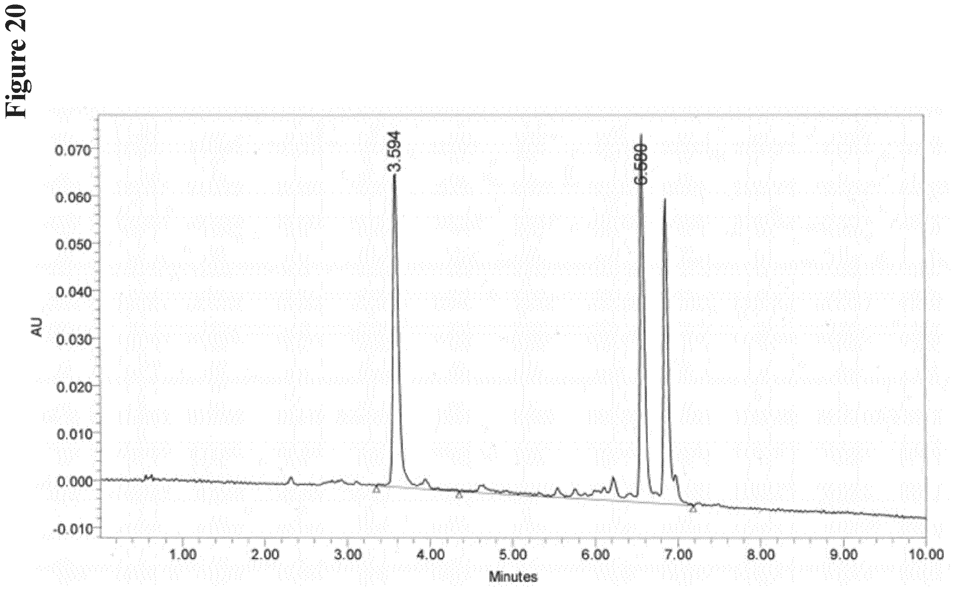

Figure 20 illustrates identification of a binding pair showing slow hybridization. Calculation of ratio of mixed LNAs 5'-Bi-Heg- cgtcaggcagttcag-3' (5'-modified SEQ ID NO:47) combined with 5'-ctgaactgcctgacg-3' (SEQ ID NO:48.

| **LNA oligo** | **retention time [min]** | **HPLC%** | **extinction coefficient (ε)** | **HPLC% * ε⁻¹ * 1000** |
|---|---|---|---|---|
| | | | **[l*mol⁻¹*cm⁻¹]** | |
| LNA of SEQ ID NO:48 (single strand) | 3.59 | 37.44 | 158000 | 0.237 |
| LNAs of SEQ ID NO:47 / SEQ ID NO:48 (duplex) | 6.58 | 33.29 | 320400 | 0.104 |

HPLC% * ε⁻¹ * 1000 (LNA duplex) / HPLC% * ε⁻¹ * 1000 (LNA single strand) = 0.104/0.237 = 0.44

### Example 4

### Biospecific interaction analysis, immobilized first LNA oligonucleotide contacted with second LNA oligonucleotide, three different motifs; kinetic characteriziation at 25°C and 37°C

### a) outline of the approach

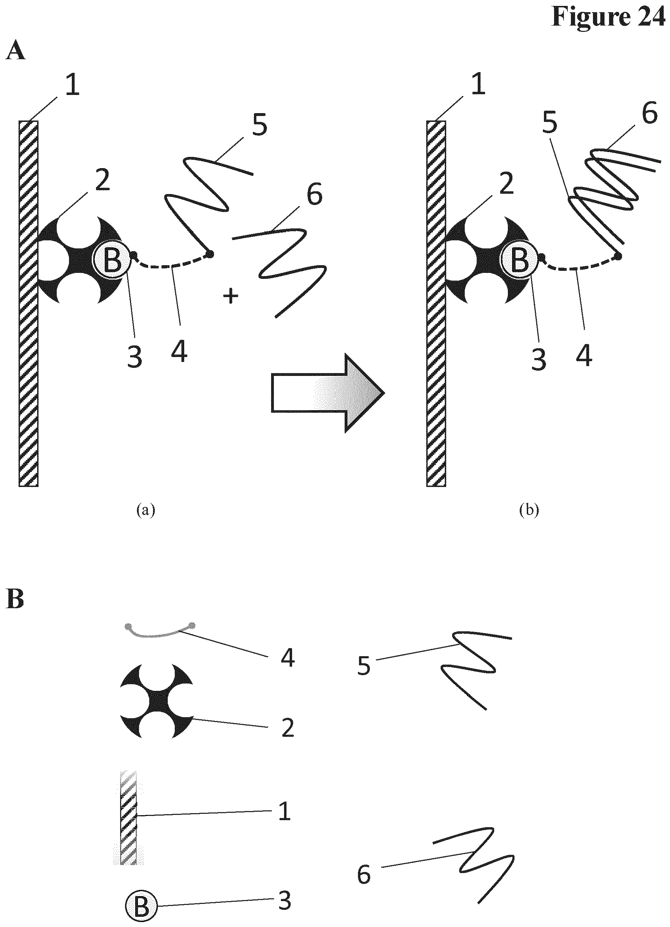

- 12 different oligonucleotide LNA sequences were designed and terminally biotinylated (Bi-LNA-sequences)
- 7 LNA oligonucleotide sequences were analyzed for binding to the 12 immobilized Bi-LNA-sequences at 25 °C / 37 °C
- 3 min association time and 5 respectively 30 min dissociation time with flow rate 60 µl/min
- LNA-samples were preincubated over night at RT (room temperature) in slightly basic buffer as recommended by the manufacturer (chemical deactivation)
- Test setup is depicted on Figure 24

### b) technical procedure

Kinetic investigations were performed on a GE Healthcare Biacore 8k instrument.

A Biacore Biotin Capture Kit, Series S sensor ( Cat.-No.28-9202-34) was mounted into the instrument and was hydrodynamically addressed and preconditioned according to the manufacturer's instructions. The system buffer was HBS-T (10 mM HEPES pH 7.4, 150 mM NaCl, 0.05 % TWEEN 20). The sample buffer was the system buffer. The Biotin Capture Reagent, as provided by the manufacturer GE Healthcare, was diluted 1:50 in system buffer and was injected at 10 µl/min for 60 sec into all measurement flow cells. The reference cells were not immobilized and remained blanc controls. 10 nM of the respective biotinylated ligand was injected at 30 µl/min to obtain ligand capture levels between 4 RU to 30 RU. Concentration series of analytes in solution were injected at 60 µl/min for 3 min association time. Dissociation was monitored for 5 minutes. High affinity interactions were monitored for 30 minutes dissociation time. Analyte concentration series were 0 nM (buffer), 0.11 nM, 0.33 nM, 3 nM, 9 nM, 27 nM. In another embodiment 0 nM, 0.56 nM, 1.67 nM, 5nM, 15nM and 45 nM. The CAP sensor was fully regenerated by 1 minute injection of 100 mM NaOH. Kinetic data was determined using the Biacore Evaluation software.

### c) results

7 different LNA oligonucleotides representing 3 different sequence motifs were analyzed for binding 12 different complementary biotinylated LNA oligonucleotides (Bi-LNA) having sequences of varying length, at two different temperatures: 25 °C and 37 °C.

0.05 % Tween 20 was used as detergent in the SPR-measurements.

Initial Molar ratio MR = 1.0-0.7 (data not shown) indicated 1:1 binding, MR decreased during analysis cycles (MR = 0.5-0.3), most probably due to deactivaton of streptavidin surface by the use of harsh basic pH during regeneration steps.

### Motif "Sequence 1"

### 9mer LNA showed binding to complementary Bi-LNA 7-9mers with motif 1

At 37°C ' ' Bi-(HEG)₄- 5' caggagca 3' (5'-modified SEQ ID NO:2)' and ' Bi-(HEG)₄- 5' caggagc 3' (5'-modified SEQ ID NO:15) showed high complex stabilities with the complementary oligonucleotide 5' tgctcctgt 3' (SEQ ID NO:9) ' with and without (HEG)₄-MH-5'-tag.

t_{/2 diss} (Bi-LNA 7&8mer/9mer) => 247/ 228 minutes,

t_{/2 diss} (Bi-LNA 7&8mer/9mer with Heg4-MH5') => 734/ 800 minutes, resulting in high affinities (*K_{D}* = 6-9 pM):
Hybridization with "Sequence 2"-LNA showed weaker binding to Sequence 1 Bi-LNA 7-9mer
No binding detectable for 9-mer A sequence '5' aaaaaaaaa '3' (SEQ ID NO:28) including the (HEG)₄-MH-5'-tag.

### Motif "Sequence 2"

Bi-LNAs representing motif 2 with varying length (12mer, 10mer, 8mer,7mer and 6mer) didn't show any binding to LNA-motifs 1 or 3.

Motif 2-LNA-binding with varying length showed comparable complex formation for 6-8mers, and only slightly slower complex formation for the 12mer.

Complex stability varied: Bi-LNA 7mer showed highest complex stabilities in these experiments, followed by the Bi-LNA 8mer
t_{/2 diss} (Bi-LNA 7mer/6mer) = 238 minutes,
t_{/2 diss} (Bi-LNA 7mer/7mer) = 720 minutes,
t_{/2 diss} (Bi-LNA 7mer/8mer) = 644 minutes,
t_{/2 diss} (Bi-LNA 8mer/7mer) = 545 minutes,
t_{/2 diss} (Bi-LNA 8mer/8mer) = 433 minutes, resulting in high affinities (*K_{D} =* 1- 5 pM)
Motif 2 was estimated to be superior to motif 1

### Motif "Sequence 3"

The 5' modified poly-A Sequence (HEG)₄-MH-5' sequence 5' aaaaaaaaa 3' (SEQ ID NO:28) '' was used as negative control. This control did n'ot show any binding to any of the biotinylated Sequences 1 and 2. However, specific binding was detected with complementary Poly-T-Sequences from "group 3".

At 37°C complex stability increased significantly with increasing LNA-length from 6-9mer by factor 1000 (t/2 diss= 1 to >1160 minutes) & complex formation decreased by factor 55, resulting affinities were in a range KD = 300pM-10 pM -ssL-DNA hybridization is slower with increasing length from 6mer to 9mer, complex stabilities are persistently high; Overhangs with > 2 unpaired nucleotides obviously decreased the complex stability for PolyA/PolyT-pairing.

### d) conclusion

With the goal in mind to provide a replacement for the streptavidin:biotin binding pair it is important to select a low affinity (desired to be in the pM range) binding pair with very fast association rate constant already at 25 °C, and a persisting high complex stability at 37°C.

All-LNA oligonucleotide duplex with 4 to 5 complementary LNA nucleobase pairings representing motif "Sequence 2" meet the demands for a desired binding pair in that they show fast association into saturation and high complex stability.

In order to secure quick association, the binding pair is desired to be as short as possible to circumvent time consuming "mispriming" intermediates. 'Bi-(HEG)₄-5' caccaac 3' (7mer oligonucleotide, SEQ ID NO:19) binding to 5' gttggt 3' ' and Bi-(HEG)₄-MH- 5' gttggtgt 3' (5'-modified SEQ ID NO:16) ' showed complex formation and complex stability with t_{/2 diss} = 720 respectively 644 minutes and a resulting high affinity (*K_{D}* = 2 pM) at 37°C. 'Bi-(HEG)₄- 5' acaccaac 3' (8mer, 5'-modified SEQ ID NO:14) binding to (HEG)₄-MH- 5' gttggtg 3' (5'-modified SEQ ID NO:21) and to (HEG)₄-MH- 5' gttggtgt 3' (5'-modified SEQ ID NO: 13)' showed sufficient complex formation and complex stability with t/2 diss = 545 and 433 minutes, respectively, and a resulting high affinity (KD = 2 pM) at 37°C.

LNA with varying length with motif "sequence 1" did not bind. As a negative control the sequence 5' aaaaaaaaa 3' (SEQ ID NO:28) including the (HEG)₄-MH-5'-tag was tested, 'without any observation of measurable intermolecular interactions.

### Example 5

### Biospecific interaction analysis

**a) outline of the approach and assay set-up**
   - reversible captured streptavidin-conjugate via CAP-Kit
   - streptavidin is conjugated with complementary ss-LNA
   - oligo binding reversible to pre-immobilized ss-LNA oligo on a SCM
   Determination of:
   - Capture Level (CL), association rate constant *kₐ,*
   - dissociation rate constant *k_{d},*
   - dissociation equilibrium constant *K_{D}*
   - Molar ratio (MR)
   - Test setup is depicted on Figure 51 A
   - 4 free LNA constructs "motif 2" with varying length (6-8mer & 12mer) and LNA-Fab<TSH>-conjugates (LNA-Fab<TSH> = antibody Fab fragments specific for the TSH antigen) were analyzed for binding to complementary Bi-LNA-Sequences at 25°/ 37°C.
   - Bi-LNA-Sequences were captured as ligands on a CAP-Chip via reversible Biotin-Capture-Kit;
   - free LNA or LNA-Fab<TSH>-conjugates were used as analytes in solution
   - Hybridization was analyzed with 3 minutes association time and 30 minutes dissociation time,
   - flow rate 60 µl/min
   - C_{(free LNAs)}= 9 - 0.1nM , _{c(LNA-Fab<TSH>-conjugates)}= 45 - 0.6 nM, c_{(12merLNA/Fab<TSH>-conjugates)}= 45 - 0.6 nM
   - LNA-samples were pre-incubated over night at RT in slightly basic buffer as recommended from customer (chemically deactivation)
**b) Reagents: Sequence "Motif 2"**
   **biotinylated Ligands**
      'Bi-(HEG)₄- 5' accaac 3' (SEQ ID NO:20)
      BMO 28.542740, GO4094, ID 6681, 6mer, MW 3.8 kDa
      'Bi-(HEG)₄- 5' caccaac 3' (SEQ ID NO:19)
      BMO 28.542739, GO4093, ID 6681, 7mer, MW 4.1 kDa
      'Bi-(HEG)₄- 5' acaccaac 3' (SEQ ID NO:14)
      BMO 28.542738, GO4092, ID 6680, 8mer, MW 4.4 kDa
      'Bi-(HEG)₄- 5' caacacaccaac 3' (SEQ ID NO:52)
      BMO 28.542742, GO4096, ID 6684, 12mer, MW 5.8 kDa
   **Analytes**
      2300/103 (HEG)₄-MH- 5' gttggt 3' (SEQ ID NO:16) '
      BMO 28.170333, AO581, ID 6719, 6mer, MW 3.8 kDa
      2300/104 (HEG)₄-MH- 5' gttggtg 3' (SEQ ID NO:21)'
      BMO 28.170334, AO582, ID 6720, 7mer, MW 4.1 kDa
      2300/105 (HEG)₄-MH- 5' gttggtgt 3' (SEQ ID NO:13)'
      BMO 28.170335, AO583, ID 6721, 8mer, MW 4.4 kDa
      2300/102 (HEG)₄-MH- 5' gttggtgtgttg 3' (SEQ ID NO:53)'
      BMO 28.542727, GO4073, ID 6653, 12mer, MW 5.8 kDa
   **'mAb<TSH>M-Tu1.20-F(ab')2-SATP-D-LNA-conjugates**
      2331/111 mAb<TSH>M-Tu1.20-F(ab')₂-SATP-D-LNA- 5' gttggt 3' (SEQ ID NO:16), 6mer, MW 104 kDa
      2331/112 mAb<TSH>M-Tu1.20-F(ab')₂-SATP-D-LNA- 5' gttggtg 3' (SEQ ID NO:21), 7mer, MW 104 kDa
      2331/113 mAb<TSH>M-Tu1.20-F(ab')₂-SATP-D-LNA- 5' gttggtgt 3' (SEQ ID NO:13), 8mer, MW 104 kDa
      2331/114 mAb<TSH>M-Tu1.20-F(ab')₂-SATP-D-LNA- 5' gttggtgtgttg 3' (SEQ ID NO:53), 12mer, MW 106 kDa
      mAb<TSH>M-Tu1.20-F(ab')₂ signifies a F(ab')₂ fragment of a monoclonal antibody specific for TSH which is human Thyroid-stimulating hormone. D-LNA signifies that the oligonucleotide with the subsequent nucleobase sequence consists of D-LNA monomers. D-LNA oligonucleotides were used
**c) results**
   The four different 5'-modified LNA oligonucleotides as given above, and oligonucleotides with the same respective sequences but comprised in F(ab')₂<TSH> conjugates represented sequence "motif 2" with 6-, 7-, 8- & 12-mer length. All were analyzed for binding their complementary Bi-LNA-Sequence at 25°/37°C.
   TSH-binding (TSH being the analyte ) to hybridized LNA-Fab<TSH> conjugates was analyzed, too.

### Sequence "Motif 2"

Bi-LNAs of varying length showed comparable complex formation, the complex stabilities range from t_{/2 diss} 154 to >232 minutes with pM affinity range (*K_{D}* 3 - 10 pM) at 25 °C. At 37 °C, the complex formations are in the pM affinity range (*K_{D}* 11-26 pM). Hybridization kinetics of the free oligos are mass-transport limited (data depicted in red) at 25 °C & 37 °C, correction for MTL was performed using SW Scrubber. Molar Ratios (MR) 0.8 - 1.1 indicated stoichiometric 1:1 hybridization at both temperatures. Oversaturation of 12-mer association phase at 25°C.

The LNA-Fab<TSH> conjugates showed a factor 2-4 slowed down complex formation in comparison to the unconjugated LNA oligonucleotides. No MTL during hybridization of the Fab<TSH>-LNA-conjugates. Complex stabilities t_{/2 diss} > 232 minutes, resulting in pM affinity range (*K_{D}* = 22 -11 pM) at 25°C. At 37°C FAb<TSH>-LNA-conjugates (7-, 8- & 12mer) showed no signifiant differences in complex formation when compared to free LNA of same length, complex stabilities t_{/2 diss} > 232 minutes, ranging in the pM affinity range (K_{D} < 12-14 pM).

Molar Ratios for LNA-Fab<TSH> conjugates MR 0.1 - 0.6 indicated sub-stoichiometric 1:1 binding. TSH binding to hybridized LNA-FAb<TSH> conjugates of varying length was analyzed. TSH-binding to LNA-Fab<TSH> conjugates (6-8mer & 12mer) showed comparable kinetic profiles Fast complex formation and sufficient complex formation with t_{/2 diss} 31-33 minutes, resulting affinities *K_{D} =* 0.7 nM

The binding constants are in the known affinity range for this interaction.

Molar Ratios (MR) 1.8/1.9 showed fully functional stoichiometric 2:1 binding indicating binding functional conjugates.

It was found that hybridized Fab conjugates show full antigen binding activity oft he antibody moieties in the conjugates. Results see Figure 52.

Also see Figure 51 C for the following data

| | secondary antibodies | | | |
|---|---|---|---|---|
| primary antibodies | Antibody A | 23C11-IgG | 6C6-IgG | 4H4-IgG |
| Antibody A | 0.1 | 0.1 | 0.1 | 0.1 |
| 23C11-IgG | 0.2 | 0.0 | 0.0 | 0.0 |
| 6C6-IgG | 0.4 | 0.0 | 0.0 | 0.0 |
| 4H4-IgG | 0.2 | 0.0 | 0.0 | 0.0 |

Table: Molar Ratio Epitope Accessibility Matrix showing the hTK sandwich formation of 4 anti-hTK antibodies. MR_{EA} = 1, fully independent epitope, MR_{EA} < 1 overlapping epitopes.

Antibody A is able to form immunocomplexes with 23C11, 6C6 and 4H4. 23C11, 6C6 and 4H4 share the same epitope.

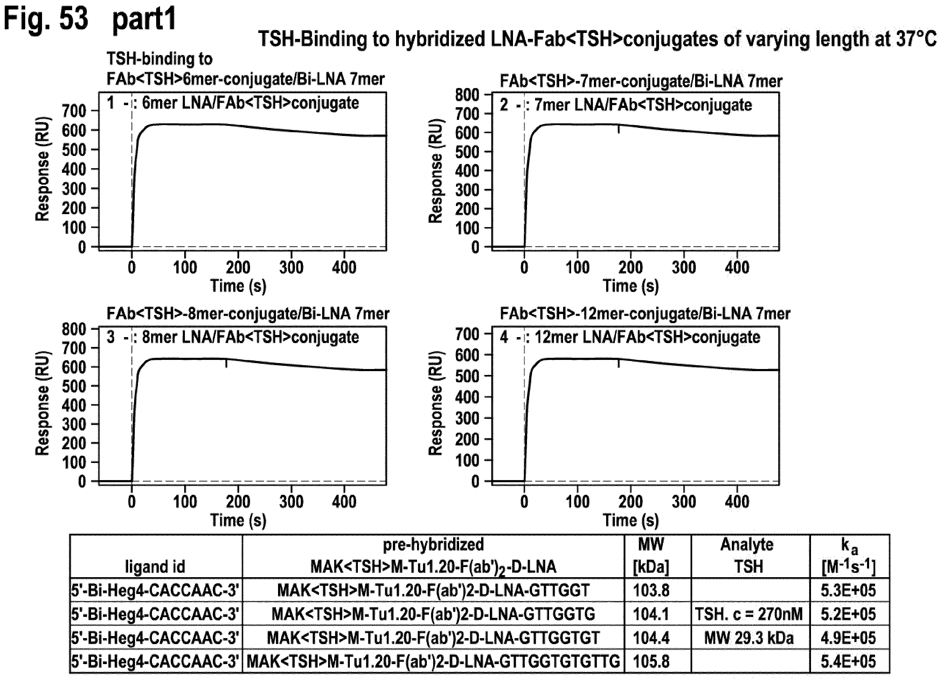

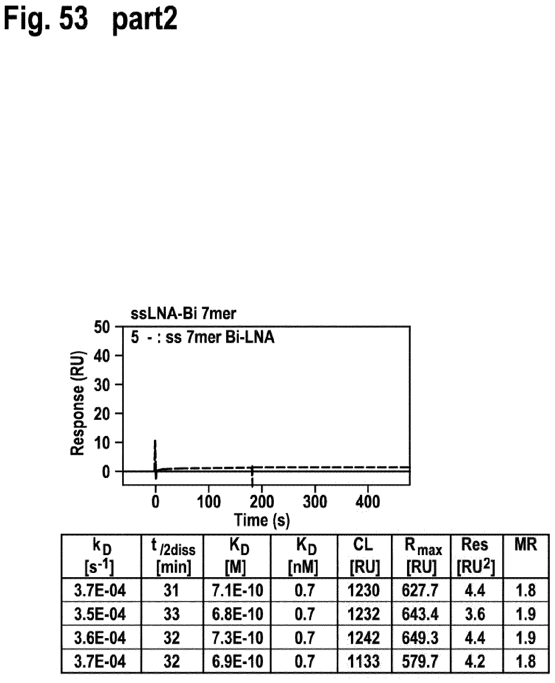

### d) alternative approach (see Figure 51 B, results Figure 53)

- TSH-binding to pre-hybridized LNA-FAb<TSH> conjugates of varying length (6-, 7-, 8- & 12mer) was analyzed at 37°C
- Assay format see slide 10, binding profiles see slides 11
- 3 Bi-LNA-Sequences were irreversibly bound to a SA-Chip on Fc2-4
- LNA-Fab<TSH> conjugations (6-,7- & 8mer) were pre-hybridized with their complementary Bi-LNA at 37°C
- TSH was used as analyt ein solution with 3 minutes association time and 5 minutes dissociation time,
- flow rate 60 µl/min, c_{TSH}= 270 nM

Results see Figure 53.

### Example 6

### Biospecific interaction analysis

**a) outline of the approach and assay set-up**
   - Schematic overview of the experiment is presented on Figure 54
   - free LNA constructs of varying length (5-,-6-, 9- or 15mer) and sequences were analyzed for binding to complementary Bi-LNA-Sequences (4-6-, 9- or 15mer) at 25°/ 37°C
   - Bi-LNA-Sequences were captured as ligands on a CAP-Chip via reversible Biotin-Capture-Kit;
      free LNAs were used as analytes in solution
   - Hybridization was analyzed with 3 minutes association time and 30 minutes dissociation time,
      flow rate 60 µl/min
   - _{C(free LNAs)}= optimized for each interaction

Determination of: Capture level (CL), association rate constant kₐ, dissociation rate constant k_{d}, dissociation equilibrium constant K_{D}, molar ratio (MR).
reversible captured SA-conjugate via CAP-Kit, streptavidin (= SA) -conjugated with complementary ss-LNA oligo binding reversible to pre-immobilized ss-LNA oligo.

### b) reagents

### biotinylated Ligands

2387/L01 Bi-(HEG)- 5' accaac 3' (SEQ ID NO:20)
BMO 28.170341, AO591, ID 6730, 6mer, MW 2.71 kDa
2387/L02 Bi-(HEG)- 5' cacaccaac 3' (SEQ ID NO:30)
BMO 28.170342, AO592, ID 6731, 9mer, MW 3.71 kDa
2387/L03 Bi-(HEG)- 5' caccaacacaccaac 3' (SEQ ID NO:54)
BMO 28.170343, AO593, ID6732, 15mer, MW 5.73 kDa
2387/L04 Bi-(HEG)- 5' ggaag 3' (SEQ ID NO:34)
BMO 28.170347, AO597, ID6736, 5mer, MW 2.44 kDa
2387/L05 Bi-(HEG)- 5' ggaaga 3' (SEQ ID NO:36)
BMO 28.170348, AO598, ID 6737, 6mer, MW 2.78 kDa
2387/L06 Bi-(HEG)- 5' ggaagagaa 3' (SEQ ID NO:38) BMO 28.170349, AO599, ID 6738, 9mer, MW 3.82 kDa
2300/12 Bi-(HEG)₄- 5' tttttt 3' (SEQ ID NO:27)
BMO 28.170336, AO584, ID 6722, 6mer, MW 3.71 kDa
2387/L08 Bi-(HEG)- 5' ctgtca 3' (SEQ ID NO:40)
BMO 28.170354, AO604, ID 6743, 6mer, MW 2.71 kDa
2387/L09 Bi-(HEG)- 5' cgtcaggcagttcag 3' (SEQ ID NO:55)
BMO 28.170356, AO606, ID 6745, 15mer, MW 5.12 kDa
2387/L10 Bi-(HEG)- 5' ggagc 3' (SEQ ID NO:43)
BMO 28.170358, AO608, ID 6747, 5mer, MW 2.43 kDa
2387/L11 Bi-(HEG)- 5' ggagca 3' (SEQ ID NO:45)
BMO 28.170360, AO610, ID 6749, 6mer, MW 2.77 kDa
2387/L12 Bi-(HEG)₄- 5' -ccaac 3' (SEQ ID NO:46)
BMO 28.542748, GO4105, ID 6764, 5mer, MW 3.40 kDa
2387/L13 Bi-(HEG)₄- 5' caac 3' (SEQ ID NO:56)
BMO 28.542749, GO4106, ID 6765, 4mer, MW 3.07 kDa
2387/L14 Bi-(HEG)₄- 5' ttttt 3' (SEQ ID NO:57)
BMO 28.542750, GO4107, ID 6766 5mer, MW 3.38 kDa 2387/L15 Bi-(HEG)₄- 5' tttt 3' (SEQ ID NO:58)
BMO 28.542751, GO4108, ID 6768 4mer, MW 3.05 kDa

### Analytes

2387/A01 3'-TGG TTG-5'
BMO 28.170344, AO594, ID, 6733, 6mer, MW 2.01 kDa
2387/A02 3'-GTG TGG TTG-5'
BMO 28.170345, AO595/ ID 6734, 9mer, MW 3.05 kDa
2387/A03 3'-GTG GTT GTG TGG GTT -5'
BMO 28.170346, AO596, ID 6735, 15mer, MW 5.12 kDa
2387/A04 3'-CCT TC-5'
BMO 28.170350, AO600, ID 6739, 5mer, MW 1.60 kDa
2387/A05 3-'CCT-TCT-5'
BMO 28.170351, AO601, ID 6740, 6mer, MW 1.93 kDa
2387/A06 3'-CCT TCT CTT-5'
BMO 28.170352, AO602, ID 6741, 9mer, MW 2.92 kDa
2387/A07 3'-AAA AAA-5'
BMO 28.170353, AO603, ID 6742, 6mer, MW 1.99 kDa
2387/A08 3'-GAC AGT-5'
BMO 28.170355, AO605, ID 6744, 6mer, MW 2.00 kDa
2387/A09 3'-GCA GTC CGT CAA GTC-5'
BMO 28.170357, AO607, ID 6746, 15mer, MW 5.04 kDa
2387/A10 3'-CCT CG-5'
BMO 28.170359, AO609, ID 6748, 5mer, MW 1.62 kDa
2387/A11 3'-CCT CGT-5'
BMO 28.170361, AO611, ID 6750, 6mer, MW 1.95 kDa

### c) results

11 LNAs with different sequences were analyzed for binding to their complementary Bi-LNA-Sequences. Additionally 2 Bi-LNAs of different length (5mer & 4mer) pairing with free LNA 6mer were analyzed at 25°/37°C.

Sequence "Motif 2" (2387/L01-L03) & "Short Motif 2" (2387/L12& L13) 6mer & 9mer Bi-LNA 5'-Bi-Heg-ACC AAC-3' and 5'-Bi-Heg-CAC ACC AAC-3' show high affinity-binding to their complementary LNAs 6mer 3'-TGG TTG-5' respectively 9mer 3'-GTG TGG TTG-5'. Kinetic signatures are characterized by fast hybridization, complex stabilities are persistingly high with t_{/2 diss} = 160 to >232 minutes and pM-affinity range (*K_{D} =* 1 - 9 pM) at 25° & 37°C; hybridization kinetics are mass-transport limited at 25 °C & 37 °C, correction for MTL was made, too.

Molar Ratios (MR) 1.1/1.2 indicate stoichiometric 1:1 hybridization at both temperatures for the 6mer pair; MR 1.3/1.5 indicate over-stoichiometric binding for the 9mer-pair. The Bi-LNA 15mer shows slowed down hybridization kinetics compared to 6 & 9mers, resulting in slightly lower affinities. *K_{D}* = 24/53 pM at both temperatures; MR 1.3 indicates slightly over-stoichiometric binding.

When binding to 5mer (2387/L12) Bi-LNA 5'Bi-4x(HEG)-CCA AC-3' free 6mer-LNA 3'-TGG TTG-5' shows reduced complex stability t_{/2 diss} = 50 minutes with 2 digit pM-affinity range, when binding to 4mer (2387/L13) 5'Bi-4x(HEG)- CAA C-3' the complex stability drops down to t_{/2 diss} < 1 minute, resulting in 2 digit nM affinity. MR 1.1-1.3 indicate slightly over-stoichiometric binding Overhangs with 1 or 2 unmatched nucleotides may thus be interpreted in the present case to decrease complex stability to some extent.

"Motif 3" Poly-T controls "short" (2300/12 and 2387/L14 & L15); 3 PolyT-controls Bi-LNA of varying length (5- &-6mer) show binding to PolyA-6mer with typical fast on-/off- profiles, complex-half-lifes t_{/2 diss} < 2 minutes at 25° & 37°C, the Bi-LNA 4mer shows only weak/ no binding to PolyA-6mer.

"Motif 4" (2387/L04-L06) 5-, 6- or 9mer 5'-Bi-Heg-GGA AG-3', 5'-Bi-Heg-GGA AGA-3', or 5'-Bi-Heg-GGA AGA GAA-3' are inferior to "motif 2" when binding to their complementary LNAs, complex half-lifes t _{/2 diss} between 20-65 minutes with resulting 2-3 digit pM affinities at 25°C.

"Motif 5" (2387/L08) 5'-Bi-Heg -CTG TCA-3' binding to complementary LNA 3'-GAC AGT-5' shows slightly slower hybridization than 6mer of "motif 2", complex stabilities with 2-digit pM affinities for 25° & 37°C. The Molar Ratios 0.3/0.4 indicate sub-stoichiometric binding.

"Motif 6" (2387/L09) The 15mer 5'-Bi-Heg -CGT CAG GCA GTT CAG-3' binding 3'-GCA GTC CGT CAA GTC-5' shows a 1-digit nM-affinity interaction caused by slowed down hybridization and reduced complex stability; The Molar Ratios 0.1/0.4 indicate sub-stoichiometric binding.

### "Motif 7" (2387/L10 & L11)

5mer 5'-Bi-Heg -GGA GC-3' outperformed the 6mer 5'-Bi-Heg -GGA GCA-3' binding their complementary LNAs; complex half-lifes t_{/2 diss} between 174 and 62 minutes with 32 /186 pM affinity-range at 25°C, 5 mer shows 29 pM interaction at 37°C, Molar Ratios MR 0.5/0.3 indicate sub-stoichiometric binding at 25°C and increasing at 37°C (MR 1.2/0.6).

### d) conclusion

Both Bi-LNA 5'-Bi-Heg-ACC AAC-3' and 5'-Bi-Heg-CAC ACC AAC-3' ("motif 2") show high-affinity binding to their complementary LNAs 6mer 3'-TGG TTG-5' resp. 9mer 3'-GTG TGG TTG-5'.The Molar Ratio indicates fully functional 1:1 LNA-hybridization. 5'-Bi-Heg-ACC AAC-3' / 3'-TGG TTG-5' shows slightly improved hybridization kinetics in comparison to 5'-Bi-(HEG)4-ACCAAC-3' / 3'-TGG-TTG-5'- Heg4-MH-5', due to 2-fold improved complex stability.

The absolute density for captured Bi-LNA 5'-Bi-Heg-ACC AAC-3' on the sensor surface used in the experiments was 11 fmol/ mm² (30 pg/ mm²), based on the vendor information 1000 RU = 1 ng/mm². In a hyrogel it is assumed to correspond to a concentration 0.3 mg/mL.

## Claims

1. A method for selecting and providing a binding pair of single-stranded all-LNA oligonucleotides capable of forming in aqueous solution at a temperature from 0 °C to 40 °C an antiparallel duplex with 5 to 15 consecutive base pairs, the method comprising the steps of
(a) providing a first single-stranded (= ss-) oligonucleotide consisting of 5 to 15 locked nucleic acid (= LNA) monomers, each monomer comprising a nucleobase, the nucleobases of the first ss-oligonucleotide forming a first nucleobase sequence;
(b) providing a second ss-oligonucleotide consisting of 5 to 15 LNA monomers, the second ss-oligonucleotide comprising at least the number of monomers as the first ss-oligonucleotide, each monomer of the second ss-oligonucleotide comprising a nucleobase, the nucleobases of the second ss-oligonucleotide forming a second nucleobase sequence, the second nucleobase sequence comprising or consisting of a nucleobase sequence complementary to the first nucleobase sequence in antiparallel orientation and predicting the capability of the first and second ss-oligonucleotide to form with each other an antiparallel duplex, the predicted duplex comprising or consisting of 5 to 15 consecutive base pairs, wherein the two bases of each base pair are bound to each other by hydrogen bonds;
(c) mixing in an aqueous solution about equal molar amounts of the first and second ss-oligonucleotide, wherein this step is performed at a non-denaturing temperature, more specifically at a temperature from 0 °C to 40 °C;
(d) incubating the mixture of (c) for a time interval of 20 min or less, thereby obtaining a mixture still comprising the first and second oligonucleotide as ss-oligonucleotides or a mixture containing or consisting of the first and second oligonucleotide as duplex;
(e) detecting and quantifying in the mixture obtained in step (d) ss-oligonucleotides and duplex oligonucleotides; followed by
(f) selecting the binding pair if in step (e) duplex is detectably present, and the molar amount of duplex is higher than the molar amount of ss-oligonucleotides;
(g) optionally synthesizing separately the first and the second ss-oligonucleotide of a binding pair selected in step (f);
thereby selecting and providing the binding pair of single-stranded all-LNA oligonucleotides.

2. The method of claim 1, wherein steps (c) and (d) are performed at a non-denaturing temperature, specifically at a temperature selected from the grop consisting of 0 °C to 5 °C, 5 °C to 10 °C, 10 °C to 15 °C, 15 °C to 20 °C, 20 °C to 25 °C, 25 °C to 30 °C, 30 °C to 35 °C, and 35 °C to 40 °C.

3. The method of claim 1 or 2, wherein each LNA monomer comprises a nucleobase selected from the group consisting of N⁴-acetylcytosine, 5-acetyluracil, 4-amino-6-chloropyrimidine, 4-amino-5-fluoro-2-methoxypyrimidine, 6-amino-1-methyluracil, 5-aminoorotic acid, 5-aminouracil, 6-aminouracil, 6-azauracil, N⁴-benzoylcytosine, 5-bromouracil, 5-chlorouracil, 6-chlorouracil, 6-chloromethyluracil, 6-chloro-3-methyluracil, cytosine, 5,6-dimethyluracil, 5-ethyluracil, 5-ethynyluracil, 5-fluorocytosine, 5-fluoroorotic acid, 5-fluorouracil, 5-iodo-2,4-dimethoxypyrimidine, 5-iodouracil, isocytosine, 5-methylcytosine, 6-methyl-5-nitrouracil, 2-methylthio-4-pyrimidinol, 5-methyl-2-thiouracil, 6-methyl-2-thiouracil, 6-methyluracil, 5-nitrouracil, orotic acid, 6-phenyl-2-thiouracil, 6-propyl-2-thiouracil, 2-thiouracil, 4-thiouracil, thymine, 5-(trifluoromethyl)uracil, uracil, adenine, 8-azahypoxanthine, 8-azaguanine, allopurinol, 4-aminopyrazolo [3,4-d]pyrimidine, 2-aminopurine, 2-acetamido-6-hydroxypurine, 2-amino-6-chloropurine, 2-amino-6-iodopurine, azathioprine, 4-amino-6-hydroxypyrazolo [3,4-d]pyrimidine, aminophylline, N⁶-benzyladenine, N⁶-benzoyladenine, 6-benzyloxypurine, 8-bromotheophylline, 8-bromo-3-methylxanthine, 8-bromo-7-(2-butyn-1-yl)-3-methylxanthine, 6-chloropurine, 8-chlorotheophylline, 6-chloro-2-fluoropurine, 6-chloro-7-deazapurine, 2-chloroadenine, 6-chloro-7-iodo-7-deazapurine, 2,6-diaminopurine, 2,6-dichloropurine, 6-(dimethylamino)purine, 2,6-dichloro-7-deazapurine, 5,6-dichlorobenzimidazole hydrochloride, 7-deazahypoxanthine, 2-fluoroadenine, guanine, hypoxanthine, 9-(2-hydroxyethyl)adenine, isoguanine, 3-iodo-1*H*-pyrazolo-[3,4-*d*]pyrimidin-4-amine, kinetin, 6-mercaptopurine, 6-methoxypurine, 3-methylxanthine, 1-methylxanthine, 3-methyladenine, O⁶-(cyclohexylmethyl)guanine, 6-thioguanine, 2-thioxanthine, xanthine, 5-propynyl-uracil, 5-propynyl-cytidine, 7-deazaadenine, 7-deazaguanine, 7-propynyl-7-deazaadenine, 7-propynyl-7-deazaguanine, and a derivative thereof.

4. The method of claim 3, wherein each LNA monomer comprises a nucleobase selected from the group consisting of adenine, thymine, uracil, guanine, cytosine, and 5-methylcytosine.

5. The method of any of the claims 3 and 4, wherein one or more cytosine(s), if present, is/are replaced by 5-methylcytosine, in an embodiment wherein each cytosine is replaced by 5-methylcytosine.

6. A pair of separate complementary ss-oligonucleotides, each ss-oligonucleotide consisting of 5 to 15 LNA monomers, the separate ss-oligonucleotides in aqueous solution being capable of forming with each other an antiparallel duplex in the absence of denaturing conditions prior to duplex formation or during duplex formation.

7. The pair of separate complementary ss-oligonucleotides of claim 6, wherein the pair of ss-oligonucleotides is obtained by performing a method of any of the claims 1 to 5.

8. The pair of separate complementary ss-oligonucleotides of any of the claims 6 or 7, wherein the pair is selected from the group consisting of
(SEQ ID NO: 1) : (SEQ ID NO:2),
(SEQ ID NO:9) : (SEQ ID NO: 10),
(SEQ ID NO: 11) : (SEQ ID NO:12),
(SEQ ID NO: 13) : (SEQ ID NO:14),
(SEQ ID NO: 15) : (SEQ ID NO: 16),
(SEQ ID NO: 16) : (SEQ ID NO:20),
(SEQ ID NO:17) : (SEQ ID NO:18),
(SEQ ID NO: 19) : (SEQ ID NO:20),
(SEQ ID NO:21) : (SEQ ID NO:22),
(SEQ ID NO:23) : (SEQ ID NO:24),
(SEQ ID NO:25) : (SEQ ID NO:26).

9. The pair of separate complementary ss-oligonucleotides of any of the claims 6 to 8, wherein the first ss-oligonucleotide of the pair is attached to a first target, and the second ss-oligonucleotide is attached to a second target.

10. The pair of separate complementary ss-oligonucleotides of claim 9, wherein a target is independently selected from the group consisting of a solid phase, a biomolecule, and a chemically synthesized compound.

11. A method of forming an antiparallel all-LNA duplex in the absence of denaturing conditions, the method comprising the steps of
(a) providing separately the first and the second member of a pair of single-stranded all-LNA oligonucleotides of claim 6 or claim 7, wherein each single-stranded all-LNA oligonucleotide is separately dissolved in aqueous solution in the absence of a denaturant and kept at a temperature from 0 °C to 40 °C;
(b) contacting the single-stranded all-LNA oligonucleotides of the pair with each other at a temperature from 0 °C to 40 °C in the absence of a denaturant;
thereby forming the antiparallel all-LNA duplex.

12. An antiparallel duplex formed by a first and the second member of a pair of single-stranded all-LNA oligonucleotides, wherein the antiparallel duplex is obtained by a method of claim 11.

13. Use of a pair of separate ss-oligonucleotides of any of the claims 6 to 10 in a receptor-based assay for determining an analyte, the receptor-based assay comprising an analyte-specific receptor and a solid phase for immobilizing the analyte on the solid phase, wherein the first ss-oligonucleotide of the pair is coupled to the analyte-specific receptor and the second ss-oligonucleotide of the pair is coupled to the solid phase.

14. A kit for performing a receptor-based assay for determining an analyte, the kit comprising in a first container an analyte-specific receptor having attached thereto a first member of a pair of separate ss-oligonucleotides of any of the claims 6 to 10, the kit further comprising in a second container a solid phase having attached thereto a second member of the pair.

15. A method of performing an receptor-based assay for determining an analyte, the method comprising the steps of contacting the analyte with an analyte-specific receptor having attached thereto a first member of a pair of separate ss-oligonucleotides of any of the claims 6 to 8, and with a solid phase having attached thereto a second member of the pair, incubating thereby forming a complex comprising the solid phase, the analyte-specific receptor bound to the solid phase and the analyte bound to the analyte-specific receptor, wherein an antiparallel duplex is formed, the duplex consisting of the first and the second member of the pair, wherein the duplex connects the analyte-specific receptor and the solid phase in the complex, followed by detecting analyte bound in the complex, thereby determining the analyte.
